# EUROPEAN PATENT APPLICATION

(11) **EP 4 803 531 A1**
(43) Date of publication of application: **09.09.2026**
(21) Application number: 24885819.3
(22) Date of filing: 31.10.2024
(51) Int. Cl.: C07H 21/04, A61K 31/7088, A61K 48/00, A61P 31/12, C12N 15/10

(54) **CARBOCYCLIC NUCLEOSIDE-CONTAINING OLIGONUCLEOTIDE**

(30) Priority: 01.11.2023 JP 2023187759
(71) Applicant: LIID Pharmaceuticals, Inc., Suita-shi, Osaka 564-8565 (JP)
(72) Inventor: YAMAMOTO, Tsuyoshi, Nagasaki-shi, Nagasaki 852-8521 (JP); TERADA, Chisato, Nagasaki-shi, Nagasaki 852-8521 (JP)
(74) Representative: Weickmann & Weickmann PartmbB
(86) International application number: PCT/JP2024/038873
(87) International publication number: WO 2025/095052

(57) **Abstract**

In the present application, as one embodiment of the invention, an oligonucleotide or a salt thereof that exhibits superior effects on target RNA and the like, while maintaining high safety, and contains, in an oligonucleotide sequence thereof, at least one carbocyclic nucleoside derivative residue (B) which is a divalent group represented by the following formula (B): 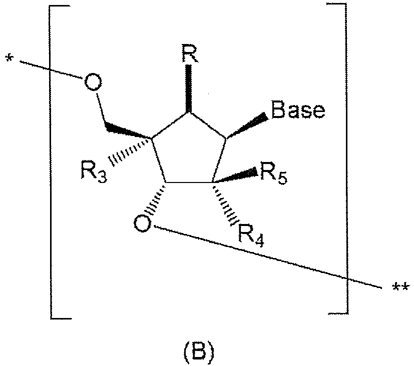 wherein each symbol is as defined in the specification, is disclosed.

## Description

### [Technical Field]

The present invention provides, as an embodiment thereof, an oligonucleotide containing a carbocyclic nucleoside, and the like. More specifically, the present invention provides an oligonucleotide containing a carbocyclic nucleoside that, when introduced into an oligonucleotide, can increase the maximum tolerable dose of the oligonucleotide in the body or enhance its therapeutic index, and is useful, for example, in the field of medicine.

### [Background Art]

In recent years, research and development of nucleic acid drugs has been actively performed. Nucleic acid drugs are drugs that use natural nucleotides or chemically modified nucleotides as the basic skeleton, and the nucleic acids produced by chemical synthesis act directly on body. In addition to high specificity based on base-pair forming, nucleic acid drugs can target molecules that cannot be targeted by conventional drugs, such as mRNA and non-coding RNA, and once the platform is completed, it is easy to standardize in a relatively short time. For this reason, nucleic acid drugs are expected to be applied as next-generation pharmaceutical products following small molecule drugs and antibody drugs.

Such nucleic acid drugs exist in various types depending on the mechanism of action, such as antisense nucleic acids (RNase H-mediated type, steric block type, splicing-regulated type, RNA editing type, etc.), siRNA, aptamers, antigenic nucleic acids, and the like. For example, antisense nucleic acids and siRNA act on RNA in the body, and inhibit or regulate the function thereof to exert therapeutic effects. Aptamers act by utilizing their higher-order structure to bind to targets such as proteins. Antigene nucleic acids are expected to exert therapeutic effects by acting on genomic DNA. Due to these diverse mechanisms of action, application thereof to many genetic diseases and intractable diseases, including neurodegenerative diseases, metabolic diseases, cancer, infectious diseases, and the like, is being developed (see Non Patent Literatures 1-3).

### [Citation List]

### [Non Patent Literature]

[Non Patent Literature 1]
   Stanley T. Crooke, Xue-Hai Liang, Brenda F. Baker, Rosanne M. Crooke, Antisense technology: A review, Journal of Biological Chemistry, Volume 296, 2021, 100416, https://doi.org/10.1016/j.jbc.2021.100416
[Non Patent Literature 2]
   Guillermo Aquino-Jarquin, Novel Engineered Programmable Systems for ADAR-Mediated RNA Editing, Molecular Therapy - Nucleic Acids, Volume 19,2020, Pages 1065-1072M. May Zhang, Raman Bahal, Theodore P. Rasmussen, Jose E. Manautou, Xiao-bo Zhong
[Non Patent Literature 3]
   The growth of siRNA-based therapeutics: Updated clinical studies, Biochemical Pharmacology, Volume 189,2021,114432
[Non Patent Literature 4]
   Guideline for preclinical safety assessment of oligonucleotide therapeutics, PSEHB/PED Notification No. 0330-1, Mar. 30, 2020, https://www.pmda.go.jp/english/review-services/regulatory-info/0003.html
[Non Patent Literature 5]
   Terada C, Kawamoto S, Yamayoshi A, Yamamoto T. Chemistry of Therapeutic Oligonucleotides That Drives Interactions with Biomolecules. Pharmaceutics. 2022 Nov 29; 14(12):2647.
[Non Patent Literature 6]
   Hu, B., Zhong, L., Weng, Y. et al. Therapeutic siRNA: state of the art. Sig Transduct Target Ther 5, 101 (2020). https://doi.org/10.1038/s41392-020-0207-x

### [Summary of Invention]

### [Technical Problem]

However, conventional oligonucleotide therapeutic agents as described above have shown safety problems such as side effects and toxicity due to nonspecific interactions (off-target effects) with proteins and RNA that are not treatment targets in the body (see Non Patent Literatures 4-6). The present invention aims to solve the problems of the conventional techniques and provide an oligonucleotide therapeutic agent that has an increased maximum tolerated dose or an enhanced therapeutic index.

### [Solution to Problem]

The present invention relates to a DNA analog having a carbocyclic structure in which the oxygen atom at the 4' position of the furanose ring of 2'-deoxyguanosine is substituted by an exocyclic double bond as exemplified by Entecavir which is known to have antiviral activity, or in which the oxygen atom is substituted by a spiro ring, namely, a DNA analog having a structure in which the oxygen atom at the 4'-position is substituted by an sp² carbon or a spiro carbon. Based on the novel idea that, when Entecavir or a derivative thereof is introduced into oligonucleotide therapeutic agents, the removal of the 4' position oxygen atom, which is generally important for enzymatic nucleic acid recognition, and the introduction of a large exoolefin structure that can cause steric hindrance to interactions, can reduce nonspecific protein interactions (hybridization-independent interactions), which are known to be the main mechanism of developing adverse reactions of oligonucleotide therapeutic agents, as well as can reduce hybridization-dependent toxicity by affecting the activity of key enzymes such as RNase H, RISC (RNA-Induced Silencing Complex), and ADAR (adenosine deaminase RNA specific), the present inventors have conducted intensive studies in an attempt to solve the above-mentioned problems and found that introduction of a carbocyclic nucleoside derivative (A) represented by the following formula (A) (hereinafter also denoted as "nucleoside (A)") into the nucleotide sequence constituting the oligonucleotide reduces the toxicity of the oligonucleotide (e.g., ASO (antisense oligonucleotide)) compared to that before introduction, and completed the present invention. 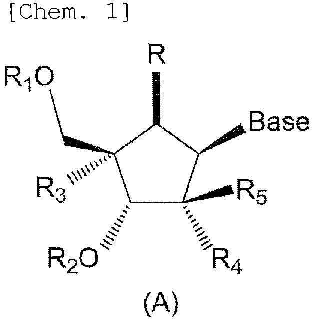 wherein each group and partial structure are respectively as defined for the corresponding group and partial substructure defined for the carbocyclic nucleoside derivative residue represented by the formula (B) in [1] below.

The present invention is described below with reference to specific embodiments. However, the present invention is not limited to these embodiments.
[1] An oligonucleotide or a salt thereof, comprising,
   in an oligonucleotide sequence thereof, at least one carbocyclic nucleoside derivative residue (B) (hereinafter also denoted as "nucleoside residue (B)") which is a divalent group represented by the following formula (B): 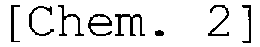 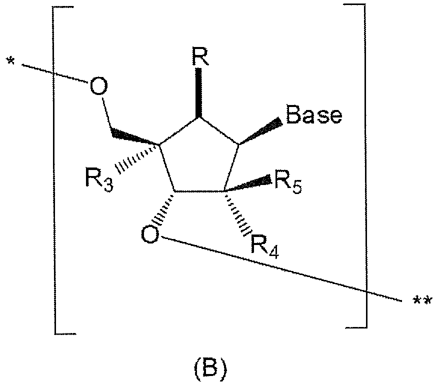 wherein
      Base is a purin-9-yl group or a 2-oxo-1,2-dihydropyrimidin-1-yl group, each optionally having any one or more substituents selected from substituent group (a),
      wherein the substituent group (a) consists of a hydroxyl group, a hydroxyl group protected by a nucleic acid synthesis protecting group, an oxo group, a linear alkyl group having 1 to 6 carbon atoms, a linear alkoxy group having 1 to 6 carbon atoms, a mercapto group, a mercapto group protected by a nucleic acid synthesis protecting group, a linear alkylthio group having 1 to 6 carbon atoms, an amino group, a linear alkylamino group having 1 to 6 carbon atoms, an amino group protected by a nucleic acid synthesis protecting group, and a halogen atom (wherein if the purin-9-yl group or the 2-oxo-1,2-dihydropyrimidin-1-yl group has an oxo group as a substituent selected from substituent group (a), the bond between the carbon atom to which the oxo group is bonded and the adjacent atom is a single bond);
   R₃ and R₄ are each a hydrogen atom;
   R₅ is a hydrogen atom;
   a group represented by the following partial structural formula (i): 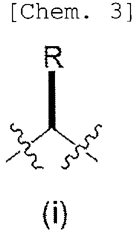
   is a group represented by the following partial structural formula (i-1) or (i-2): 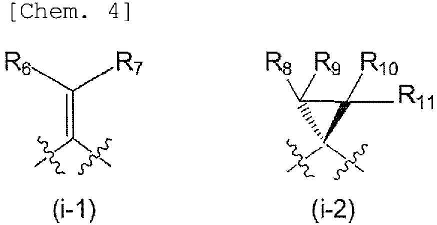 wherein
      R₆ and R₇ are each independently a hydrogen atom, a halogen atom, or an alkyl group having 1 to 6 carbon atoms, or R₆ and R₇ are bonded to each other to form, together with the adjacent carbon atom, a carbocycle having 3 to 6 carbon atoms,
      R₈, R₉, R₁₀ and R₁₁ are each independently a hydrogen atom, a halogen atom, or an alkyl group having 1 to 6 carbon atoms;
   * is a binding site to an adjacent oligonucleotide component, or R₁ if the nucleoside residue (B) is located at the 5' end of the oligonucleotide sequence
      wherein R₁ is a hydrogen atom, a hydroxyl-protecting group in nucleic acid synthesis, an optionally branched or optionally ring-forming alkyl group having 1 to 7 carbon atoms, an optionally branched or optionally ring-forming alkenyl group having 2 to 7 carbon atoms, an aryl group having 3 to 10 carbon atoms, which optionally has any one or more substituents selected from substituent group (a) and which optionally contains heteroatoms, an aralkyl group having an aryl moiety having 3 to 12 carbon atoms, wherein said aryl moiety optionally has any one or more substituents selected from substituent group (a) and optionally contains heteroatoms, an acyl group optionally having any one or more substituents selected from substituent group (a), a silyl group optionally having any one or more substituents selected from substituent group (a), a phosphate group optionally having any one or more substituents selected from substituent group (a), a phosphate group protected by a nucleic acid synthesis protecting group, or -P(R₁₂)R₁₃ wherein R₁₂ and R₁₃ are each independently a hydroxy group, a hydroxyl group protected by a nucleic acid synthesis protecting group, a mercapto group, a mercapto group protected by a nucleic acid synthesis protecting group, an amino group, an alkoxy group having 1 to 6 carbon atoms, an alkylthio group having 1 to 6 carbon atoms, a cyanoalkoxy group having 1 to 6 carbon atoms, or a dialkylamino group having an alkyl group having 1 to 6 carbon atoms; and
   ** is a binding site to an adjacent oligonucleotide component, or R₂ if the nucleoside residue (B) is located at the 3' end of the oligonucleotide sequence
      wherein R₂ is as defined for the aforementioned R₁ (hereinafter also denoted as "oligonucleotide (I)").
[2] The oligonucleotide of the above-mentioned [1], wherein, in the nucleoside residue (B),
   the group represented by the partial structural formula (i): 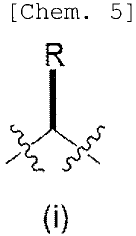
   is a group represented by the following partial structural formula: (i-1): 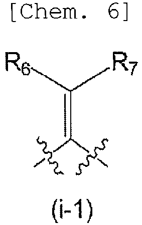
   wherein R₆ and R₇ are each as defined above,
   or a salt thereof.

The structure of the nucleoside residue (B) in this section is shown specifically as follows:

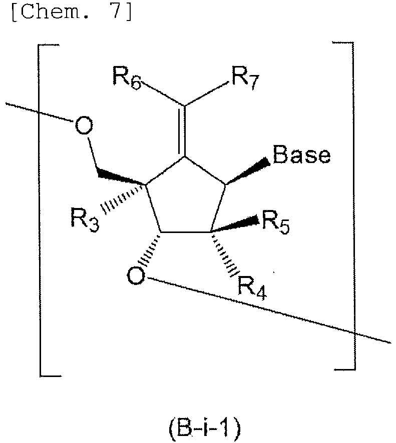

[3] The oligonucleotide or a salt thereof of the above-mentioned [1], wherein, in the nucleoside residue (B),
the group represented by the partial structural formula (i): 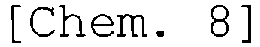 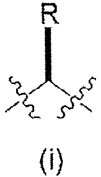
is a group represented by the following partial structural formula (i-2): 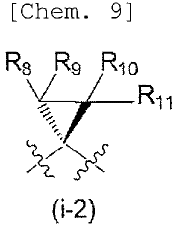
wherein R₈, R₉, R₁₀ and R₁₁ are each as defined above.

The structure of the nucleoside residue (B) in this section is shown specifically as follows:

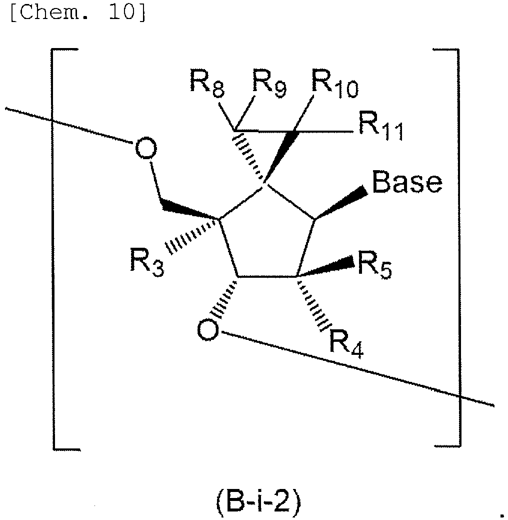

[4] The oligonucleotide or a salt thereof of any of the above-mentioned [1] to [3], wherein the oligonucleotide sequence comprises 1 to 10 nucleoside residues (B).
[5] The oligonucleotide or a salt thereof of any of the above-mentioned [1] to [4], wherein the oligonucleotide is 7 to 30 bases in length.
[6] The oligonucleotide or a salt thereof of any of the above-mentioned [1] to [5], wherein the oligonucleotide is 10 to 25 bases in length.
[7] The oligonucleotide or a salt thereof of any of the above-mentioned [1] to [6], wherein toxicity (e.g., hepatotoxicity and/or weight loss effect) is reduced compared to before the introduction of nucleoside residue (B).
[8] The oligonucleotide or a salt thereof of any of the above-mentioned [1] to [7], wherein the oligonucleotide is a gapmer consisting of a gap region of 2 to 14 bases in length, a 5'-wing region of 2 to 5 bases in length, and a 3'-wing region of 2 to 5 bases in length, and the gap region is located between the 5'-wing region and the 3'-wing region.
[9] The oligonucleotide or a salt thereof of the above-mentioned [8], wherein the gap region comprises at least one nucleoside residue (B).
[10] The oligonucleotide or a salt thereof of the above-mentioned [8] or [9], wherein the 5'-wing region and/or 3'-wing region contain at least one nucleoside residue (B).
[11] The oligonucleotide or a salt thereof of any of the above-mentioned [1] to [10], wherein at least one of internucleotide linkages in the oligonucleotide is a phosphorothioate linkage.
[12] The oligonucleotide or a salt thereof of any of the above-mentioned [1] to [11], wherein all of internucleotide linkages in the oligonucleotide are phosphorothioate linkages.
[13] The oligonucleotide or a salt thereof of any of the above-mentioned [1] to [12], wherein, in the nucleoside residue (B), Base is a purin-9-yl group or a 2-oxo-1,2-dihydropyrimidin-1-yl group, each optionally having any 1 to 3 substituents selected from substituent group (a),
   wherein the substituent group (a) consists of a hydroxyl group, a hydroxyl group protected by a nucleic acid synthesis protecting group, an oxo group, a linear alkyl group having 1 to 6 carbon atoms, a linear alkoxy group having 1 to 6 carbon atoms, a mercapto group, a mercapto group protected by a nucleic acid synthesis protecting group, a linear alkylthio group having 1 to 6 carbon atoms, an amino group, a linear alkylamino group having 1 to 6 carbon atoms, an amino group protected by a nucleic acid synthesis protecting group, and a halogen atom.
[14] The oligonucleotide or a salt thereof of the above-mentioned [1], [2], [4] to [13], wherein, in the nucleoside residue (B),
   R₆ and R₇ are hydrogen atoms;
   * is a binding site to an adjacent oligonucleotide component, or a hydrogen atom if the nucleoside residue (B) is located at the 5' end of the oligonucleotide sequence; and
   ** is a binding site to an adjacent oligonucleotide component, or a hydrogen atom if the nucleoside residue (B) is located at the 3' end of the oligonucleotide sequence.
[15] The oligonucleotide or a salt thereof of the above-mentioned [1], [3] to [13], wherein, in the nucleoside residue (B),
   R₈, R₉, R₁₀ and R₁₁ are each a hydrogen atom;
   * is a binding site to an adjacent oligonucleotide component, or a hydrogen atom if the nucleoside residue (B) is located at the 5' end of the oligonucleotide sequence; and
   ** is a binding site to an adjacent oligonucleotide component, or a hydrogen atom if the nucleoside residue (B) is located at the 3' end of the oligonucleotide sequence.
[16] Use of the oligonucleotide or a salt thereof of any of the above-mentioned [1] to [15], as an antisense oligonucleotide, or as an oligonucleotide constituting siRNA.
[17] A method for reducing the toxicity of an oligonucleotide or a salt thereof, comprising introducing at least one carbocyclic nucleoside derivative residue (B) ("nucleoside residue (B)"), which is a divalent group represented by the following formula (B) 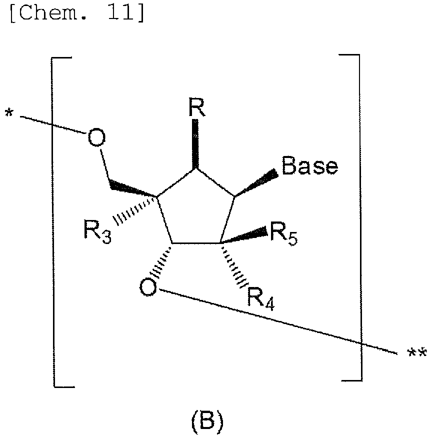 wherein each group and partial structure are as defined for the corresponding groups defined for "nucleoside residue (B)" in the above-mentioned [1], into the oligonucleotide sequence.
[18] Use of a carbocyclic nucleoside derivative represented by the following formula (A) ("nucleoside (A)") or a salt thereof, for reducing the toxicity of an oligonucleotide 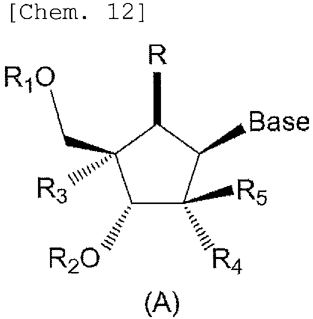 wherein each group and partial structure are as defined for the corresponding groups defined for "nucleoside residue (B)" in the above-mentioned [1].
[19] The use of the above-mentioned [18], wherein the reduction of the toxicity of the oligonucleotide comprises introducing at least one nucleoside (A) into the oligonucleotide sequence.
[20] A medicament comprising the oligonucleotide or a salt thereof of any of the above-mentioned [1] to [16] as an active ingredient.

### [Advantageous Effects of Invention]

According to the present invention, in one embodiment, an oligonucleotide that exhibits superior effects on target RNA and the like, while maintaining high safety, is provided.

### [Brief Description of Drawings]

[Fig. 1]

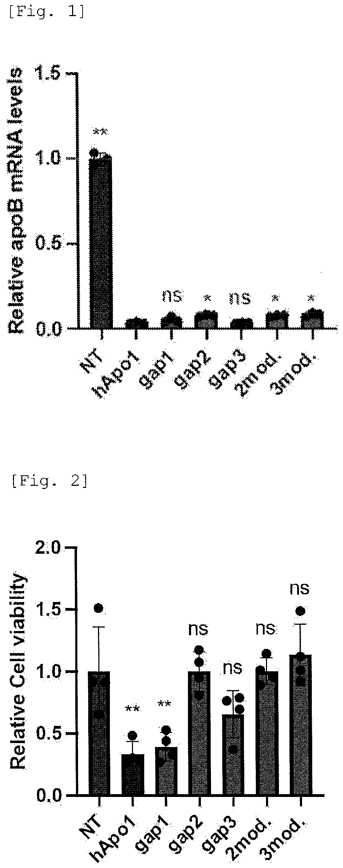

   Fig. 1 shows the evaluation results of the target gene expression suppressive effect in Example 5 described later.
[Fig. 2]
   Fig. 2 shows the evaluation results of the cytotoxicity (cell survival rate) in Example 6 described later.
[Fig. 3]

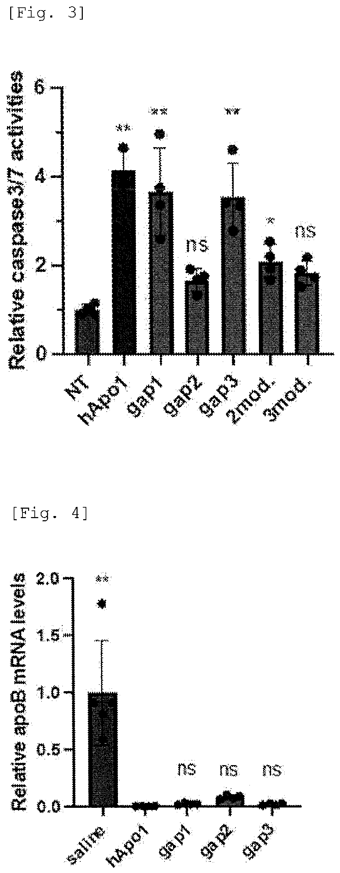

   Fig. 3 shows the evaluation results of the cytotoxicity (Caspase 3/7 activity) in Example 7 described later.
[Fig. 4]
   Fig. 4 shows the evaluation results of the target gene expression suppressive effect in Example 9 described later.
[Fig. 5]

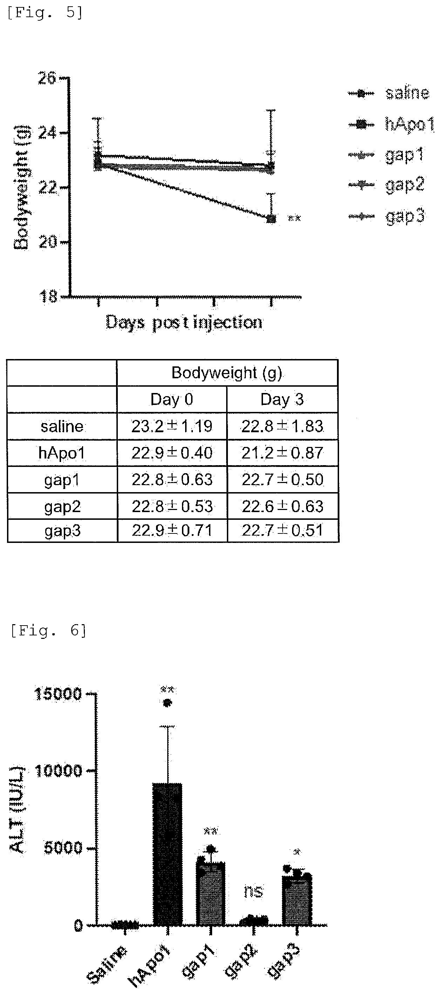

   Fig. 5 shows the evaluation results of the body weight change in Example 10 described later.
[Fig. 6]
   Fig. 6 shows the evaluation results of ALT in Example 10 described later.
[Fig. 7]

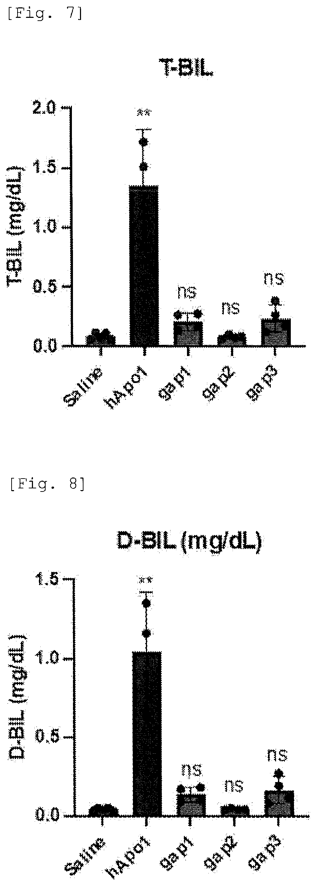

   Fig. 7 shows the evaluation results of total bilirubin in Example 10 described later.
[Fig. 8]
   Fig. 8 shows the evaluation results of direct bilirubin in Example 10 described later.
[Fig. 9]

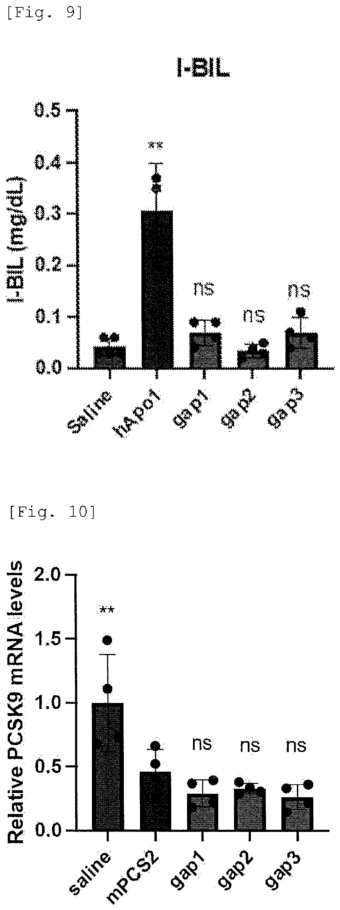

   Fig. 9 shows the evaluation results of indirect bilirubin in Example 10 described later.
[Fig. 10]
   Fig. 10 shows the evaluation results of the target gene expression suppressive effect in Example 12 described later.
[Fig. 11]

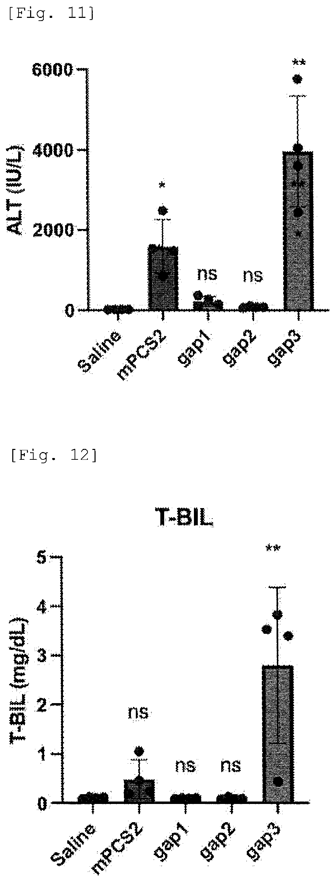

   Fig. 11 shows the evaluation results of ALT in Example 13 described later.
[Fig. 12]
   Fig. 12 shows the evaluation results of total bilirubin in Example 13 described later.
[Fig. 13]

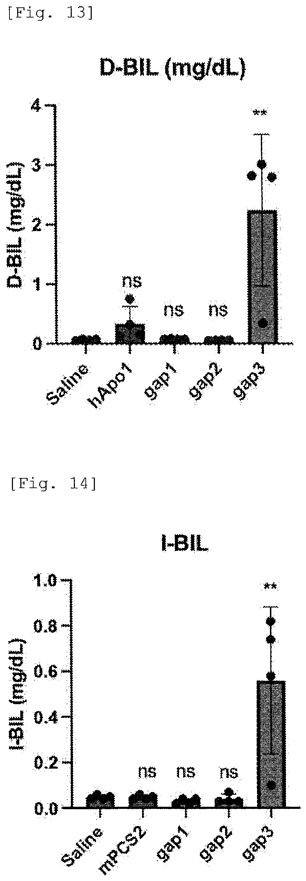

   Fig. 13 shows the evaluation results of direct bilirubin in Example 13 described later.
[Fig. 14]
   Fig. 14 shows the evaluation results of indirect bilirubin in Example 13 described later.
[Fig. 15]

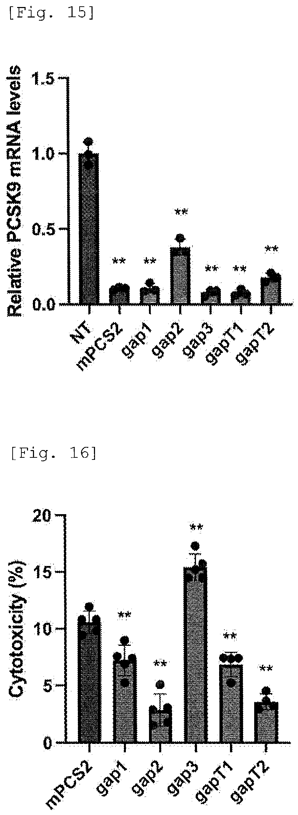

   Fig. 15 shows the evaluation results of the target gene expression suppressive effect in Example 15 described later.
[Fig. 16]
   Fig. 16 shows the evaluation results of the cytotoxicity (cell survival rate) in Example 16 described later.
[Fig. 17]

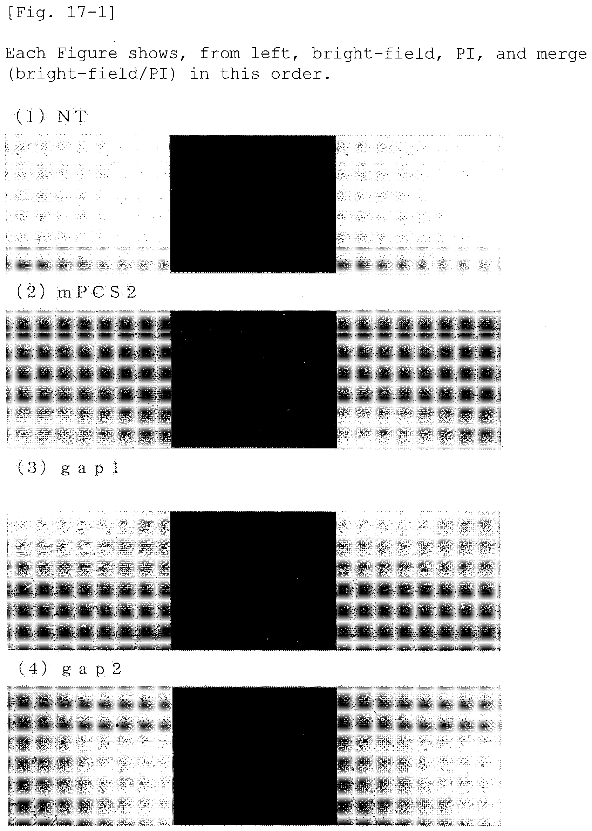

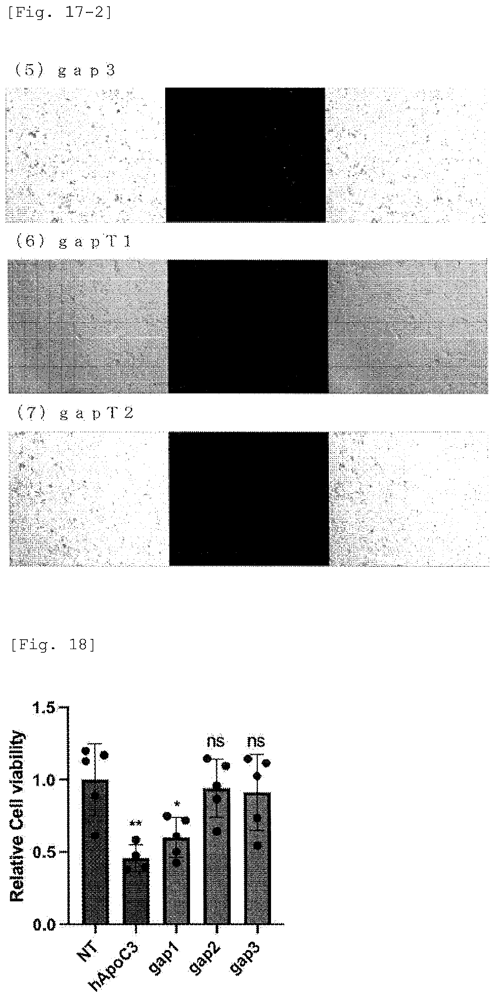

   Fig. 17 shows the evaluation results of the cytotoxicity (cell survival rate) in Example 17 described later.
[Fig. 18]
   Fig. 18 shows the evaluation results of the cytotoxicity (cell survival rate) in Example 19 described later.
[Fig. 19]

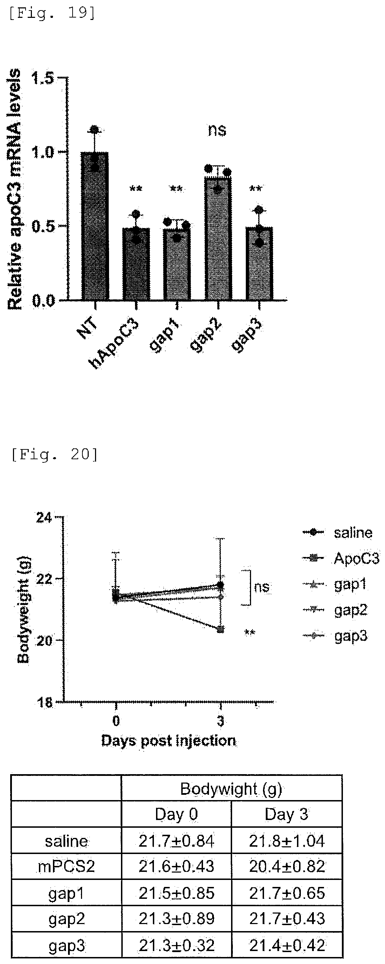

   Fig. 19 shows the evaluation results of the target gene expression suppressive effect in Example 20 described later.
[Fig. 20]
   Fig. 20 shows the evaluation results of the body weight change in Example 21 described later.
[Fig. 21]

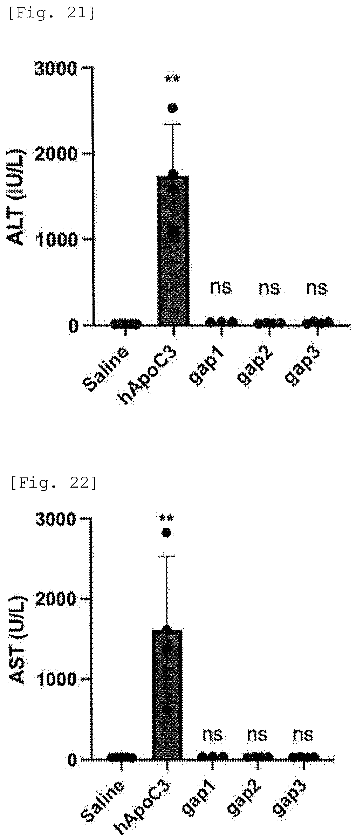

   Fig. 21 shows the evaluation results of ALT in Example 21 described later.
[Fig. 22]
   Fig. 22 shows the evaluation results of AST in Example 21 described later.
[Fig. 23]

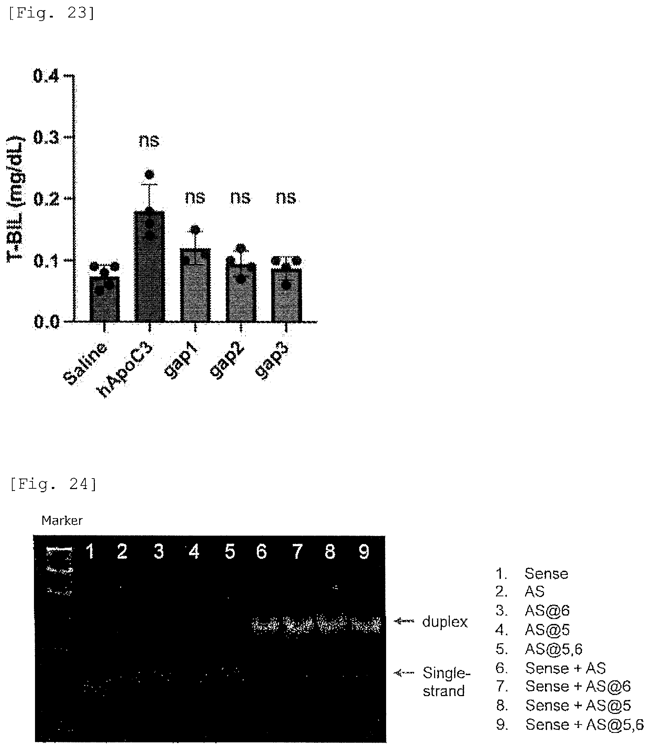

   Fig. 23 shows the evaluation results of total bilirubin in Example 21 described later.
[Fig. 24]
   Fig. 24 shows the evaluation results of the double-strand formation ability of siRNA incorporating an Entecavir derivative in the antisense strand in Example 41 described later.
[Fig. 25]

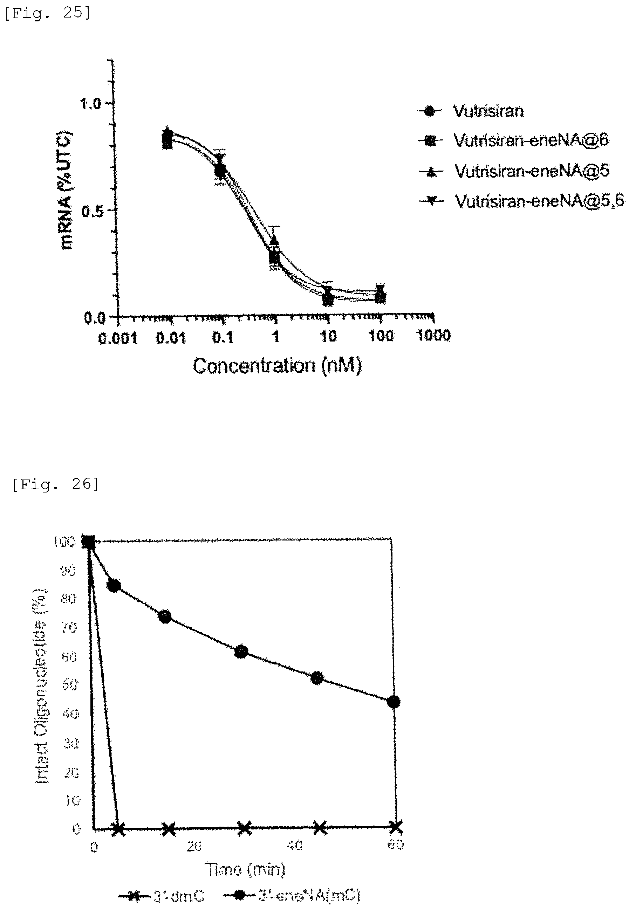

   Fig. 25 shows the evaluation results of the in vitro target gene expression suppressive effect of siRNA incorporating an Entecavir derivative in the AS strand in Example 42 described later.
[Fig. 26]
   Fig. 26 shows the evaluation results of stability in Example 56 described later.
[Fig. 27]

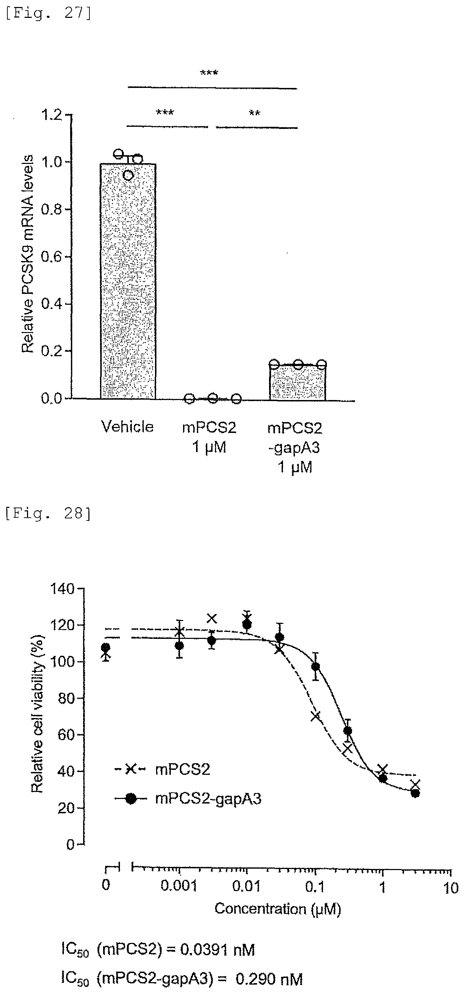

   Fig. 27 shows the evaluation results of the in vitro target gene expression suppressive effect of the oligonucleotide drug in Example 57 described later.
[Fig. 28]
   Fig. 28 shows the evaluation results of the cytotoxicity (cell survival rate) of the oligonucleotide drug in Example 58 described later.

### [Description of Embodiments]

The present invention is described in detail below. Unless otherwise specified in the text, all technical terms and scientific terms used in the present specification have the same meaning as those generally understood by those of ordinary skill in the art to which the present invention pertains. Any methods and materials similar or equivalent to those described in the present specification can be used in the practice or testing of the present invention, and preferred methods and materials are described below. All publications and patents mentioned in the present specification are incorporated herein by reference, for the purpose of, for example, describing and disclosing the constructs and methodologies described in the publications that may be used in connection with the described invention.

### [Oligonucleotide (I)]

The present invention provides, in one embodiment,
"[1] An oligonucleotide or a salt thereof, containing in an oligonucleotide sequence thereof, at least one carbocyclic nucleoside derivative residue (B) ("nucleoside residue (B)") which is a divalent group represented by the following formula (B) 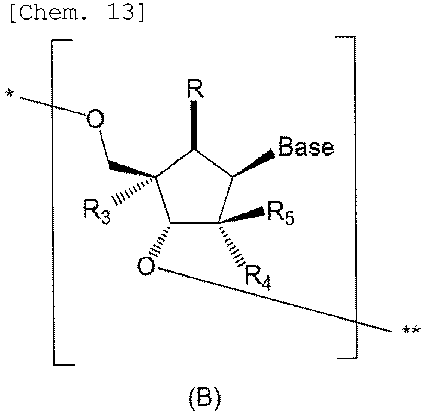 wherein each group and partial structure are respectively as defined for the corresponding group and partial structure defined for "nucleoside residue (B)" in [1] in the above-mentioned [Means of Solving the Problems] section. The oligonucleotide (I) is described in detail below.

As described above, oligonucleotide (I) is composed of (1) a nucleoside residue (B) and (2) a nucleoside (or nucleoside derivative) selected according to the oligonucleotide sequence of interest.

### (1) Nucleoside residue (B)

First, "nucleoside residue (B)" is described in detail. The definition of each group of nucleoside residue (B) is as described above, and preferred embodiments thereof are as follows.

In the present specification, the notation "Ca-b" (e.g., C1-6) or "Ca-Cb" (e.g., C1-C6) shows that the number of carbon atoms constituting the group is a to b (e.g., 1 to 6).

In the present specification, as the "linear alkyl group having 1 to 6 carbon atoms", a linear alkyl group having 1 to 6 carbon atoms can be mentioned, and specifically, for example, methyl, ethyl, n-propyl, n-butyl, n-pentyl, n-hexyl and the like can be mentioned. On the other hand, as the "alkyl group having 1 to 6 carbon atoms", any linear, branched or cyclic alkyl group having 1 to 6 carbon atoms can be mentioned, and specifically, in addition to those mentioned above, for example, branched alkyl groups such as isopropyl, isobutyl, tert-butyl, isopentyl and the like, and any cyclic alkyl groups having 3 to 6 carbon atoms such as cyclopropyl, cyclobutyl, cyclopentyl, cyclohexyl and the like can be mentioned.

In the present specification, as the "linear alkoxy group having 1 to 6 carbon atoms", a linear alkoxy group having 1 to 6 carbon atoms can be mentioned, and specifically, for example, methoxy, ethoxy, n-propoxy and the like can be mentioned. On the other hand, as the "alkoxy group having 1 to 6 carbon atoms", any linear, branched or cyclic alkoxy group having 1 to 6 carbon atoms can be mentioned, and specifically, in addition to those mentioned above, for example, branched alkoxy groups such as isopropoxy, isobutoxy, tert-butoxy, isopentyloxy and the like, and any cyclic alkoxy groups having 3 to 6 carbon atoms such as cyclopropyloxy, cyclobutyloxy, cyclopentyloxy, cyclohexyloxy and the like can be mentioned.

In addition, the "linear alkoxy group having 1 to 6 carbon atoms optionally substituted by a linear alkoxy group having 1 to 6 carbon atoms" refers to the above-mentioned "linear alkoxy group having 1 to 6 carbon atoms", or an alkoxy group, in which one or more hydrogen atoms constituting the "linear alkoxy group having 1 to 6 carbon atoms" is/are substituted by another, the same or different, "linear alkoxy group having 1 to 6 carbon atoms". Examples of such "linear alkoxy group having 1 to 6 carbon atoms optionally substituted by a linear alkoxy group having 1 to 6 carbon atoms" include methoxy group, ethoxy group, n-propoxy group, methoxymethoxy group, ethoxymethoxy group, n-propoxymethoxy group, methoxyethoxy group (e.g., 2-methoxyethoxy group), ethoxyethoxy group (e.g., 2-ethoxyethoxy group), n-propoxyethoxy group and the like.

In the present specification, as the "cyanoalkoxy group having 1 to 6 carbon atoms", a group in which at least one hydrogen atom in any linear, branched, or cyclic alkoxy group having 1 to 6 carbon atoms is substituted by a cyano group can be mentioned.

In the present specification, as the "linear alkylthio group having 1 to 6 carbon atoms", an alkylthio group having any linear alkyl group having 1 to 6 carbon atoms can be mentioned. For example, a methylthio group, an ethylthio group, an n-propylthio group, and the like can be mentioned. On the other hand, as the "alkylthio group having 1 to 6 carbon atoms", any linear, branched, or cyclic alkylthio group having 1 to 6 carbon atoms can be mentioned.

In the present specification, the "linear alkylamino group having 1 to 6 carbon atoms" includes an amino group having any one or two linear alkyl groups having 1 to 6 carbon atoms. For example, a methylamino group, a dimethylamino group, an ethylamino group, a methylethylamino group, a diethylamino group, and the like can be mentioned.

In the present specification, as the "an optionally branched or optionally ring-forming alkyl group having 1 to 7 carbon atoms", any linear alkyl group having 1 to 7 carbon atoms, any branched alkyl group having 3 to 7 carbon atoms, and any cyclic alkyl group having 3 to 7 carbon atoms can be mentioned. It is sometimes simply referred to as "C1-7 alkyl group". For example, as any linear alkyl group having 1 to 7 carbon atoms, methyl group, ethyl group, n-propyl group, n-butyl group, n-pentyl group, n-hexyl group, and n-heptyl group can be mentioned. As any branched alkyl group having 3 to 7 carbon atoms, isopropyl group, isobutyl group, tert-butyl group, isopentyl group and the like can be mentioned, and as any cyclic alkyl group having 3 to 7 carbon atoms, cyclobutyl group, cyclopentyl group, cyclohexyl group and the like can be mentioned.

In the present specification, the "an optionally branched or optionally ring-forming alkenyl group having 2 to 7 carbon atoms" includes any linear alkenyl group having 2 to 7 carbon atoms, any branched alkenyl group having 3 to 7 carbon atoms, and any cyclic alkenyl group having 3 to 7 carbon atoms. It may also be simply referred to as "a C2-7 alkenyl group". For example, any linear alkenyl group having 2 to 7 carbon atoms includes ethenyl group, 1-propenyl group, 2-propenyl group, 1-butenyl group, 2-butenyl group, 1-pentenyl group, 2-pentenyl group, 3-pentenyl group, 4-pentenyl group, 1-hexenyl group and the like. The any branched alkenyl group having 3 to 7 carbon atoms includes, for example, isopropenyl group, 1-methyl-1-propenyl group, 1-methyl-2-propenyl group, 2-methyl-1-propenyl group, 2-methyl-2-propenyl group, 1-methyl-2-butenyl group and the like. The any cyclic alkenyl group having 3 to 7 carbon atoms includes, for example, cyclobutenyl group, cyclopentenyl group, cyclohexenyl group and the like.

In the present specification, as the "carbocycle having 3 to 6 carbon atoms", cyclopropane, cyclobutane, cyclopentane, and cyclohexane can be mentioned.

In the present specification, as the "aryl group having 3 to 10 carbon atoms which may contain heteroatoms", any aryl group having 6 to 10 carbon atoms composed solely of hydrocarbons, and any heteroaryl group having 3 to 12 carbon atoms in which at least one carbon atom constituting the ring structure of the aryl group is substituted by a heteroatom (e.g., nitrogen atom, oxygen atom, and sulfur atom, and combinations thereof) can be mentioned. Examples of the aryl group having 6 to 10 carbon atoms include phenyl group, naphthyl group, indenyl group, azulenyl group and the like, and examples of the heteroaryl group having 3 to 12 carbon atoms include pyridyl group, pyrrolyl group, quinolyl group, indolyl group, imidazolyl group, furyl group, thienyl group and the like.

In the present specification, examples of the "aralkyl group having an aryl moiety having 3 to 12 carbon atoms, which may contain heteroatoms" include benzyl group, phenethyl group, naphthylmethyl group, 3-phenylpropyl group, 2-phenylpropyl group, 4-phenylbutyl group, 2-phenylbutyl group, pyridylmethyl group, indolylmethyl group, furylmethyl group, thienylmethyl group, pyrrolylmethyl group, 2-pyridylethyl group, 1-pyridylethyl group, 3-thienylpropyl group, and the like.

In the present specification, as the "acyl group", aliphatic acyl group and aromatic acyl group can be mentioned.

Specifically, examples of the aliphatic acyl group include alkylcarbonyl groups such as formyl group, acetyl group, propionyl group, butyryl group, isobutyryl group, pentanoyl group, pivaloyl group, valeryl group, isovaleryl group, octanoyl group, nonanoyl group, decanoyl group, 3-methylnonanoyl group, 8-methylnonanoyl group, 3-ethyloctanoyl group, 3,7-dimethyloctanoyl group, undecanoyl group, dodecanoyl group, tridecanoyl group, tetradecanoyl group, pentadecanoyl group, hexadecanoyl group, 1-methylpentadecanoyl group, 14-methylpentadecanoyl group, 13,13-dimethyltetradecanoyl group, heptadecanoyl group, 15-methylhexadecanoyl group, octadecanoyl group, 1-methylheptadecanoyl group, nonadecanoyl group, eicosanoyl group and heneicosanoyl group; carboxylated alkylcarbonyl groups such as succinoyl group, glutaroyl group, and adipoyl group; halogeno C1-6 alkylcarbonyl groups such as chloroacetyl group, dichloroacetyl group, trichloroacetyl group, and trifluoroacetyl group; C1-6 alkoxyC1-6 alkylcarbonyl group such as methoxyacetyl group; and unsaturated alkylcarbonyl groups such as (E)-2-methyl-2-butenoyl group.

Examples of the aromatic acyl group include arylcarbonyl groups such as benzoyl group, α-naphthoyl group, and β-naphthoyl group; halogenoarylcarbonyl groups such as 2-bromobenzoyl group and 4-chlorobenzoyl group; C1-6 alkylated arylcarbonyl groups such as 2,4,6-trimethylbenzoyl group and 4-toluoyl group; C1-6 alkoxylated arylcarbonyl groups such as 4-anisoyl group; carboxylated arylcarbonyl groups such as 2-carboxybenzoyl group, 3-carboxybenzoyl group, and 4-carboxybenzoyl group; nitrated arylcarbonyl groups such as 4-nitrobenzoyl group and 2-nitrobenzoyl group; C1-6 alkoxycarbonylated arylcarbonyl groups such as 2-(methoxycarbonyl)benzoyl group; arylated arylcarbonyl groups such as 4-phenylbenzoyl group, and the like. Preferred are formyl group, acetyl group, propionyl group, butyryl group, isobutyryl group, pentanoyl group, pivaloyl group, and benzoyl group.

In the present specification, as the "silyl group", triC1-6 alkylsilyl groups such as trimethylsilyl group, triethylsilyl group, isopropyldimethylsilyl group, t-butyldimethylsilyl group, methyldiisopropylsilyl group, methyldi-t-butylsilyl group, triisopropylsilyl group; and triC1-6 alkylsilyl groups substituted by one or two aryl groups such as diphenylmethylsilyl group, butyldiphenylbutylsilyl group, diphenylisopropylsilyl group, phenyldiisopropylsilyl group can be mentioned. Preferred are trimethylsilyl group, triethylsilyl group, triisopropyl silyl group, t-butyldimethylsilyl group, and t-butyldiphenylsilyl group, and further preferred is trimethylsilyl group.

In the present specification, examples of the "halogen atom" include fluorine, chlorine, bromine, and iodine. Fluorine atom or chlorine atom is preferred.

In the present specification, the "protecting group" in the "amino-protecting group in nucleic acid synthesis", "hydroxyl-protecting group in nucleic acid synthesis", "hydroxyl group protected by a nucleic acid synthesis protecting group", "phosphate group protected by a nucleic acid synthesis protecting group", "mercapto group protected by a nucleic acid synthesis protecting group" is not particularly limited as long as it can stably protect an amino group, hydroxyl group, phosphate group, or mercapto group during nucleic acid synthesis. Specifically, it refers to a protecting group that is stable under acidic or neutral conditions and can be cleaved by chemical methods such as hydrogenolysis, hydrolysis, electrolysis, and photolysis. Examples of such protecting group include C1-6 alkyl group, C1-6 alkenyl group, acyl group, tetrahydropyranyl or tetrahydrothiopyranyl group, tetrahydrofuranyl or tetrahydrothiofuranyl group, silyl group, C1-6 alkoxymethyl group, C1-6 alkoxylated C1-6 alkoxymethyl group, halogeno C1-6 alkoxymethyl group, C1-6 alkoxylated ethyl group, halogenated ethyl group, methyl group substituted by 1 to 3 aryl groups, "methyl group substituted by 1 to 3 aryl groups in which aryl ring is substituted by C1-6 alkyl group, C1-6 alkoxy group, halogen atom or cyano group", C1-6 alkoxycarbonyl group, "aryl group substituted by halogen atom, C1-6 alkoxy group or nitro group", "C1-6 alkoxycarbonyl group substituted by halogen atom or triC1-6 alkylsilyl group", alkenyloxycarbonyl group, "aralkyloxycarbonyl group in which aryl ring is optionally substituted by a C1-6 alkoxy or nitro group", and the like.

More specifically, as the tetrahydropyranyl group or tetrahydrothiopyranyl group, tetrahydropyran-2-yl group, 3-bromotetrahydropyran-2-yl group, 4-methoxytetrahydropyran-4-yl group, tetrahydrothiopyran-4-yl group, 4-methoxytetrahydrothiopyran-4-yl group and the like can be mentioned. As the tetrahydrofuranyl group or tetrahydrothiofuranyl group, tetrahydrofuran-2-yl group and tetrahydrothiofuran-2-yl group can be mentioned. As the C1-6 alkoxymethyl group, methoxymethyl group, 1,1-dimethyl-1-methoxymethyl group, ethoxymethyl group, propoxymethyl group, isopropoxymethyl group, butoxy methyl group, t-butoxy methyl group and the like can be mentioned. As the C1-6 alkoxylated C1-6 alkoxymethyl group, 2-methoxyethoxymethyl group and the like can be mentioned. As the halogeno C1-6 alkoxymethyl group, 2,2,2-trichloroethoxymethyl group, bis(2-chloroethoxy)methyl group and the like can be mentioned. As the C1-6 alkoxylated ethyl group, 1-ethoxyethyl group, 1-(isopropoxy)ethyl group and the like can be mentioned. As the halogenated ethyl group, 2,2,2-trichloroethyl group and the like can be mentioned. As the methyl group substituted by 1 to 3 aryl groups, benzyl group, α-naphthylmethyl group, β-naphthylmethyl group, diphenylmethyl group, triphenyl methyl group, α-naphthyldiphenylmethyl group, 9-anthrylmethyl group and the like can be mentioned. As the "methyl group substituted by 1 to 3 aryl groups in which aryl ring is substituted by C1-6 alkyl group, C1-6 alkoxy group, halogen atom or cyano group", 4-methylbenzyl group, 2,4,6-trimethyl benzyl group, 3,4,5-trimethyl benzyl group, 4-methoxybenzyl group, 4-methoxyphenyldiphenylmethyl group, 4,4'-dimethoxytriphenyl methyl group, 2-nitrobenzyl group, 4-nitrobenzyl group, 4-chlorobenzyl group, 4-bromobenzyl group, 4-cyanobenzyl group and the like can be mentioned. As the C1-6 alkoxycarbonyl group, methoxycarbonyl group, ethoxycarbonyl group, t-butoxycarbonyl group, isobutoxycarbonyl group and the like can be mentioned. As the "aryl group substituted by a halogen atom, a C1-6 alkoxy group or a nitro group", 4-chlorophenyl group, 2-chlorophenyl group, 4-methoxyphenyl group, 4-nitrophenyl group, 2,4-dinitrophenyl group and the like can be mentioned. As the "C1-6 alkoxycarbonyl group substituted by a halogen atom or triC1-6 alkylsilyl group", 2,2,2-trichloroethoxycarbonyl group, 2-trimethylsilyl ethoxycarbonyl group and the like can be mentioned. As the alkenyloxycarbonyl group, vinyloxycarbonyl group, aryloxycarbonyl group and the like can be mentioned. As the "aralkyloxycarbonyl group in which aryl ring is optionally substituted by a C1-6 alkoxy or nitro group", benzyloxycarbonyl group, 4-methoxybenzyloxycarbonyl group, 3,4-dimethoxybenzyloxycarbonyl group, 2-nitrobenzyloxycarbonyl group, 4-nitrobenzyloxycarbonyl group and the like can be mentioned.

In one embodiment, examples of the "hydroxyl-protecting group in nucleic acid synthesis" include aliphatic acyl group, aromatic acyl group, methyl group substituted by 1 to 3 aryl groups, "methyl group substituted by 1 to 3 aryl groups in which aryl ring is substituted by C1-6 alkyl group, C1-6 alkoxy group, halogen or cyano group", and silyl group. Alternatively, in another embodiment, examples of the "hydroxyl-protecting group in nucleic acid synthesis" include acetyl group, benzoyl group, benzyl group, p-methoxybenzoyl group, dimethoxytrityl group, monomethoxytrityl group, tert-butyldiphenylsilyl group, tert-butyldimethylsilyl (TBDMS) group, [(triisopropyl silyl)oxy]methyl (TOM) group, [(2-nitrobenzyl)oxy]methyl (NBOM) group, bis(acetoxyethoxy)methylether (ACE) group, tetrahydro-4-methoxy-2H-pyran-2-yl (Mthp) group, 1-(2-cyanoethoxy)ethyl (CEE) group, 2-cyanoethoxymethyl (CEM) group, tert-butyldithiomethyl (DTM) group, 2-(4-tolylsulfonyl)ethoxymethyl (TEM) group, and 4-(N-dichloroacetyl-N-methylamino)benzyloxymethyl (4-MABOM) group.

In one embodiment, examples of the protecting group of the "hydroxyl group protected by a nucleic acid synthesis protecting group" include aliphatic acyl group, aromatic acyl group, "methyl group substituted by 1 to 3 aryl groups", "aryl group substituted by halogen atom, C1-6 alkoxy group or nitro group", C1-6 alkyl group, and C1-6 alkenyl group. Alternatively, in another embodiment, examples of the protecting group of the "hydroxyl group protected by a nucleic acid synthesis protecting group" include benzoyl group, benzyl group, 2-chlorophenyl group, 4-chlorophenyl group, and 2-propenyl group.

In one embodiment, examples of the "amino -protecting group in nucleic acid synthesis" include acyl group, preferably benzoyl group.

In one embodiment, examples of the protecting group of the "phosphate group protected by a nucleic acid synthesis protecting group" include C1-6 alkyl group, C1-6 alkyl group substituted by cyano group, aralkyl group, "aralkyl group in which aryl ring is substituted by nitro group or halogen atom", and "aryl group substituted by C1-6 alkyl group, halogen atom, or nitro group". Alternatively, in one embodiment, examples of the protecting group of the "phosphate group protected by a nucleic acid synthesis protecting group" include 2-cyanoethyl group, 2,2,2-trichloroethyl group, benzyl group, 2-chlorophenyl group, and 4-chlorophenyl group.

In one embodiment, examples of the protecting group of the "mercapto group protected by a nucleic acid synthesis protecting group" include aliphatic acyl group and aromatic acyl group, preferably benzoyl group.

In the present specification, among the groups represented by -P(R₁₂)R₁₃ wherein R₁₂ and R₁₃ are each independently a hydroxy group, a hydroxyl group protected by a nucleic acid synthesis protecting group, a mercapto group, a mercapto group protected by a nucleic acid synthesis protecting group, an amino group, an alkoxy group having 1 to 6 carbon atoms, an alkylthio group having 1 to 6 carbon atoms, a cyanoalkoxy group having 1 to 6 carbon atoms, or a dialkylamino group having an alkyl group having 1 to 6 carbon atoms, a group in which R₁₂ is OR₁₂ₐ and R₁₃ is NR₁₃ₐ is called a "phosphoramidite group" (wherein R₁₂ₐ is, for example, a C1-C6 cyanoalkoxy group, and R₁₃ₐ is, for example, an alkyl group having 1 to 6 carbon atoms). The phosphoramidite group is preferably a group represented by the formula - P(OC₂H₄CN)(N(iPr)₂), or a group represented by the formula - P(OCH₃)(N(iPr)₂). Here, iPr is an isopropyl group.

### (2) Nucleoside (or nucleoside derivative) of interest or that selected according to oligonucleotide sequence

In the present specification, "nucleoside" and "nucleoside analog" are not particularly limited and refer to those generally used in the art, and refer to unnatural types of "nucleosides" in which a purine or pyrimidine base is bonded to a sugar, and those in which an aromatic heterocycle or aromatic hydrocarbon ring other than purine and pyrimidine, which can be substituted for a purine or pyrimidine base, is bonded to a sugar.

In practicing the present invention, those skilled in the art can appropriately select and use them according to the sequence of the oligonucleotide (I) of interest.

In the present specification, oligonucleotide (I) also includes "artificial oligonucleotides" and "oligonucleotide analogs." As used herein, the "artificial oligonucleotides" and "oligonucleotide analogs" refer to unnatural derivatives of "oligonucleotides" in which, for example, 2 to 50 the same or different "nucleosides" or "nucleoside analogs" are linked by phosphate diester bonds. Preferred examples of such analogs include sugar derivatives in which the sugar moiety is modified; thioate derivatives in which the phosphate diester moiety is thioated; esters in which the terminal phosphate moiety is esterified; and amides in which the amino group on the purine base is amidated. More preferably, sugar derivatives in which the sugar moiety is modified can be mentioned.

In the present specification, examples of salts of oligonucleotide (I) include alkali metal salts such as sodium salt, potassium salt, and lithium salt, alkaline earth metal salts such as calcium salt and magnesium salt, metal salts including aluminum salt, iron salt, zinc salt, copper salt, nickel salt, and cobalt salt; amine salts including inorganic salts such as ammonium salt, and organic salts such as t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenethylamine salt, piperazine salt, tetramethylammonium salt, and tris(hydroxymethyl)aminomethane salt; salts with inorganic acid including halogenated hydrochloride salts such as hydrofluoride salt, hydrochloride, hydrobromide, and hydroiodide, nitrate, perchlorate, sulfate, and phosphate; salts with organic acids including C1-6 alkanesulfonates such as methanesulfonate, trifluoromethanesulfonate, and ethanesulfonate, arylsulfonates such as benzenesulfonate and p-toluenesulfonate, acetates, malates, fumarates, succinates, citrates, tartrates, oxalates, and maleates; and salts with amino acids such as glycine salt, lysine salts, arginine salts, ornithine salts, glutamates, and aspartates.

The following describes in detail the preferred embodiments of the nucleoside residue (B) in oligonucleotide (I).

### Nucleoside residue (B) (I)

A nucleoside residue (B), wherein
Base is a purin-9-yl group or a 2-oxo-1,2-dihydropyrimidin-1-yl group, each optionally having 1 to 3 substituents selected from α group,
wherein 1) the α group consists of a hydroxyl group, a hydroxyl group protected by a nucleic acid synthesis protecting group, a linear alkyl group having 1 to 6 carbon atoms, a linear alkoxy group having 1 to 6 carbon atoms, a mercapto group, a mercapto group protected by a nucleic acid synthesis protecting group, a linear alkylthio group having 1 to 6 carbon atoms, an amino group, a linear alkylamino group having 1 to 6 carbon atoms, an amino group protected by a nucleic acid synthesis protecting group, and a halogen atom;
* is a binding site to an adjacent oligonucleotide component, or R₁ if the nucleoside residue (B) is located at the 5' end of the oligonucleotide sequence
wherein R₁ is a hydrogen atom, a hydroxyl-protecting group in nucleic acid synthesis, an optionally branched or optionally ring-forming alkyl group having 1 to 7 carbon atoms, or an optionally branched or optionally ring-forming alkenyl group having 2 to 7 carbon atoms; and
** is a binding site to an adjacent oligonucleotide component, or R₂ if the nucleoside residue (B) is located at the 3' end of the oligonucleotide sequence
wherein R₂ is as defined for the aforementioned R₁.

### Nucleoside residue (B) (II)

The above-mentioned nucleoside residue (B) (I), wherein, in the nucleoside residue (B), the partial structure represented by the formula (i) is a group represented by the following partial structural formula (i-1): 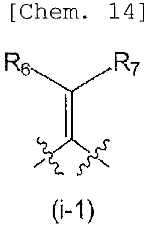 wherein
R₆ and R₇ are each independently a hydrogen atom, a halogen atom, or an alkyl group having 1 to 6 carbon atoms, or R₆ and R₇ are bonded to each other to form, together with the adjacent carbon atom, a carbocycle having 3 to 6 carbon atoms.

More preferably, the above-mentioned nucleoside residue (B) (I), wherein, in the above-mentioned formula (i-1),
R₆ and R₇ are hydrogen atoms;
* is a binding site to an adjacent oligonucleotide component, or a hydrogen atom if the nucleoside residue (B) is located at the 5' end of the oligonucleotide sequence; and
** is a binding site to an adjacent oligonucleotide component, or a hydrogen atom if the nucleoside residue (B) is located at the 3' end of the oligonucleotide sequence.

### Nucleoside residue (B) (III)

The above-mentioned nucleoside residue (B) (I), wherein, in the nucleoside residue (B), the partial structure represented by the formula (i) is a group represented by the following partial structural formula (i-2): 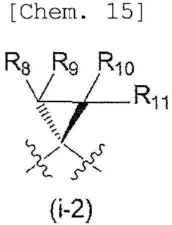 wherein R₈, R₉, R₁₀ and R₁₁ are each independently a hydrogen atom, a halogen atom, or an alkyl group having 1 to 6 carbon atoms.

More preferably, the above-mentioned nucleoside residue (B) (I), wherein, in the above-mentioned formula (i-2),
R₈, R₉, R₁₀ and R₁₁ are each a hydrogen atom;
* is a binding site to an adjacent oligonucleotide component, or a hydrogen atom if the nucleoside residue (B) is located at the 5' end of the oligonucleotide sequence; and
** is a binding site to an adjacent oligonucleotide component, or a hydrogen atom if the nucleoside residue (B) is located at the 3' end of the oligonucleotide sequence.

The embodiments of oligonucleotide (I) have been described in detail above, primarily from the viewpoint of "nucleoside residue (B)" which is the constituent nucleic acid monomer thereof. The embodiments thereof are now described in detail from other viewpoints.

The number of nucleoside residues (B) contained in oligonucleotide (I) is not particularly limited. One embodiment of the present invention includes, for example, oligonucleotide (I) containing 1 to 25 nucleoside residues (B) in the oligonucleotide sequence, and oligonucleotide (I) containing 1 to 10 nucleoside residues (B), and salts thereof.

However, as mentioned above, the number of nucleoside residues (B) introduced is not limited and can be appropriately determined according to the intended use of oligonucleotide (I). For example, it is also possible to constitute the entire sequence of oligonucleotide (I) with nucleoside residues (B).

By introducing at least one nucleoside residue (B), for example, 1 to 10 residues, the toxicity of an oligonucleotide having a base sequence determined in a sequence-dependent manner with respect to the target RNA (hereinafter also referred to as "parent oligonucleotide") can be effectively reduced. Examples of the toxicity to be reduced include hepatotoxicity, weight loss effects, and the like of the parent oligonucleotide.

This "method for reducing the toxicity of oligonucleotide or a salt thereof" is another embodiment of the present invention.

The preferred number of nucleoside residues (B) introduced into the sequence can be appropriately determined by those of ordinary skill in the art depending on the sequence of the parent oligonucleotide and the type and severity of the toxicity. The number of introduction may be, for example, 1 to 30, preferably 1 to 10, more preferably 1 to 6.

The introduction position of nucleoside residue (B) into oligonucleotide (I) is not particularly limited and can be appropriately determined by those of ordinary skill in the art depending on the purpose.

The total base length of oligonucleotide (I) is not particularly limited, and in one embodiment of the present invention, the oligonucleotide is oligonucleotide (I) or a salt thereof having a length of 7 to 30 bases.

The base length of oligonucleotide (I) can be appropriately determined by those skilled in the art depending on the purpose of use and the like, and can be designed. When oligonucleotide (I) or a salt thereof is used, for example, as an antisense oligonucleotide, a length of 7 to 30 bases is one preferred form. Furthermore, a length of 10 to 25 bases or 10 to 20 bases is a more preferred form.

In one embodiment of the present invention, oligonucleotide (I) may be a gapmer consisting of a gap region of 2 to 10 bases in length, a 5'-wing region of 2 to 5 bases in length, and a 3'-wing region of 2 to 5 bases in length, with the gap region positioned between the 5'-wing region and the 3'-wing region.

In this embodiment, a modified nucleic acid with strong binding affinity to RNA may be provided in the wing region.

In this embodiment, the number and position of nucleoside residues (B) introduced into oligonucleotide (I) are not particularly limited. For example, the gap region may contain at least one nucleoside residue (B), and the 5'-wing region and/or 3'-wing region may contain at least one nucleoside residue (B). Furthermore, the gap region, the 5'-wing region, and/or 3'-wing region may each contain at least one nucleoside residue (B).

Those of ordinary skill in the art can appropriately determine the number and position of nucleoside residues (B) introduced into oligonucleotide (I) depending on, for example, the sequence of the target RNA, the sequence of the parent oligonucleotide, and the like.

In one embodiment of the present invention, oligonucleotide (I) may be an oligonucleotide constituting the antisense strand (guide strand) and/or sense strand (passenger strand) of siRNA, which is composed of a double-stranded RNA of about 21 nucleotides in length.

In this embodiment, the nucleotide sequences of the antisense strand and sense strand in oligonucleotide (I) are designed based on the sequence of the target RNA, and 2'-O-methyl modification (2'OMe) or 2'-F-RNA (2'-F) may be introduced for the purpose of reducing off-target effects, controlling pharmacokinetics, immune responses, and the like.

Furthermore, in this embodiment, the number and position of nucleoside residues (B) introduced into oligonucleotide (I) are not particularly limited. For example, nucleoside modifications may be introduced into overhang region, seed region, or cleavage region. Those of ordinary skill in the art can appropriately determine the number and position of modifications to oligonucleotide (I) depending on the sequence of the target RNA, the sequence of the parent oligonucleotide, and the like.

In one embodiment of the present invention, in oligonucleotide (I), at least one of the nucleotide linkages in the nucleic acid sequence may be a phosphorothioate linkage. Furthermore, all of the nucleotide linkages may be phosphorothioate linkages.

In oligonucleotide (I), from the viewpoint of improving the resistance of the oligonucleotide to nucleases, phosphorothioate linkage may be used instead of the usual phosphodiester linkage where necessary. The number and position of phosphorothioate linkages can be appropriately determined by those of ordinary skill in the art, and one preferred embodiment includes phosphorothioate linkages for all nucleotide linkages.

In the embodiment of the present invention, oligonucleotide (I) itself can be used as a single-stranded oligonucleotide (e.g., antisense oligonucleotide), and it can also be used as an oligonucleotide constituting a double-stranded oligonucleotide (e.g., siRNA). In this case, oligonucleotide (I) may constitute one of the double-stranded oligonucleotides, or both.

### [Production method of oligonucleotide (I)]

Oligonucleotides (I) or a salt thereof can be produced by using the aforementioned "nucleoside (A)" as one of the nucleic acid monomers constituting the oligonucleotide, together with other nucleic acid monomers, and introducing same into the oligonucleotide sequence as appropriate. Depending on the sequence of the target oligonucleotide, one or more nucleotides (A) can be introduced not only in the sequence but also at the desired position including the 3' or 5' end. When introducing two or more, they may be introduced consecutively in the sequence or in a form in which one or more other nucleic acid monomers are present.

Specific production of oligonucleotide (I) can be performed by methods widely used in the art. For example, when performing solid-phase synthesis, it can be performed by the following procedure. However, the procedure is not limited to this.

For the chemical synthesis of oligonucleotides, solid-phase synthesis methods using the phosphoramidite method are widely used. In the first stage of the synthesis cycle, the nucleoside at the 3' end of the oligonucleotide (nucleic acid) sequence to be synthesized is supported on a solid-phase support. If the phosphoramidite of the nucleic acid monomer is commercially available, it can be purchased and used. Also, for example, by the method described in Example 1 below, or according to this method, those of ordinary skill in the art can appropriately synthesize and obtain same, including the phosphoramidite of nucleoside (A).

In recent years, universal linkers capable of coupling with any nucleoside are widely used in solid-phase synthesis because the 3' terminal nucleoside can be arbitrarily introduced into oligonucleotide, and oligonucleotides with any sequence, regardless of the 3' terminal nucleoside, can be effectively synthesized.

More specifically, a universal linker is pre-supported on a solid support via a cleavable linker (spacer) such as succinyl group, and an arbitrary 3'-terminal nucleoside is coupled. Subsequently, an oligonucleotide extension reaction, generally consisting of the following steps, is performed in a reaction column according to the synthesis program of an automated nucleic acid synthesizer:
(1) a step of deprotecting 5'-OH group of protected nucleoside with an acid such as trichloroacetic acid/dichloromethane solution;
(2) a step of coupling nucleoside phosphoramidite (also denote as "nucleic acid monomer") to the deprotected 5'-OH group in the presence of an activator (tetrazole, etc.);
(3) a step of capping unreacted 5'-OH group with acetic anhydride or the like; and
(4) a step of oxidizing phosphite with hydrated iodine or the like, or sulfidizing phosphite with 3-((N,N-dimethylaminomethylidene)amino)-3H-1,2,4-dithiazole-5-thion or the like.

By repeating the above synthesis cycle according to the desired sequence and conducting the oligonucleotide extension reaction from the 3' end to the 5' end, oligonucleotide (I) with the desired sequence is synthesized.

Finally, by hydrolyzing the cleavable linker with aqueous ammonia, methylamine solution, or the like, and cleaving out the synthesized oligonucleotide from the solid support and universal linker, oligonucleotide (I) can be obtained.

### (Nucleoside (A))

Each group and partial structure of nucleoside (A) are respectively as defined for the corresponding group and partial structure defined for "nucleoside residue (B)" in [1] in the above-mentioned [Means of Solving the Problems] section.

Therefore, for the preferred embodiments of each group and partial structure, and preferred embodiments as nucleoside (A), the corresponding detailed descriptions of "nucleoside residue (B)" can be respectively referred to.

Preferred embodiments of nucleoside (A) are shown by the structural formulas as follows. Hereinafter, nucleosides having the structures are also denoted as nucleoside (A-i-1) and nucleoside (A-i-2).

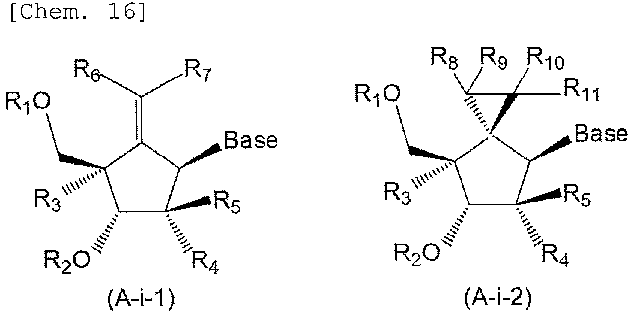

Nucleoside (A) can be used to reduce the toxicity of oligonucleotides. More specifically, nucleoside (A) can be used to reduce the toxicity of an oligonucleotide by introducing at least one nucleoside (A) into the target oligonucleotide sequence.

The use of nucleoside (A) in such form is another embodiment of the present invention.

### (Production method of nucleoside derivative (A))

Those of ordinary skill in the art can produce nucleoside derivative (A) as appropriate, starting from known compounds and using known methods. For example, it can be produced by referring to the method specifically described in the following examples. The raw material compounds can also be appropriately obtained by those of ordinary skill in the art, starting from known compounds.

### [Use of oligonucleotide (I)]

Oligonucleotide (I) or a salt thereof (hereinafter collectively referred to as "this oligonucleotide") can afford superior effects of reducing toxicity while improving or maintaining the efficacy of the drug, as demonstrated in the following examples, by introducing nucleoside derivative (A) into the original antisense oligonucleotide sequence. In addition, this oligonucleotide itself can also be used, for example, as a novel antisense oligonucleotide. Therefore, this oligonucleotide is useful as a medicament for the prevention or treatment of diseases as, for example, an antisense oligonucleotide.

Such use of this oligonucleotide as a medicament is yet another embodiment of the present invention.

The embodiments of using this oligonucleotide as a medicament is described in detail below.

In the present specification, the "prophylaxis" includes preventing the onset of a disease (all pathological conditions or one or more pathological conditions) and delaying the onset of the disease. A "prophylactically effective amount" refers to a dose of the oligonucleotide sufficient to achieve such purposes.

In the present specification, the "treatment" includes curing a disease (all pathological conditions or one or more pathological conditions), improving the disease, and suppressing the progression of the severity of the disease. A "therapeutically effective amount" refers to a dose of the oligonucleotide sufficient to achieve such purposes.

In practicing the present invention, the oligonucleotide can be used either alone or in the form of a pharmaceutical composition containing the oligonucleotide as an active ingredient together with a pharmaceutically acceptable carrier.

Examples of such pharmaceutical composition include tablets (including sugar-coated tablet, film-coated tablet, sublingual tablet, orally disintegrating tablet, buccal tablet, and the like), pill, powder, granule, capsules (including soft capsule, and microcapsule), syrup, liquid, emulsion, suspension, controlled-release preparations (e.g., immediate-release preparation, sustained-release preparation, sustained-release microcapsule), aerosols, films (e.g., orally disintegrable films, and buccal mucoadhesive film), injections (e.g., subcutaneous injection, intravenous injections (e.g., bolus), intramuscular injection, and intraperitoneal injection), intrathecal injection, intraventricular injection), infusion, transdermal absorption type preparation, ointment, lotion, adhesive preparation, suppositories (e.g., rectal suppository, vaginal suppository), pellet, nasal preparation, pulmonary preparation (inhalant), eye drop, and the like.

In the present specification, as the "pharmaceutically acceptable carrier", various carriers conventionally used in the field of pharmaceutical technology can be used.

As the specific examples of the "pharmaceutically acceptable carrier", in solid preparations, excipients (e.g., lactose, sucrose, D-mannitol, starch, cornstarch, crystalline cellulose, light anhydrous silicic acid, etc.), lubricants (e.g., magnesium stearate, talc, colloid silica, etc.), binders (e.g., crystalline cellulose, sucrose, D-mannitol, dextrin, hydroxypropylcellulose, hydroxypropylmethylcellulose, polyvinylpyrrolidone, starch, sucrose, gelatin, methylcellulose, sodium carboxymethylcellulose, etc.), disintegrants (e.g., starch, carboxymethylcellulose, carboxymethylcellulose calcium, sodium carboxymethyl starch, L-hydroxypropylcellulose, etc.), and the like can be used.

In liquid preparations, solvents (e.g., water for injection, isotonic brine, alcohol, propylene glycol, macrogol, sesame oil, etc.), solubilizing agents (e.g., polyethylene glycol, propylene glycol, D-mannitol, benzyl benzoate, ethanol, triethanolamine, sodium carbonate, sodium citrate, etc.), suspending agents (e.g., surfactants such as stearyltriethanolamine, sodium lauryl sulfate, lauryl aminopropionic acid, lecithin, benzalkonium chloride, glycerin monostearate, and the like; for example, hydrophilic polymers such as poly(vinyl alcohol), polyvinylpyrrolidone, sodium carboxymethylcellulose, methylcellulose, hydroxymethylcellulose, hydroxypropylcellulose, and the like, and the like), isotonicity agents (e.g., glucose, D-sorbitol, sodium chloride, glycerin, D-mannitol, etc.), buffering agents (e.g., buffer of phosphate, citrate, or the like, etc.), and soothing agents (e.g., benzyl alcohol etc.), and the like can be used.

Where necessary, formulation additives such as antiseptics (e.g., paraoxybenzoic acid esters, chlorobutanol, benzyl alcohol, sorbic acid, etc.), antioxidants (e.g., sulfite, ascorbic acid, α-tocopherol, etc.), colorants, sweeteners, and the like may be further added.

The pharmaceutical composition of the present invention generally containing the oligonucleotide of the present invention in an amount of 0.01 to 99% (w/w), preferably 0.1 to 85% (w/w), of the total amount of the preparation, can be produced, though the amount varies depending on the dosage form, administration method, carrier, and the like. The pharmaceutical composition can be produced by methods conventionally used in the field of pharmaceutical technology. The pharmaceutical composition of the present invention may be formulated into a sustained release preparation containing the active ingredient.

### (Subject of administration)

The oligonucleotide is expected to have low toxicity and few side effects, and also has superior properties as a pharmaceutical product. Therefore, the oligonucleotide can be safely administered to mammals (e.g., particularly human).

### (Administration route)

In practicing the present invention, the oligonucleotide may be administered orally or parenterally (e.g., intravenously, intramuscularly, subcutaneously, intraviscerally, intranasally, intradermally, ophthalmically, intracerebrally (intraventricularly), intrathecally, rectally, intravaginally, intraperitoneally, intralesionally) alone or as a pharmaceutical composition.

### (Dose)

The dose of the pharmaceutical composition of the present invention varies depending on the purpose of administration, method of administration, type and severity of the target disease, and the circumstances of the subject of administration (sex, age, body weight, and the like), and is not particularly limited. For example, when administered systemically to adults, the single dose of the complex of the present invention is generally desirably 0.01 mg/kg or more and 1000 mg/kg or less, and when administered topically, desirably 0.001 mg/body or more and 100 mg/body or less. It is desirable to administer such doses 1 to 10 times, more preferably 5 to 10 times.

The pharmaceutical composition of the present invention can be used in combination with, for example, a drug already on the market for treating a disease. These concomitant drugs can be formulated together with the pharmaceutical composition of the present invention and administered as a single preparation, or they can be formulated separately from the pharmaceutical composition of the present invention and administered simultaneously or with a time difference via the same or different route as the pharmaceutical composition of the present invention. In addition, the dose of these concomitant drugs may be the amount generally used when the medicament is administered alone, or it may be a reduced amount.

### [Example]

The present invention is further described in detail by the following Examples. They do not limit the present invention and the present invention may be modified without departing from the scope of the present invention.

Oligonucleotide (I) was synthesized using Entecavir as nucleoside (A) and pharmacological evaluation (pharmacological efficacy/toxicity) thereof was performed. Various examples showing the results thereof and the like are shown below.

### [Example 1] Synthesis of amidite

Entecavir phosphoramidite was synthesized according to the following synthesis scheme (previously reported).

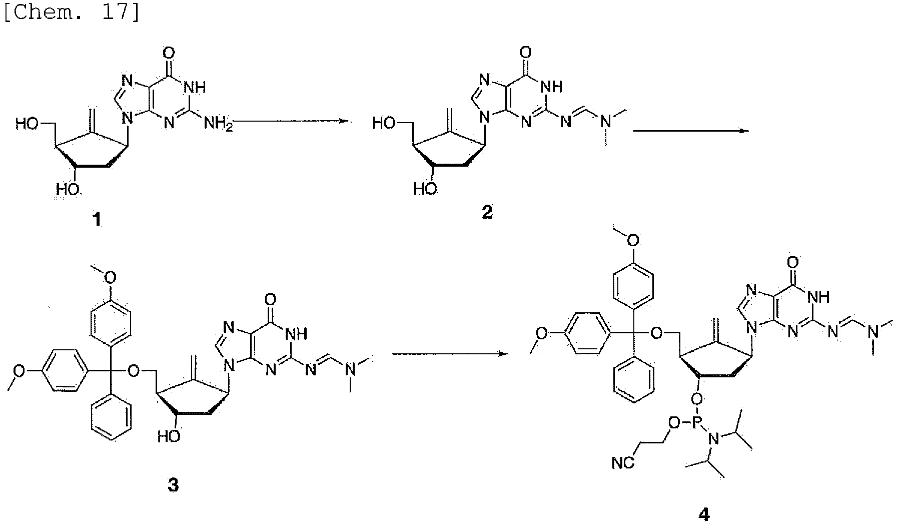

To compound 1 (Entecavir) (500 mg, 1.80 mmol) was added dry MeOH (17 mL) under a nitrogen atmosphere, N,N-dimethylformamide dimethyl acetal (905 µL, 6.77 mmol) was added, and the mixture was stirred at room temperature for 12 hr. After confirming the consumption of the starting material by TLC, the solvent was concentrated under reduced pressure to obtain the object compound 2 (581 mg, 98%).

To compound 2 (658 mg, 1.98 mmol) were added dry Pyridine (19 mL) and dry DMF (5 mL) under a nitrogen atmosphere, 4,4-dimethoxytrityl chloride (470 mg, 1.39 mmol) was added, and the mixture was stirred at room temperature for 1 hr. Furthermore, 4,4-dimethoxytrityl chloride (470 mg, 1.39 mmol) was added, and the mixture was stirred at room temperature for 3 hr. After confirming the consumption of the starting material by TLC, the solvent was concentrated under reduced pressure. To the residue was added saturated NaHCO₃ solution (100 mL), and the mixture was extracted three times with EtOAc (100 mL). The organic layer was washed with brine and dried over Na₂SO₄. The solvent was evaporated under reduced pressure, and the residue was purified by silica gel column chromatography (eluent: DCM/MeOH=98:2+0.5%TEA) to give the object compound 3 (1.01 g, 81%).

Compound 3 (563 mg, 0.89 mmol) and 1H-tetrazole (93.5 mg, 1.34 mmol) were added to a heated and dried reaction vessel. Dry DCM (19 mL) and DIPEA (303 µL, 1.34 mmol) were then added under a nitrogen atmosphere. 2-Cyanoethyl N,N,N',N'-tetraisopropylphosphordiamidite (367 µL, 1.16 mmol) was added, and the mixture was stirred at room temperature for 2 hr. After confirming the consumption of the starting material by TLC, saturated NaHCO₃ solution (150 mL) was added, and the mixture was extracted three times with DCM (150 mL). The organic layer was washed with brine, dried over Na₂SO₄, and the solvent was evaporated under reduced pressure. The residue was purified by silica gel column chromatography (eluent: DCM/MeOH=100:0 to 95:5 + 1% TEA) to give the object compound 4 (514 mg, 0.616 mmol, 69%).

### [Example 2]

### Purpose: Synthesis of oligonucleotide

### Method and Results:

### (1) Synthesis scheme

The synthesis of oligonucleotides was performed by preparing 0.1 M anhydrous acetonitrile solutions of commercially available dA(Bz), dG(iBu), dC(Bz), dT, LNA-A(Bz), LNA-G(DMF), LNA-mC (Bz), LNA-T and phosphoramidite of Entecavir synthesized in Example 1, and following a standard phosphoramidite method using a DNA/RNA synthesizer (NTS M-2-TRS, Nippon Techno Service).

The synthesis scale was 1 µmol, and the process was performed under trityl-on conditions. 5-benzylthio-1H-tetrazole (0.25 M anhydrous acetonitrile) was used as the activator, and the condensation time was 3 min for LNA amidite block and 30 seconds for natural amidite block. After completion of the synthesis, a treatment with 28% aqueous ammonia was performed at 55°C for 13 hr, cleavage from the column support and deprotection of the base moiety and phosphate diester moiety were performed, followed by purification using a simple reversed-phase column (Glen-Pak^{™} DNA Purification Cartridge, Glen Research), and further purification by reversed-phase HPLC. The HPLC measurement conditions are shown below.

### (Eluent)

Solution A: 100 mM hexafluoro-2-propanol 8.6 mM triethylamine (pH 8.36)
Solution B: methanol

### (Gradient)

Solution B concentration: 1) 5-30% (30 min) (purification) 2) 5-40% (20 min) (purity confirmation)

### (Column)

1) nacalai 5C₁₈-MS-II (10 × 250 mm) (purification)
2) nacalai 5C₁₈-MS-II (4.6 × 50 mm) (purity confirmation)
3) Column temperature: 60°C

### (Flow rate)

1) 2.0 mL/min (purification) 2) 0.5 mL/min (purity confirmation) (Detection) UV (260 nm)

### (2) Synthesized oligonucleotide

According to the above-mentioned synthesis scheme, oligonucleotides having the sequences shown in Table 1 below (nucleotide linkage: all phosphorothioate linked) were obtained. In the Table, E is Entecavir.

In Table 1 and Tables 2-9, 12-14, and 16 below, unless otherwise specified, uppercase nucleosides represent LNA, and lowercase nucleosides represent DNA. Entecavir may also be indicated by the subscript E. In hApo1-cRNA and hApol-cDNA, uppercase letters represent RNA and DNA, respectively.

**[Table 1]**

| Abbreviation | ID | | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|---|
| hApo1 | hApoB-521-BNA(13) | 5' | A | A | t | g | g | c | c | a | g | c | T | T | G | 3' |
| gap1 | hApoB-521-BNA(13)-gap1 | | A | A | t | E | g | c | c | a | g | c | T | T | G | |
| gap2 | hApoB-521-BNA(13)-gap2 | | A | A | t | g | E | c | c | a | g | c | T | T | G | |
| gap3 | hApoB-521-BNA(13)-gap3 | | A | A | t | g | g | c | c | a | E | c | T | T | G | |
| 2mod | HApoB-521-BNA(13)-2mod | | A | A | t | E | E | c | c | a | g | c | T | T | G | |
| 3mod | hApoB-521-BNA(13)-3mod | | A | A | t | E | E | c | c | a | E | c | T | T | G | |
| | hApo1-cRNA | 3' | U | U | A | C | C | G | G | U | C | G | A | A | C | 5' |
| | hApo1-cDNA | 3' | T | T | A | C | C | G | G | T | C | G | A | A | C | 5' |

### Discussion:

From the above, it has been clarified that nucleoside (A) can be introduced into oligonucleotide sequences according to conventional methods, and that multiple nucleosides and consecutive nucleosides can also be introduced into a single sequence.

### [Example 3]

### Purpose: Measurement of melting temperature (Tₘ) of double-strand formed with complementary strand RNA

### Method and Results:

A sample solution (150 µL) with final concentrations of 10 mM phosphate buffer (pH 7.0), 100 mM sodium chloride, 0.1 mM ethylenediaminetetraacetic acid, and 4 µM of each oligonucleotide and complementary RNA (hApo1-cRNA, Table 1) listed in Table 2 below was heated to 95°C for 3 min, then annealed by slow cooling to 20°C at a rate of 1°C per min, and the measurement was started. The temperature was increased to 95°C at a rate of 0.5°C per min, and the absorbance at 260 nm was plotted at 1°C intervals. All Tₘ values were calculated using the midline method.

The measurement results are shown in the following Table 2. In the Table, E represents Entecavir.

**[Table 2]**

| Abbreviation | ID | *T*m | Δ*T*m |
|---|---|---|---|
| hApo1 | hApoB-521-BNA(13) | 62.3 | - |
| gap1 | hApoB-521-BNA(13)-gap1 | 63.0 | +0.7 |
| gap2 | hApoB-521-BNA(13)-gap2 | 62.8 | +0.5 |
| gap3 | hApoB-521-BNA(13)-gap3 | 62.8 | +0.5 |
| 2mod | hApoB-521-BNA(13)-2mod | 64.8 | +2.5 |
| 3mod | hApoB-521-BNA(13)-3mod | 63.2 | +0.9 |

### Discussion:

From the above, it was shown that nucleoside (A) is a unique non-natural nucleotide that does not impair the thermodynamic stability of ASO with respect to target RNA.

### [Example 4]

### Purpose: Measurement of melting temperature (Tₘ) of double-strand formed with complementary strand DNA

### Method and Results:

A sample solution (150 µL) with final concentrations of 10 mM phosphate buffer (pH 7.0), 100 mM sodium chloride, 0.1 mM ethylenediamine tetraacetic acid, and 4 µM of each oligonucleotide and complementary DNA (hApo1-cDNA, Table 1) was heated to 95°C for 3 min, then annealed by slow cooling to 20°C at a rate of 1°C per min, and the measurement was started. The temperature was increased to 95°C at a rate of 0.5°C per min, and the absorbance at 260 nm was plotted at 1°C intervals. All Tₘ values were calculated using the midline method.

The measurement results are shown in the following Table 3. In the Table, E represents Entecavir.

**[Table 3]**

| Abbreviation | ID | *T*m | Δ*T*m |
|---|---|---|---|
| hApo1 | hApoB-521-BNA(13) | 57.7 | - |
| gap1 | hApoB-521-BNA(13)-gap1 | 59.4 | 1.7 |
| gap2 | hApoB-521-BNA(13)-gap2 | 58.8 | 1.1 |
| gap3 | hApoB-521-BNA(13)-gap3 | 54.6 | -3.1 |
| 2mod | hApoB-521-BNA(13)-2mod | 57.9 | 0.2 |
| 3mod | hApoB-521-BNA(13)-3mod | 55.8 | -1.9 |

From the above, it was shown that nucleoside (A) is a unique non-natural nucleotide that does not impair the thermodynamic stability of complementary strand DNA.

### [Example 5]

### Purpose: Evaluation of in vitro effect of oligonucleotide drug incorporating nucleoside (A) in the strand on inhibiting target gene expression

### Method and Results:

ASO was diluted to a final concentration of 2 µM in a cell culture medium (9 mM CaCl₂ added) and added to a 96-well plate. Huh-7 cells diluted in the above-mentioned medium were seeded by 10,000 cells into each well and cultured in an incubator for 24 hr. Then, cDNA was prepared according to the manufacturer's protocol from the cell lysate by using SuperPrep (registered trademark) II Cell Lysis & RT Kit for qPCR (TOYOBO). The expression level of ApoB mRNA was analyzed using a CFX real-time PCR system (BIO RAD). For the analysis of human ApoB, hApoB-F: 5'-TTCTCAAGAGTTACAGCAGATCCA-3' (SEQ ID NO: 1) and hApoB-R: 5'-TGGAAGTCCTTAAGAGCAACTAACA-3' (SEQ ID NO: 2) were used. For the analysis of human Gapdh as a housekeeping gene, the primer set of hGAPDH-F: 5'-GCACCGTCAAGGCTGAGAAC-3' (SEQ ID NO: 3) and hGAPDH-R: 5'-TGGTGAAGACGCCAGTGGA-3' (SEQ ID NO: 4) was used. KD activity was calculated based on relative expression levels, by converting the difference in Ct values to the difference in expression levels.

The evaluation results are shown in Fig. 1.

### Discussion:

Fig. 1 shows that gap2, 2mod, and 3mod exhibited slightly reduced activity, while gap3 showed activity equivalent to or slightly higher than hApo1 (parent strand). It was shown that oligonucleotide (I) (oligonucleotide drug) incorporating nucleoside (A) in the strand has gene suppressive activity.

### [Example 6]

### Purpose: Evaluation of cytotoxicity (cell survival rate) of oligonucleotide drug incorporating nucleoside (A) in the strand on inhibiting target gene expression

### Method and Results:

ASO was diluted to a final concentration of 1 or 2 µM in a cell culture medium (9 mM CaCl₂ added) and added to a 96-well plate. Huh-7 cells diluted in the above-mentioned medium were seeded by 10,000 cells into each well and cultured in an incubator for 72 hr. Then, Cell Counting Kit-8 (Dojin Chemical Co., Ltd.) was added at 10 µL (/well) and the mixture was incubated in an incubator for 2 hr. The absorbance at 450 nm was measured using a microplate reader.

The evaluation results are shown in Fig. 2.

### Discussion:

From Fig. 2, cell death was suppressed and cytotoxicity was reduced in gap2, gap3, 2mod, and 3mod. It was shown that oligonucleotide (I) (oligonucleotide drug) incorporating nucleoside (A) at the positions shown in Table 3 has low cytotoxicity.

### [Example 7]

### Purpose: Evaluation of cytotoxicity (Caspase 3/7 activity) of oligonucleotide drug incorporating nucleoside (A) in the strand

### Method and Results:

ASO was diluted to a final concentration of 1 or 2 µM in a cell culture medium (9 mM CaCl₂ added) and added to a 96-well plate. Huh-7 cells diluted in the above-mentioned medium were seeded by 10,000 cells into each well and cultured in an incubator for 72 hr. Then, the activation of Caspase 3/7 was measured using Caspase-Glo (registered trademark) 3/7 Assay Systems, Caspase-Glo (registered trademark) (Promega) according to the manufacturer's protocol.

The measurement results are shown in Fig. 3.

### Discussion:

From Fig. 3, an increase in Caspase3/7 was significantly suppressed in gap2, 2mod, and 3mod. It was shown that the toxicity of oligonucleotide drugs can be reduced by introducing nucleoside (A) into the strand at the positions shown in Table 3.

### [Example 8]

### Purpose: Synthesis of oligonucleotide drug containing nucleoside (A) equipped with targeting ligand

### Method and Results:

### (1) Synthesis scheme

The synthesis of oligonucleotides was performed by preparing 0.1 M anhydrous acetonitrile solutions of commercially available dA(Bz), dG(iBu), dC(Bz), dT, LNA-A(Bz), LNA-G(DMF), LNA-mC (Bz), LNA-T and phosphoramidite of Entecavir synthesized in Example 1, and following a standard phosphoramidite method using a DNA/RNA synthesizer (NTS M-2-TRS, Nippon Techno Service). The synthesis scale was 1 µmol, and the process was performed under trityl-on conditions. 5-Benzylthio-1H-tetrazole (0.25 M anhydrous acetonitrile) was used as the activator, and the condensation time was 3 min for LNA amidite block and 30 seconds for natural amidite block.

After synthesis, the solid support was transferred to a 1.0 mL gastight syringe, and the ligand moiety was extended by manual synthesis. The ligand moiety was extended by allowing a GalNAc amidite block (0.1 M anhydrous acetonitrile) and 5-ethylthio-1H-tetrazole (0.5 M anhydrous acetonitrile) as an activator to act, according to the standard phosphoramidite method. The GalNAcamidite block was prepared according to a report in "Terada C, Wada F, Uchida M, Yasutomi Y, Oh K, Kawamoto S, Kayaba Y, Yamayoshi A, Harada-Shiba M, Obika S, Yamamoto T. Programmed Instability of Ligand Conjugation Manifold for Efficient Hepatocyte Delivery of Therapeutic Oligonucleotides. Nucleic Acid Ther. 2021 Dec; 31(6):404-416. doi: 10.1089/nat.2021.0036." After completion of the synthesis, a treatment with 28% aqueous ammonia was performed at 55°C for 13 hr, cleavage from the column support and deprotection of the base moiety and phosphate diester moiety were performed, followed by purification using a simple reversed-phase column (Glen-Pak^{™} DNA Purification Cartridge, Glen Research), and further purification by reversed-phase HPLC. The HPLC measurement conditions are shown below.

### (Eluent)

Solution A: 100 mM hexafluoro-2-propanol 8.6 mM triethylamine (pH 8.36)
Solution B: methanol

### (Gradient)

Solution B concentration: 1) 5-30% (30 min) (purification) 2) 5-40% (20 min) (purity confirmation)

### (Column)

1) nacalai 5C₁₈-MS-II (10 × 250 mm) (purification)
2) nacalai 5C₁₈-MS-II (4.6 × 50 mm) (purity confirmation)
3) Column temperature: 60°C

### (Flow rate)

1) 2.0 mL/min (purification) 2) 0.5 mL/min (purity confirmation) (Detection) UV (260 nm)

### (2) Synthesized oligonucleotide

According to the above-mentioned synthesis scheme, oligonucleotides having the sequences shown in Table 4 below (nucleotide linkage: two linkages from 5' end are phosphodiester linkages, and the remaining linkages are phosphorothioate linkages) could be obtained. In the Table, E is Entecavir and X is GalNAc_{APD}.

**[Table 4]**

| Abbreviation | ID | | | | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|---|---|---|
| hApo1 | hApoB-521-BNA(13) | 5' | X | X | A | A | t | g | g | c | c | a | g | c | T | T | G | 3' |
| gap1 | hApoB-521-BNA(13)-gap1 | | X | X | A | A | t | E | g | c | c | a | g | c | T | T | G | |
| gap2 | hApoB-521-BNA(13)-gap2 | | X | X | A | A | t | g | E | c | c | a | g | c | T | T | G | |
| gap3 | hApoB-521-BNA(13)-gap31 | | X | X | A | A | t | g | g | c | c | a | E | c | T | T | G | |

### Discussion:

From the above, it was clarified that an oligonucleotide drug (oligonucleotide (I)) containing nucleoside (A) equipped with a targeting ligand can be constructed.

### [Example 9]

### Purpose: Verification of in vivo effect of oligonucleotide drug containing nucleoside (A) equipped with targeting ligand on inhibiting target gene expression

### Method and Results:

All animal experiment protocols were practiced after approved by the Animal Experiment Committee of Nagasaki University. ASO targeting ApoB (human-mouse common sequence) was administered subcutaneously at a single dose of 200 nmol/kg to 7-week-old C57Bl/6J (male, Japan SLC). After 72 hours from administration, whole blood was collected under isoflurane inhalation anesthesia, and the liver was collected. The liver was stored overnight at 4°C in RNAlater^{™} solution, and then stored at -20°C until analysis. Liver total RNA was extracted using the QuickGene RNA tissue kit SII (Fujifilm Corporation) according to the attached instructions. Using the extracted total RNA as a template, reverse transcription was performed using the High Capacity RNA-to-cDNA^{™} kit (Thermo Fisher Scientific), and the expression level of ApoB mRNA was analyzed using the CFX Real-Time PCR System (BIO RAD). For the analysis of mouse ApoB, mApoB-F: 5'-TCCTCGGTGAGTTCAATGACTTTC-3' (SEQ ID NO: 5) and mApoB-R: 5'-TGGACCTGCTGTAGCTTGTAGGA-3' (SEQ ID NO: 6) were added. For the analysis of mouse Gapdh as a housekeeping gene, the primer set of mGAPDH-F: 5'-TGTGTCCGTCGTGGATCTGA-3' (SEQ ID NO: 7) and mGAPDH-R: 5'-TTGCTGTTGAAGTCGCAGGAG-3' (SEQ ID NO: 8) was used. KD activity was calculated based on relative expression levels, by converting the difference in Ct values to the difference in expression levels.

The evaluation results are shown in Fig. 4.

### Discussion:

From Fig. 4, the in vivo knockdown activity of the target, similar to the in vitro evaluation results, still exhibited a high gene expression suppressive effect, although gap2 showed slightly reduced activity. On the other hand, gap1 and gap3 showed high activity comparable to hApo1 (parent strand). Although differences depending on the introduction site were confirmed, it was clarified that oligonucleotide (I) generally has high activity.

### [Example 10]

### Purpose: Verification of influence of nucleoside (A) on toxicity of oligonucleotide drug using mouse

### Method and Results:

All animal experiment protocols were practiced after approved by the Animal Experiment Committee of Nagasaki University. ASO targeting ApoB, obtained in Example 8, was administered subcutaneously at a single dose of 200 nmol/kg to 7-week-old C57Bl/6J (male, Japan SLC). After 72 hours from administration, whole blood was collected under isoflurane inhalation anesthesia, and the liver was collected. Body weight changes were evaluated, and serum collected from the inferior vena cava was also used for evaluation. ALT, total bilirubin, direct bilirubin, and indirect bilirubin were measured by Oriental Yeast Co., Ltd.

The evaluation results are shown in Figs. 5 to 9.

### Discussion:

The introduction of nucleoside (A) eliminated weight loss (Fig. 5), hepatotoxicity (Fig. 6), and jaundice symptoms (Figs. 7, 8, 9) associated with side effects of antisense nucleic acid drugs in all oligonucleotides (I) with gap1, gap2, and gap3. In particular, gap2, which also showed effects in cell experiments, drastically improved abnormal ALT and bilirubin levels observed in hApo1.

It was clarified that, in the oligonucleotide (I) of the present invention, the toxicity of oligonucleotide is improved by the introduction of nucleoside (A) compared to that before introduction, and that oligonucleotide drug with highly superior activity and safety can be obtained by selecting the introduction site and number thereof.

### [Example 11]

### Purpose: Measurement of melting temperature (Tₘ) of double-strand formed with complementary strand RNA

### Method and Results

### (1) Synthesis of oligonucleotide

In the same manner as in the method described in Example 2, various oligonucleotides with mPCS2 as the parent sequence (nucleotide linkage: all phosphorothioate linked) described in the following Table 5 were synthesized and subjected to the following evaluation.

### (2) Measurement of melting temperature (Tₘ) of double-strand formed with complementary strand RNA

A sample solution (150 µL) with final concentrations of 10 mM phosphate buffer (pH 7.0), 100 mM sodium chloride, 0.1 mM ethylenediaminetetraacetic acid, and 4 µM of each oligonucleotide and complementary RNA was heated to 95°C for 3 min, then annealed by slow cooling to 20°C at a rate of 1°C per min, and the measurement was started. The temperature was increased to 95°C at a rate of 0.5°C per min, and the absorbance at 260 nm was plotted at 1°C intervals. All Tₘ values were calculated using the midline method.

The measurement results are shown in the following Table 5. In the Table, E represents Entecavir.

**[Table 5]**

| Abbreviation | ID | | 1 | 2 | 3 | 4 | 5 | 6 | 7 | 8 | 9 | 10 | 11 | 12 | 13 | 14 | *T*m | Δ*T*m |
|---|---|---|---|---|---|---|---|---|---|---|---|---|---|---|---|---|---|---|
| mPCS2 | mPCSK9-1418-BNA(13) | 5' | C | T | g | t | g | a | t | g | a | c | C | T | c | 3' | 60 | - |
| gap1 | mPCSK9-1418-BNA(13)-gap1 | | C | T | E | t | g | a | t | g | a | c | C | T | c | | 58 | -1.8 |
| gap2 | mPCSK9-1418-BNA(13)-gap2 | | C | T | g | t | E | a | t | g | a | c | C | T | c | | 61 | +1.6 |
| gap3 | mPCSK9-1418-BNA(13)-gap3 | | C | T | g | t | g | a | t | E | a | c | C | T | c | | 61 | +1.9 |
| | mPCS2-cRNA | 3' | G | A | C | A | C | U | A | C | U | G | G | A | G | 5' | | |

### Discussion:

From the above, it was shown that nucleoside (A) is a unique non-natural nucleotide that does not impair the thermodynamic stability with respect to complementary strand RNA even in different ASO sequence (mPCS2).

### [Example 12]

### Purpose: Verification of in vivo effect of oligonucleotide drug containing nucleoside (A) equipped with targeting ligand on inhibiting target gene expression

### Method and Results

### (1) Synthesis of oligonucleotide drug containing nucleoside (A) equipped with targeting ligand

In the same manner as in the method described in Example 8, various oligonucleotide drugs containing nucleoside (A) equipped with targeting ligand with mPCS2 as the parent sequence, described in the following Table 6, were synthesized and subjected to the following evaluation.

**[Table 6]**

| Abbreviation | ID | | | | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|---|---|---|
| mPCS2 | mPCSK9-1418-BNA(13) | 5' | X | X | C | T | g | t | g | a | t | g | a | c | C | T | c | 3' |
| gap1 | mPCSK9-1418-BNA(13)-gap1 | | X | X | C | T | E | t | g | a | t | g | a | c | C | T | c | |
| gap2 | mPCSK9-1418-BNA(13)-gap2 | | X | X | C | T | g | t | E | a | t | g | a | c | C | T | c | |
| gap3 | mPCSK9-1418-BNA(13)-gap3 | | X | X | C | T | g | t | g | a | t | E | a | c | C | T | c | |

### (2) Verification of in vivo effect of oligonucleotide drug containing nucleoside (A) equipped with targeting ligand on inhibiting gene expression

All animal experiment protocols were practiced after approved by the Animal Experiment Committee of Nagasaki University. ASO targeting PCSK9, described in Table 6, was administered subcutaneously at a single dose of 200 nmol/kg to 7-week-old C57Bl/6J (male, Japan SLC). After 72 hours from administration, whole blood was collected under isoflurane inhalation anesthesia, and the liver was collected. The liver was stored overnight at 4°C in RNAlater^{™} solution, and then stored at -20°C until analysis. Liver total RNA was extracted using the QuickGene RNA tissue kit SII (Fujifilm Corporation) according to the attached instructions. Using the extracted total RNA as a template, reverse transcription was performed using the High Capacity RNA-to-cDNA^{™} kit (Thermo Fisher Scientific), and the expression level of PCSK9 mRNA was analyzed using the CFX Real-Time PCR System (BIO RAD). For the analysis of mouse PCSK9, mPSCK9-F: 5'-TCAGTTCTGCACACCTCCAG-3' (SEQ ID NO: 9) and mPCSK9-R: 5'-GGGTAAGGTGCGGTAAGTCC-3' (SEQ ID NO: 10) were added. For the analysis of mouse Gapdh as a housekeeping gene, the primer set of mGAPDH-F: 5'-TGTGTCCGTCGTGGATCTGA-3' (SEQ ID NO: 7) and mGAPDH-R: 5'-TTGCTGTTGAAGTCGCAGGAG-3' (SEQ ID NO: 8) was used. KD activity was calculated based on relative expression levels, by converting the difference in Ct values to the difference in expression levels.

The evaluation results are shown in Fig. 10.

### Discussion:

It was clarified that activity equivalent to or greater than that of the parent sequence (mPCS2) can be obtained by introducing nucleoside (A).

### [Example 13]

### Purpose: Verification of influence of nucleoside (A) on toxicity of oligonucleotide drug using mouse

### Method and Results:

In the same manner as in the method described in Example 10 and using oligonucleotides equipped with various targeting ligands synthesized in Example 12, the test was performed. Body weight changes were evaluated, and serum collected from the inferior vena cava was also used for evaluation. ALT, total bilirubin, direct bilirubin, and indirect bilirubin were measured by Oriental Yeast Co., Ltd.

The evaluation results are shown in Figs. 11 to 14.

### Discussion:

It was clarified that the toxicity observed in the parent sequence (mPCS2) can be improved by changing the introduction position of nucleoside (A).

### [Example 14]

### Purpose: Measurement of melting temperature (Tₘ) of double-strand formed with complementary strand RNA

### Method and Results

### (1) Synthesis of oligonucleotide

In the same manner as in the method described in Example 2, various oligonucleotides with mPCS2 as the parent sequence (nucleotide linkage: all phosphorothioate linked) described in the following Table 7 were synthesized and subjected to the following evaluation.

Here, oligonucleotides were synthesized and evaluated also using a thymidine derivative of carbocyclic DNA (see Compound 17 (E^{T}) in Example 32 below) as the nucleoside (A), in addition to an Entecavir derivative (E).

### (2) Measurement of melting temperature (Tₘ) of double-strand formed with complementary strand RNA

A sample solution (150 µL) with final concentrations of 10 mM phosphate buffer (pH 7.0), 100 mM sodium chloride, 0.1 mM ethylenediaminetetraacetic acid, and 4 µM of each oligonucleotide and complementary RNA was heated to 95°C for 3 min, then annealed by slow cooling to 20°C at a rate of 1°C per min, and the measurement was started. The temperature was increased to 90°C at a rate of 0.5°C per min, and the absorbance at 260 nm was plotted at 1°C intervals. All Tₘ values were measured three times and calculated using the midline method.

The measurement results are shown in the following Table 7. In the Table, E represents Entecavir and E^{T} represents a thymidine derivative of carbocyclic DNA.

**[Table 7]**

| Abbreviation | ID | | 1 | 2 | 3 | 4 | 5 | 6 | 7 | 8 | 9 | 10 | 11 | 12 | 13 | 14 | *Tm* | Δ*T*m |
|---|---|---|---|---|---|---|---|---|---|---|---|---|---|---|---|---|---|---|
| mPCS2 | mPCSK9-1418-BNA(13) | 5' | C | T | g | t | g | a | t | g | a | c | C | T | c | 3' | 60 | - |
| gap1 | mPCSK9-1418-BNA(13)-gap1 | | C | T | E | t | g | a | t | g | a | c | C | T | c | | 58 | -1.8 |
| gap2 | mPCSK9-141e-BNA(13)-gap2 | | C | T | g | t | E | a | t | g | a | c | C | T | c | | 61 | +1.6 |
| gap3 | mPCSK9-1418-BNA(13)gap3 | | C | T | g | t | g | a | t | E | a | c | C | T | c | | 61 | +1.9 |
| gapT1 | mPCSK9-1418-BNA(13)-gapT1 | | C | T | g | E^{T} | g | a | t | g | a | c | C | T | c | | 59 | -0.4 |
| gapT2 | mPCSK9-1418-BNA(13)-gapT2 | | C | T | g | t | g | a | E^{T} | g | a | c | C | T | c | | 59 | -1.0 |
| | mPCS2-cRNA | 3' | G | A | C | A | C | U | A | C | U | G | G | A | G | 5' | | |

### Discussion:

The introduction of the thymidine derivative (E^{T}) of carbocyclic DNA into the ASO did not greatly affect the thermodynamic stability of the ASO-target RNA complex, similar to the guanosine derivative (E: Entecavir). It was shown that a similar thermodynamic effect is obtained regardless of the type of the base.

### [Example 15]

### Purpose: Evaluation of in vitro effect of oligonucleotide drug incorporating nucleoside (A) in the strand on inhibiting target gene expression

### Method and Results:

ASO (mPCS2, mPCS2-gap1, mPCS2-gap2, mPCS2-gap3, mPCS2-gapT1, and mPCS2-gapT2 described in the aforementioned Table 7) was diluted to a final concentration of 1 µM in a cell culture medium (9 mM CaCl₂ added) and added to a 96-well plate. Huh-7 cells diluted in the above-mentioned medium were seeded by 10,000 cells into each well and cultured in an incubator for 24 hr. Then, cDNA was prepared according to the manufacturer's protocol from the cell lysate by using SuperPrep (registered trademark) II Cell Lysis & RT Kit for qPCR (TOYOBO). The expression level of Pcsk9 mRNA was analyzed using a CFX real-time PCR system (BIO RAD). For the analysis of human PCSK9, hPCSK9-F: 5'-AAGGGAAGGGCACGGTTAG-3' (SEQ ID NO: 11) and hPCSK9-R: 5'-GAGTAGAGGCAGGCATCGTC-3' (SEQ ID NO: 12) were used. For the analysis of human Gapdh as a housekeeping gene, the primer set of hGAPDH-F: 5'-GCACCGTCAAGGCTGAGAAC-3' (SEQ ID NO: 3) and hGAPDH-R: 5'-TGGTGAAGACGCCAGTGGA-3' (SEQ ID NO: 4) was used. KD activity was calculated based on relative expression levels, by converting the difference in Ct values to the difference in expression levels. Significance was tested using Dunnett's multiple comparison test after one-way analysis of variance (ANOVA). **p < 0.01, *p < 0.05. "ns" no significant difference (p> 0.05)

The evaluation results are shown in Fig. 15.

### Discussion:

Even when a thymidine derivative of a carbocyclic DNA was introduced into the strand, knockdown activity equivalent to that of the parent sequence (mPCS2) could be obtained. It was clarified that similar efficacy is obtained regardless of the type of base of nucleoside (A).

### [Example 16]

### Purpose: Evaluation of cytotoxicity (cytotoxicity rate) of oligonucleotide drug incorporating nucleoside (A) in the strand (LDH assay)

### (Cell experiment)

ASO was diluted to a final concentration of 2 µM in a cell culture medium (9 mM CaCl₂ added) and added to a 96-well plate. Huh-7 cells diluted in the above-mentioned medium were seeded by 10,000 cells into each well and cultured in an incubator for 72 hr.

### (LDH assay)

Lysis solution (10 µL) was added to untreated cells and incubated at 37°C for 30 min. The supernatant (80 µL) was transferred from each well to another 96-well plate, and working solution (80 µL) was added to each well. After incubation at room temperature for 30 min, stop solution (40 µL) was added to each well. Absorbance at 490 nm was measured using a microplate reader, and the background value was subtracted from the control value or assay value. Cytotoxicity rate was calculated by ((experiment sample)-(low control))/((high control)-(low control)) x 100.

Significance was tested using Dunnett's multiple comparison test after one-way analysis of variance (ANOVA). **p < 0.01, *p < 0.05. "ns" no significant difference (p> 0.05) The evaluation results are shown in Fig. 16.

### Discussion:

Compared to the parent strand mPCS2, ASOs incorporating carbocyclic DNA guanosine derivatives (E) and thymidine derivatives (E^{T}) showed a high probability of reduced or eliminated cytotoxicity. It was suggested that introduction sites that do not affect the toxicity of the parent strand also exist.

Therefrom it was shown that nucleic acid drugs with reduced toxicity can be obtained by introducing nucleosides (A) into the ASO strand, regardless of the target gene and base sequence.

### [Example 17]

### Purpose: Evaluation of cytotoxicity (cell survival rate) of oligonucleotide drug incorporating nucleoside (A) in the strand (PI staining/dead cell staining)

### (Cell experiment)

ASO was diluted to a final concentration of 2 µM in a cell culture medium (9 mM CaCl₂ added) and added to a 96-well plate. Huh-7 cells diluted in the above-mentioned medium were seeded by 10,000 cells into each well and cultured in an incubator for 96 hr.

### (PI staining/dead cell staining)

The medium was replaced with cell culture medium (9 mM CaCl₂ added, 100 µL), and PI solution (Dojin Chemical Co., Ltd.) diluted to a final concentration of 1.0 pg/mL was added to each well. The cells were incubated at 37°C for 15 min and observed using a fluorescence microscope.

The evaluation results are shown in Fig. 17. Each Figure shows, from left to right, bright-field, PI, and merge (bright-field/PI) images. Stained dead cells exhibit white fluorescence in the PI image. Almost no PI-positive cells were observed in the control (NT) group. Compared to the control (NT) group, many PI-positive cells were observed and cell death was observed in parent strand mPCS2 and gap3 incorporating guanosine derivative (E) of carbocyclic DNA. On the other hand, the number of PI-positive cells decreased markedly in gap1 and gap2, each incorporating guanosine derivative (E) of carbocyclic DNA, and gapT1 and gapT2, which were ASOs incorporating thymidine derivative (E^{T}).

### Discussion:

Similar to the LDH assay results in Example 16, a similar tendency was confirmed in the PI staining experiment in Example 17.

### [Example 18]

### Purpose: Measurement of melting temperature (Tₘ) of double-strand formed with complementary strand RNA

### Method and Results

### (1) Synthesis of oligonucleotide

In the same manner as in the method described in Example 2, various oligonucleotides with hApoC3 as the parent sequence (nucleotide linkage: all phosphorothioate linked) described in the following Table 8 were synthesized and subjected to the following evaluation.

### (2) Measurement of melting temperature (Tₘ) of double-strand formed with complementary strand RNA (melting temperature (Tm) measurement (Double strand formation ability evaluation))

A sample solution (150 µL) with final concentrations of 10 mM phosphate buffer (pH 7.0), 100 mM sodium chloride, 0.1 mM ethylenediaminetetraacetic acid, and 4 µM of each oligonucleotide and complementary RNA was heated to 95°C for 3 min, then annealed by slow cooling to 20°C at a rate of 1°C per min, and the measurement was started. The temperature was increased to 95°C at a rate of 0.5°C per min, and the absorbance at 260 nm was plotted at 1°C intervals. All Tₘ values were calculated using the midline method.

The measurement results are shown in the following Table 8. In the Table, E represents Entecavir.

**[Table 8]**

| Abbreviation | ID | | 1 | 2 | 3 | 4 | 5 | 6 | 7 | 8 | 9 | 10 | 11 | 12 | 13 | 14 | | *T*m (°C) | Δ*T*m (°C) |
|---|---|---|---|---|---|---|---|---|---|---|---|---|---|---|---|---|---|---|---|
| hApoC3 | hApoC3-BNA(14) | 5' | A | C | C | t | g | g | g | a | c | t | c | C | T | G | 3' | 76.4 | - |
| gap1 | hApoC3-BNA(14)-gap1 | | A | C | C | t | E | g | g | a | c | t | c | C | T | G | | 76.7 | +0.3 |
| gap2 | hApoC3-BNA(14)-gap2 | | A | C | C | t | g | E | g | a | c | t | c | C | T | G | | 75.5 | -0.8 |
| gap3 | hApoC3-BNA(14)-gap3 | | A | C | C | t | g | g | E | a | c | t | c | C | T | G | | 76.4 | -0.0 |
| | hApoC3-BNA(14)-cRNA | 3' | U | G | G | A | C | C | C | U | G | A | G | G | A | C | 5' | | |

### Discussion:

The introduction of the nucleoside (A) into the ASO hardly affected the thermodynamic stability of the ASO-target RNA complex (SD = 0.52 oC/mod). Considering that a positive correlation has also been found between thermodynamic stability and toxicity (see Reference 1 below), this derivative can be said to be a superior artificial nucleic acid that enables adjustment of toxicity parameters without altering the target binding ability of ASO.
1) Watt AT, Swayze G, Swayze EE, Freier SM. Likelihood of Nonspecific Activity of Gapmer Antisense Oligonucleotides Is Associated with Relative Hybridization Free Energy. Nucleic Acid Ther. 2020 Aug; 30(4):215-228. doi: 10.1089/nat.2020.0847.

### [Example 19]

### Purpose: Evaluation of cytotoxicity of oligonucleotide drug incorporating nucleoside (A) in the strand

### Method and Results:

### (Cell experiment)

ASO described in the aforementioned Table 8 was diluted to a final concentration of 2 µM in a cell culture medium (9 mM CaCl₂ added) and added to a 96-well plate. Huh-7 cells diluted in the above-mentioned medium were seeded by 10,000 cells into each well and cultured in an incubator for 72 hr, and each assay was performed.

### (Cell survival rate assay)

Cell Counting Kit-8 (Dojin Chemical Co., Ltd.) was added at 10 µL (/well) and the mixture was incubated in an incubator for 2 hr. The absorbance at 450 nm was measured using a microplate reader, and the background value was subtracted from the control value or assay value. Significance was tested using Dunnett's multiple comparison test after one-way analysis of variance (ANOVA). **p < 0.01, *p < 0.05. "ns" no significant difference (p> 0.05)

The evaluation results are shown in Fig. 18.

### Discussion:

Compared to the parent strand hApoC3, gap1, gap2, and gap3 all showed a decrease or elimination of cytotoxicity. Therefrom it was shown that nucleic acid drugs with reduced cytotoxicity can be obtained by introducing nucleosides (A), regardless of the target gene and base sequence.

### [Example 20]

### Purpose: Evaluation of in vitro effect of oligonucleotide drug incorporating nucleoside (A) in the strand on inhibiting target gene expression

### Method and Results:

### (Cell experiment)

ASO was diluted to a final concentration of 1 µM in a cell culture medium (9 mM CaCl₂ added) and added to a 96-well plate. Huh-7 cells diluted in the above-mentioned medium were seeded by 10,000 cells into each well and cultured in an incubator for 24 hr. Then, cDNA was prepared according to the manufacturer's protocol from the cell lysate by using SuperPrep (registered trademark) II Cell Lysis & RT Kit for qPCR (TOYOBO). The expression level of ApoC3 mRNA was analyzed using a CFX real-time PCR system (BIO RAD). For the analysis of human ApoC3, hApoC3-F: 5'-CTGCTCCAGGAACAGAGGTG-3' (SEQ ID NO: 13) and hApoC3-R: 5'-GTGGCGTGCTTCATGTAACC-3' (SEQ ID NO: 14) were used. For the analysis of human Gapdh as a housekeeping gene, the primer set of hGAPDH-F: 5'-GCACCGTCAAGGCTGAGAAC-3' (SEQ ID NO: 3) and hGAPDH-R: 5'-TGGTGAAGACGCCAGTGGA-3' (SEQ ID NO: 4) was used. KD activity was calculated based on relative expression levels, by converting the difference in Ct values to the difference in expression levels. Significance was tested using Dunnett's multiple comparison test after one-way analysis of variance (ANOVA). **p < 0.01, *p < 0.05. "ns" no significant difference (p> 0.05)

The evaluation results are shown in Fig. 19.

### Discussion:

ASOs (gap1-3) with reduced cytotoxicity compared to hApoC3 (parent strand) all exhibited knockdown activity. In particular, gap1 and gap3 showed activity equivalent to that of the parent strand. Therefrom it was shown that the introduction of nucleoside (A) can improve safety while maintaining drug efficacy.

### [Example 21]

### Purpose: Verification of influence of nucleoside (A) equipped with targeting ligand on toxicity of oligonucleotide drug using mouse

### Method and Results

### (1) Synthesis of oligonucleotide drug containing nucleoside (A) equipped with targeting ligand

In the same manner as in the method described in Example 8, oligonucleotides equipped with various targeting ligands with hApoC3 as the parent sequence, described in the following Table 9, (nucleotide linkage: two linkages from 5' end are phosphodiester linkages, and the remaining linkages are phosphorothioate linkages) were synthesized and subjected to the following evaluation. In the Table, E represents Entecavir.

**[Table 9]**

| Abbreviation | | | | 1 | 2 | 3 | 4 | 5 | 6 | 7 | 8 | 9 | 10 | 11 | 12 | 13 | 14 | |
|---|---|---|---|---|---|---|---|---|---|---|---|---|---|---|---|---|---|---|
| hApoC3 | 5' | X | X | A | C | C | t | g | g | g | a | c | t | c | C | T | G | 3' |
| gap1 | | X | X | A | C | C | t | E | g | g | a | c | t | c | C | T | G | |
| gap2 | | X | X | A | C | C | t | g | E | g | a | c | t | c | C | T | G | |
| gap3 | | X | X | A | C | C | t | g | g | E | a | c | t | c | C | T | G | |

### (2) Measurement of KD activity of ASO in mouse liver

All animal experiment protocols were practiced after approved by the Animal Experiment Committee of Nagasaki University. ASO targeting human ApoC3 having no homology to mouse was administered subcutaneously at a single dose of 200 nmol/kg to 7-week-old C57Bl/6J (male, Japan SLC). Body weight change was observed and, after 72 hours from administration, whole blood was collected under isoflurane inhalation anesthesia. Serum collected from inferior vena cava blood was used, and ALT, AST, and total bilirubin were measured by Oriental Yeast Co., Ltd. For statistical analysis, the Smirnov-Grubbs test was performed, followed by significance testing using either one-way ANOVA (AST, ALT, T-BIL) or two-way ANOVA (weight change) followed by Dunnett's multiple comparison test (**p < 0.01, *p < 0.05. "ns" No significant difference (p> 0.05)). For statistical analysis, the Smirnov-Grubbs test was performed, after which significance testing using either one-way analysis of variance (AST, ALT, T-BIL) or two-way analysis of variance (body weight change) was performed, followed by Dunnett's multiple comparison test (**p < 0.01, *p < 0.05. "ns" no significant difference (p> 0.05)).

The evaluation results are shown in Figs. 20 to 23.

### (Discussion)

Significant weight loss was observed only with hApoC3 (parent strand). No weight loss was observed with any of gap1-3. Furthermore, hepatotoxicity was confirmed with hApoC3 (parent strand). All ASOs with introduced nucleoside (A) showed suppressed increase in liver enzymes and bilirubin level compared to the parent strand. The degree and tendency thereof were consistent with the tendency of cytotoxicity. Therefrom it was clarified that safety can be improved while maintaining efficacy by introducing nucleoside (A). (However, the ASOs used here did not have homology to the mouse ApoC3 gene, and knockdown activity in mice was not confirmed.)

### [Production of nucleoside (A)]

In the following, specific embodiments of nucleosides (A) other than those described in Example 1 are shown along with the production methods thereof (Examples 22-38).

First, the synthesis scheme for compound 13, which serves as a common intermediate in the synthesis of each derivative, is shown below. Compound 8 and subsequent compounds are novel compounds. Various nucleosides (A) can be produced starting from compound 13.

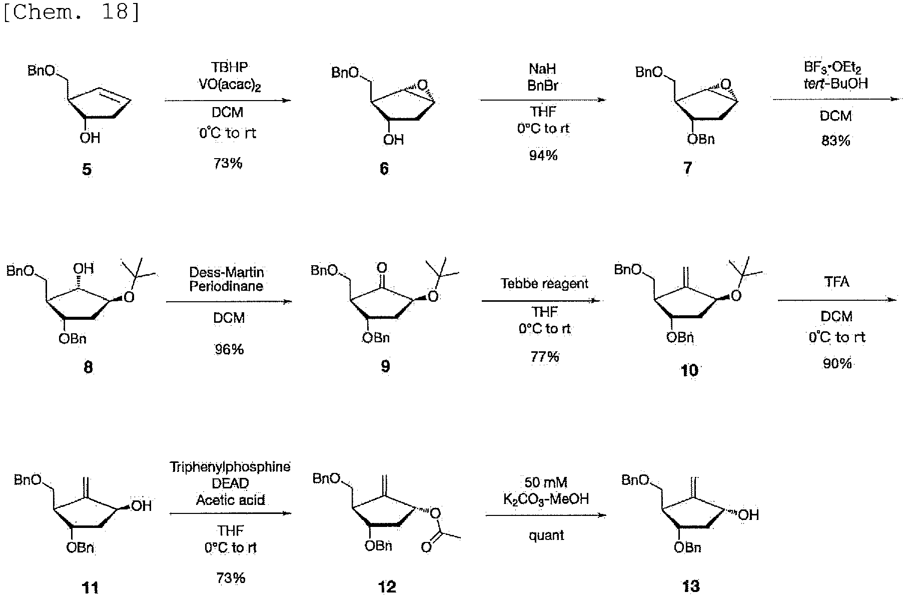

### [Example 22]

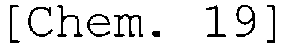

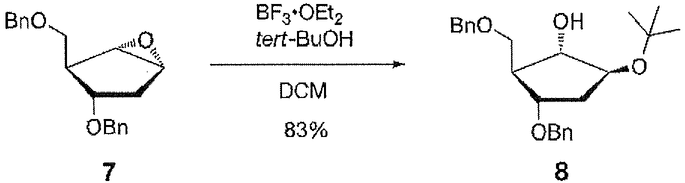

### (1S,2S,3S,5S)-3-(benzyloxy)-2-((benzyloxy)methyl)-5-(tert-butoxy)-cyclopentan-1-ol (8).

Under a nitrogen stream, to an anhydrous dichloromethane solution (100 mL) of compound 7 (3.66 g, 11.8 mmol) was added tert-BuOH (4.48 mL, 47.2 mmol, 4 eq.) dehydrated by MS4A at - 20°C under stirring. At the same temperature, BF₃·OEt₂ (148 µL, 1.18 mmol, 0.1 eq) diluted with anhydrous dichloromethane (30 mL) was added dropwise, and the mixture was stirred at room temperature for 23 hr. To the reaction solution was added saturated sodium hydrogen carbonate (200 mL), and the mixture was extracted 3 times with dichloromethane (200 mL). The organic layer was washed with saturated brine, dehydrated with anhydrous sodium sulfate, and concentrated under reduced pressure to give a crude product. The product was purified by silica gel column chromatography (SiO₂, ethyl acetate/hexane=1:3) to give compound 8 (3.78 g, 83%) as a colorless oily substance. ¹H NMR (400 MHz, CDCl₃) δ 7.36 - 7.21 (m, 11H), 4.58 - 4.44 (m, 3H), 4.40 (d, J = 11.9 Hz, 1H), 3.99 (q, J = 7.8 Hz, 1H), 3.79 - 3.62 (m, 3H), 3.53 (dd, J = 9.1, 7.0 Hz, 1H), 2.43 (dd, J = 3.0, 1.0 Hz, 1H), 2.21 - 1.99 (m, 2H), 1.75 (dt, J = 13.9, 7.9 Hz, 1H), 1.19 (s, 9H).; ¹³C NMR (101 MHz, CDCl₃) δ 138.52, 138.31, 128.48, 127.75, 127.68, 79.44, 77.35, 76.93, 76.41, 73.71, 73.38, 71.11, 70.88, 53.55, 50.34, 38.26, 28.74. HRMS (FAB)
m/z: [M + H]+ calcd for C₂₄H₃₃O₄, 385.2373; found, 385.2379.

### [Example 23]

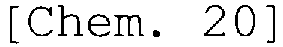

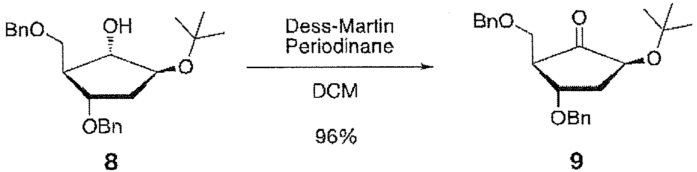

### (2R,3S,5S)-3-(benzyloxy)-2-((benzyloxy)methyl)-5-(tert-butoxy)-cyclopentan-1-one (9).

Under a nitrogen stream, to an anhydrous dichloromethane solution (88 mL) of compound 8 (3.40 g, 8.86 mmol) was added Dess-martin periodinane (4.5 g, 10.63 mmol) at 0°C under stirring, and the mixture was stirred at room temperature for 1 hr. Under stirring at 0°C, sodium thiosulfate aqueous solution (70 mL) and saturated sodium hydrogen carbonate (10 mL) were added, and the mixture was stirred at the same temperature for 20 min. To the reaction solution was added sodium thiosulfate aqueous solution :saturated sodium hydrogen carbonate =7:1 (150 mL), and the mixture was extracted 3 times with dichloromethane (200 mL). The organic layer was washed with saturated brine, dehydrated with anhydrous sodium sulfate, and concentrated under reduced pressure to give compound 9 (3.25 g, 96%) as a yellow oily substance. ¹H NMR (400 MHz, CDCl₃) δ 7.37 - 7.21 (m, 13H), 4.58 - 4.49 (m, 2H), 4.45 (s, 2H), 4.41 - 4.32 (m, 1H), 4.17 (d, J = 5.4 Hz, 1H), 3.67 (dd, J = 9.4, 4.3 Hz, 1H), 3.55 (dd, J = 9.4, 7.2 Hz, 1H), 2.59 (ddd, J = 6.6, 4.4, 2.2 Hz, 1H), 2.41 (ddt, J = 12.9, 8.6, 2.1 Hz, 1H), 1.96 (ddd, J = 13.5, 11.5, 5.5 Hz, 1H), 1.22 (s, 9H).; ¹³C NMR (101 MHz, CDCl₃) δ 215.48, 138.16, 138.03, 128.58, 128.45, 127.84, 127.73, 127.69, 77.45, 77.13, 76.82, 75.80, 74.97, 73.54, 73.31, 70.87, 68.20, 52.38, 36.71, 28.30.

### [Example 24]

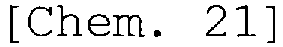

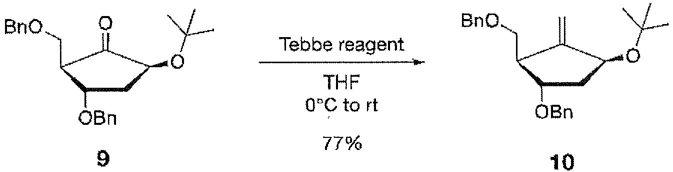

### (((1R,3S,5S)-5-(benzyloxy)-3-(tert-butoxy)-2-methylenecyclopentyl)methoxy)methyl)benzene (10).

Under a nitrogen stream, to an anhydrous tetrahydrofuran solution (50 mL) of compound 9 (2.90 g, 7.59 mmol) was added dropwise 0.5 M Tebbe reagent (19.7 mL, 9.87 mmol) at 0°C under stirring, and the mixture was stirred at room temperature for 50 min. After quenching by adding 0.1 M NaOH aq (50 mL) and stirring for 30 min at 0°C, H₂O (100 mL) was added to the reaction solution, and the mixture was extracted 3 times with ethyl acetate (200 mL). The organic layer was washed with saturated brine, dehydrated with anhydrous sodium sulfate, and concentrated under reduced pressure to give a crude product. The product was purified by silica gel column chromatography (SiO₂, ethyl acetate/hexane=1:3) to give compound 10 (2.22 g, 77%) as a yellow oily substance. ¹H NMR (400 MHz, CDCl₃) δ 7.35 - 7.22 (m, 13H), 5.17 (td, J = 2.5, 0.9 Hz, 1H), 5.07 - 5.03 (m, 1H), 4.59 - 4.42 (m, 6H), 3.92 (dt, J = 5.8, 1.9 Hz, 1H), 3.55 (dd, J = 9.6, 5.5 Hz, 1H), 3.31 (t, J = 9.6 Hz, 1H), 2.92 - 2.82 (m, 1H), 2.13 (tt, J = 7.3, 1.7 Hz, 1H), 1.61 (ddd, J = 13.4, 10.0, 5.8 Hz, 2H), 1.22 (s, 9H).; ¹³C NMR (101 MHz, CDCl₃) δ 152.40, 138.84, 138.43, 128.42, 127.76, 127.65, 127.51, 108.37, 79.10, 77.44, 77.33, 77.12, 76.80, 73.82, 73.09, 72.94, 72.18, 70.53, 47.70, 39.63, 28.63. HRMS (FAB) m/z: [M + H]+ calcd for C₂₅H₃₃O₃, 381.2424; found, 381.2428.

### [Example 25]

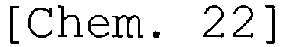

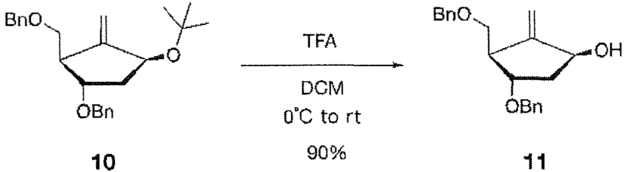

### (1S,3R,4S)-4-(benzyloxy)-3-((benzyloxy)methyl)-2-methylenecyclo-pentan-1-ol (11).

Under a nitrogen stream, to an anhydrous dichloromethane solution (37 mL) of compound 10 (2.14 g, 5.64 mmol) was added trifluoroacetic acid (14 mL) at 0°C under stirring, and the mixture was stirred at room temperature for 1 hr. Trifluoroacetic acid (5 mL) was further added, and the mixture was stirred for 30 min. Then, cold saturated sodium hydrogen carbonate solution (200 mL) was added to the reaction solution, and the aqueous layer was extracted with dichloromethane (200 mL). The organic layer was washed with saturated brine, dehydrated with anhydrous sodium sulfate, and concentrated under reduced pressure to give a crude product. The product was purified by silica gel column chromatography (SiO₂, ethyl acetate/hexane=1:3). The compound in which the secondary hydroxyl group was trifluoroacetylated during the reaction was recovered and hydrolyzed with 50 mM potassium carbonate-methanol solution (10 mL). To the reaction solution was added saturated aqueous sodium hydrogen carbonate solution (100 mL), and the mixture was extracted 3 times with ethyl acetate (100 mL). The organic layer was washed with saturated brine, dehydrated with anhydrous sodium sulfate, and concentrated under reduced pressure to give a combined compound 11 (1.66 g, 90%) as a yellow oily substance. ¹H NMR (400 MHz, CDCl₃) δ 7.38 - 7.23 (m, 13H), 5.30 - 5.25 (m, 1H), 5.09 (t, J = 1.8 Hz, 1H), 4.59 - 4.44 (m, 6H), 4.17 - 4.08 (m, 1H), 3.61 (dd, J = 9.2, 4.3 Hz, 1H), 3.51 (dd, J = 9.2, 6.1 Hz, 1H), 2.81 (dtt, J = 6.4, 4.3, 2.3 Hz, 1H), 2.20 (d, J = 6.2 Hz, 1H), 1.99 (t, J = 5.7 Hz, 2H).^{; 13}C NMR (101 MHz, CDCl₃) δ 153.37, 138.61, 138.00, 128.55, 128.47, 127.85, 127.79, 127.66, 110.21, 80.15, 77.48, 77.16, 76.84, 74.04, 73.38, 72.08, 71.17, 49.27, 40.85. HRMS (FAB) m/z: [M + H]+ calcd for C₂₁H₂₅O₃, 325.1798; found, 325.1813.

### [Example 26]

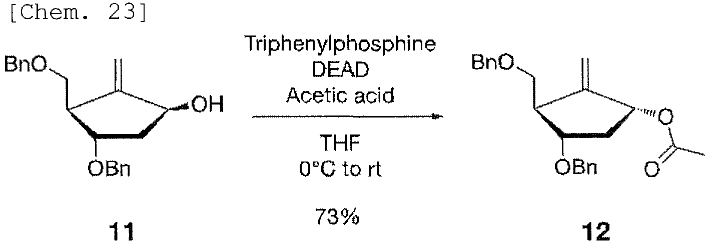

### (1R,3R,4S)-4-(benzyloxy)-3-((benzyloxy)methyl)-2-methylenecyclopentyl acetate (12).

Under a nitrogen stream, to an anhydrous tetrahydrofuran solution (22 mL) of triphenyl phosphine (2.37 g, 9.05 mmol) was added DEAD (4.2 mL, 9.05 mmol) at -20°C under stirring, and the mixture was stirred at the same temperature for 20 min. Compound 11 (1.42 g, 4.52 mmol) diluted with anhydrous tetrahydrofuran solution (10 mL) was added, and the mixture was stirred at the same temperature for 10 min. Acetic acid (679 µL, 11.3 mmol) was added, and the mixture was stirred at room temperature for 20 hr. To the reaction solution was added saturated aqueous sodium hydrogen carbonate solution (200 mL), and the mixture was extracted 3 times with ethyl acetate (200 mL). The organic layer was washed with saturated brine, dehydrated with anhydrous sodium sulfate, and concentrated under reduced pressure to give a crude product. The product was purified by silica gel column chromatography (SiO₂, ethyl acetate/hexane=1:4) to give compound 12 (1.20 g, 73%) as a colorless oily substance. ¹H NMR (400 MHz, CDCl₃) δ 7.37 - 7.23 (m, 11H), 5.48 - 5.39 (m, 1H), 5.24 (t, J = 2.2 Hz, 1H), 5.18 (t, J = 2.2 Hz, 1H), 4.55 - 4.44 (m, 4H), 3.90 (q, J = 6.1 Hz, 1H), 3.59 - 3.45 (m, 2H), 2.94 (tq, J = 5.6, 2.7 Hz, 1H), 2.49 (ddd, J = 13.5, 7.3, 6.2 Hz, 1H), 2.08 (s, 3H), 1.79 (dt, J = 13.1, 6.4 Hz, 1H).; ¹³C NMR (101 MHz, CDCl₃) δ 171.05, 148.74, 138.53, 138.32, 128.46, 127.77, 127.70, 127.67, 111.95, 78.77, 77.46, 77.14, 76.82, 74.52, 73.23, 71.30, 70.98, 49.64, 37.42, 21.42. HRMS (FAB) m/z: [M + H]+ calcd for C₂₃H₂₇O₄, 367.1904; found, 367.1908.

### [Example 27]

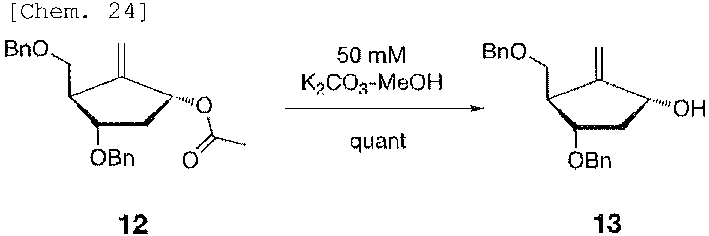

### (1R,3R,4S)-4-(benzyloxy)-3-((benzyloxy)methyl)-2-methylenecyclopentan-1-ol (13).

Under a nitrogen stream, to compound 12 (1.06 g, 2.89 mmol) was added 50 mM potassium carbonate-methanol (20 mL) and the mixture was stirred at room temperature for 1 hr. H₂O (5 mL) was added, and the mixture was concentrated under reduced pressure. To the residue were added ethyl acetate (100 mL) and saturated sodium hydrogen carbonate (100 mL), and the mixture was extracted with ethyl acetate. The organic layer was washed with saturated brine, dehydrated with anhydrous sodium sulfate, and concentrated under reduced pressure to give compound 13 (935 mg, quant) as a colorless oily substance. ¹H NMR (400 MHz, CDCl₃) 6 7.36 - 7.26 (m, 10H), 5.37 (t, J = 1.9 Hz, 1H), 5.14 (t, J = 1.8 Hz, 1H), 4.56 - 4.42 (m, 4H), 4.38 (s, 1H), 4.05 (dt, J = 5.6, 3.0 Hz, 1H), 3.49 (dd, J = 9.5, 5.1 Hz, 1H), 3.29 (dd, J = 9.5, 8.1 Hz, 1H), 3.13 - 3.02 (m, 1H), 2.48 (d, J = 9.8 Hz, 1H), 2.09 (ddd, J = 13.8, 6.0, 5.1 Hz, 1H), 1.94 (dtd, J = 13.9, 3.6, 1.5 Hz, 1H).

### [Example 28]

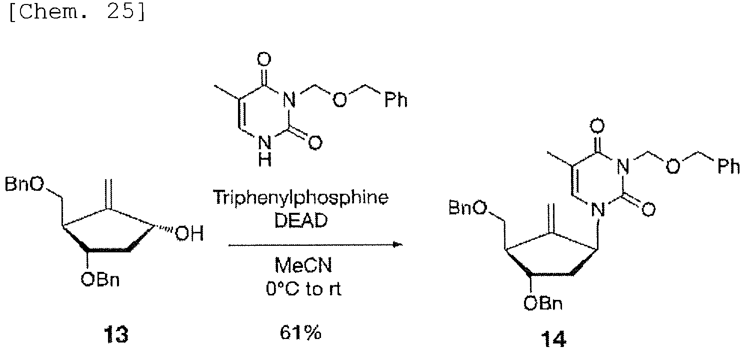

### 1-((1S,3R,4S)-4-(benzyloxy)-3-((benzyloxy)methyl)-2-methylenecyclopentyl)-3-((benzyloxy)methyl)-5-methylpyrimidine-2,4(1H,3H)-dione (14).

Under a nitrogen stream, to an anhydrous acetonitrile solution (2 mL) of triphenyl phosphine (297 mg, 1.13 mmol) was added DEAD (514 µL, 1.13 mmol) at 0°C under stirring, and the mixture was stirred at the same temperature for 20 min. N3-(benzyloxymethyl)thymine (209 mg, 0.85 mmol) and compound 13 (184 mg, 0.566 mmol) diluted with anhydrous acetonitrile solution (2 mL) were added, and the mixture was stirred at room temperature for 17 hr. To the reaction solution was added saturated aqueous sodium hydrogen carbonate solution (100 mL), and the mixture was extracted 3 times with ethyl acetate (100 mL). The organic layer was washed with saturated brine, dehydrated with anhydrous sodium sulfate, and concentrated under reduced pressure to give a crude product. The crude product was purified by silica gel column chromatography (SiO₂, ethyl acetate/hexane=1:3) to give compound 14 (189 mg, 61%) as a white foamy solid. ¹H NMR (400 MHz, CDCl₃) δ 7.40 - 7.26 (m, 15H), 7.06 (t, J = 1.2 Hz, 1H), 5.74 (t, J = 9.4 Hz, 1H), 5.51 (s, 2H), 5.24 (t, J = 2.5 Hz, 1H), 4.93 (t, J = 2.5 Hz, 1H), 4.71 (s, 2H), 4.57 - 4.44 (m, 4H), 4.12 - 4.06 (m, 1H), 3.79 - 3.65 (m, 2H), 2.90 (s, 1H), 2.34 (dd, J = 13.2, 8.1 Hz, 1H), 1.98 (ddd, J = 13.1, 10.1, 5.1 Hz, 1H), 1.57 (d, J = 1.2 Hz, 3H).; ¹³C NMR (101 MHz, CDCl₃) δ 163.68, 152.15, 149.17, 138.24, 137.88, 137.23, 128.66, 128.54, 128.46, 128.38, 128.01, 127.76, 127.67, 127.58, 111.32, 110.29, 80.24, 77.33, 73.67, 72.94, 72.31, 70.94, 70.74, 58.30, 49.58, 36.50, 12.95. HRMS (FAB) m/z: [M + H]⁺calcd for C₃₄H₃₇N₂O₅, 553.2697; found, 553.2702.

### [Example 29]

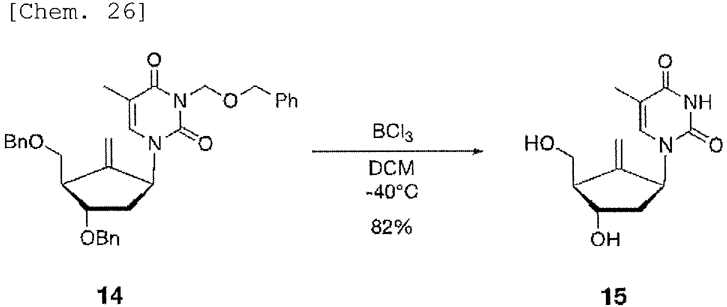

### 1-((1S,3R,4S)-4-hydroxy-3-(hydroxymethyl)-2-methylenecyclopentyl)-5-methylpyrimidine-2,4(1H,3H)-dione (15).

Under a nitrogen stream, to an anhydrous dichloromethane solution (1.0 mL) of compound 14 (189 mg, 0.343 mmol) was added 1M BCl₃-dichloromethane solution (3.43 mL, 3.43 mmol) at -40°C under stirring, and the mixture was stirred at the same temperature for 1.5 hr. 2M ammonia-methanol solution (2 mL) was added at -40°C under stirring, and the mixture was stirred at room temperature for 1 hr. The reaction solution was concentrated under reduced pressure and purified by silica gel column chromatography (SiO₂, dichloromethane/methanol=10:1) to give compound 15 (71 mg, 82%) as a colorless oily substance. ¹H NMR (400 MHz, CD₃OD) δ 7.37 (d, J = 1.4 Hz, 1H), 5.61 (td, J = 9.2, 4.5 Hz, 1H), 5.26 (t, J = 2.5 Hz, 1H), 4.91 (t, J = 2.5 Hz, 1H), 4.30 (p, J = 2.6 Hz, 1H), 3.73 (qd, J = 11.0, 5.4 Hz, 2H), 2.59 (d, J = 6.8 Hz, 1H), 2.07 (dd, J = 9.4, 3.6 Hz, 2H), 1.83 (d, J = 1.2 Hz, 3H). ¹³C NMR (101 MHz, CD₃OD) δ149.69, 139.50, 110.51, 109.84, 71.87, 63.46, 57.66, 53.65, 48.44, 48.23, 48.01, 38.17, 11.16. HRMS (FAB) m/z: [M + H]⁺ calcd for C₁₂H₁₇N₂O₄, 253.1183; found, 253.1191.

### [Example 30]

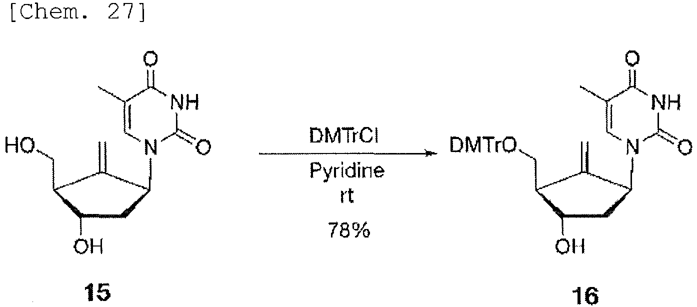

To compound 15 (71 mg, 0.282 mmol) was added anhydrous pyridine (2.8 mL) under a nitrogen atmosphere, then 4,4'-dimethoxytrityl chloride (142 mg, 0.42 mmol) was added, and the mixture was stirred at room temperature for 20 hr. To the reaction solution was added saturated aqueous sodium hydrogen carbonate solution (100 mL), and the mixture was extracted 3 times with ethyl acetate (100 mL). The organic layer was washed with saturated brine, dehydrated with anhydrous sodium sulfate, and concentrated under reduced pressure to give a crude product. The crude product was purified by silica gel column chromatography (SiO₂, dichloromethane/methanol=97:3, 0.5% triethylamine added) to give compound 16 (122 mg, 78%) as a white foamy solid. ¹H NMR (400 MHz, CDCl₃) δ 8.41 (s, 1H), 7.42 - 7.33 (m, 2H), 7.32 - 7.19 (m, 9H), 6.89 (d, J = 1.4 Hz, 1H), 6.87 - 6.76 (m, 4H), 5.71 (t, J = 9.0 Hz, 1H), 4.95 (t, J = 2.6 Hz, 1H), 4.85 (t, J = 2.6 Hz, 1H), 4.40 (d, J = 5.1 Hz, 1H), 3.78 (d, J = 0.9 Hz, 6H), 3.59 (dd, J = 9.3, 4.1 Hz, 1H), 3.21 (dd, J = 9.3, 6.3 Hz, 1H), 2.68 (s, 1H), 2.27 - 2.16 (m, 2H), 2.11 (ddd, J = 13.5, 9.0, 5.7 Hz, 1H), 1.54 (d, J = 1.2 Hz, 3H).; ¹³C NMR (101 MHz, CDCl₃) δ163.93, 158.73, 151.56, 148.84, 144.53, 138.09, 135.76, 135.48, 130.20, 128.23, 128.08, 127.18, 113.31, 111.47, 111.21, 87.09, 77.46, 77.14, 76.83, 73.91, 65.29, 56.90, 55.35, 51.63, 39.40, 12.16. HRMS (FAB) m/z: [M + H]⁺calcd for C₃₃H₃₅N₂O₆, 555.2490; found, 555.2502.

### [Example 31]

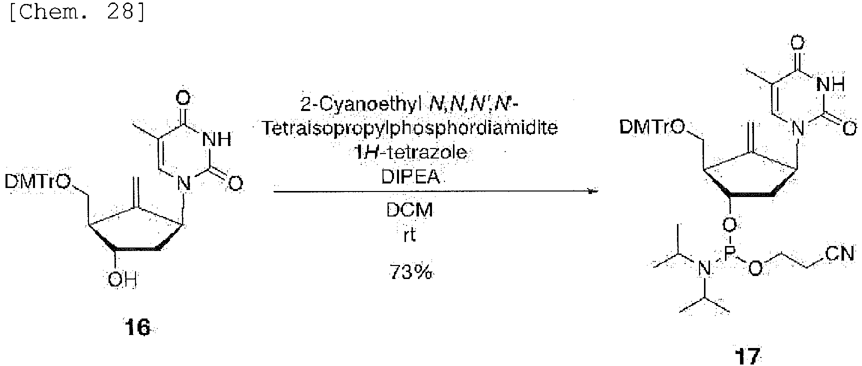

Compound 16 (122 mg, 0.220 mmol) and 1H-tetrazole (23 mg, 0.33 mmol) were added to a heated and dried reaction vessel. anhydrous dichloromethane (2.2 mL) and DIPEA (56 µL, 0.33 mmol) were added under a nitrogen atmosphere. 2-Cyanoethyl N,N,N',N'-tetraisopropylphosphordiamidite (105 µL, 0.33 mmol) was added, and the mixture was stirred at room temperature for 1.5 hr. After confirming the consumption of the starting material by TLC, saturated NaHCO₃ solution (150 mL) was added, and the mixture was extracted three times with ethyl acetate (30 mL). The organic layer was washed with saturated brine, dehydrated with anhydrous sodium sulfate, and concentrated under reduced pressure to give a crude product. The crude product was purified by silica gel column chromatography (SiO₂, dichloromethane/methanol=97:3, 1% triethylamine added) to give compound 17 (121 mg, 73%) as a white foamy solid. ¹H NMR (400 MHz, CDCl₃) δ 8.37 (s, 1H), 7.38 (dt, J = 8.2, 1.5 Hz, 2H), 7.34 - 7.16 (m, 9H), 7.05 - 6.88 (m, 1H), 6.81 (dt, J = 8.9, 2.0 Hz, 4H), 5.72 (t, J = 9.7 Hz, 1H), 4.98 (dt, J = 10.1, 2.6 Hz, 1H), 4.86 (dt, J = 12.3, 2.5 Hz, 1H), 4.53 (d, J = 9.5 Hz, 1H), 3.87 - 3.64 (m, 8H), 3.62 - 3.42 (m, 3H), 3.29 - 3.13 (m, 1H), 2.90 - 2.77 (m, 1H), 2.62 (t, J = 6.3 Hz, 1H), 2.51 (t, J = 6.4 Hz, 1H), 2.41 - 2.26 (m, 1H), 2.19 - 2.03 (m, 1H), 1.43 (dd, J = 7.8, 1.2 Hz, 3H), 1.21 - 1.07 (m, 12H).; ³¹P NMR (162 MHz, CDCl₃) δ 147.97, 147.69. HRMS (FAB) m/z: [M + H]⁺calcd for C₄₂H₅₂N₄O₇P, 755.3574; found, 755.3574.

### [Example 32]

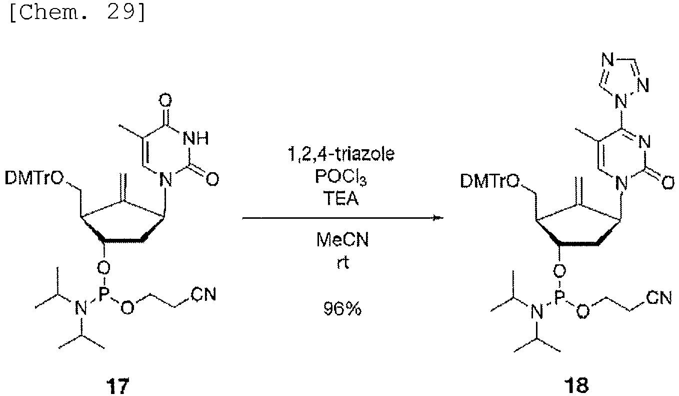

1,2,4-Triazole (123 mg, 1.78 mmol) was added to a heated and dried reaction vessel, and anhydrous acetonitrile (2 mL) was added under a nitrogen atmosphere. Phosphoryl chloride (37.5 µL, 0.403 mmol) was added dropwise, and the mixture was stirred at room temperature for 10 min. Triethylamine (331 µL, 2.385 mmol) was added dropwise, and the mixture was further stirred at room temperature for 30 min. Successively, compound 17 (40 mg, 0.053 mmol) dissolved in anhydrous acetonitrile (1 mL) was added dropwise, and the mixture was further stirred at room temperature for 1 hr. After confirming the consumption of the starting material by TLC, saturated aqueous sodium hydrogen carbonate solution (30 mL) was added, and the mixture was extracted three times with ethyl acetate (30 mL). The organic layer was washed with saturated brine, dehydrated with anhydrous sodium sulfate, and concentrated under reduced pressure to give compound 18 (41 mg, 96%) as a white foamy solid. ¹H NMR (400 MHz, CDCl₃) δ 9.26 (s, 1H), 8.07 (d, J = 1.0 Hz, 1H), 7.76 - 7.65 (m, 1H), 7.43 - 7.34 (m, 2H), 7.33 - 7.21 (m, 9H), 6.82 (ddd, J = 8.9, 2.1, 1.3 Hz, 4H), 5.93 (m, 1H), 5.05 (d, J = 15.1 Hz, 1H), 4.96 - 4.83 (m, 1H), 4.65 - 4.58 (m, 1H), 3.90 - 3.65 (m, 9H), 3.66 - 3.48 (m, 3H), 3.40 - 3.24 (m, 1H), 2.96 - 2.81 (m, 1H), 2.64 (t, J = 6.2 Hz, 1H), 2.60 - 2.42 (m, 2H), 2.32 - 2.16 (m, 1H), 1.87 (dd, J = 10.2, 0.8 Hz, 3H), 1.26 - 1.11 (m, 12H).; ³¹P NMR (162 MHz, CDCl₃) δ 148.06, 147.89. HRMS (FAB) m/z: [M + H]⁺ calcd for C₄₄H₅₃N₇O₆P, 806.3789; found, 806.3796.

### [Example 33]

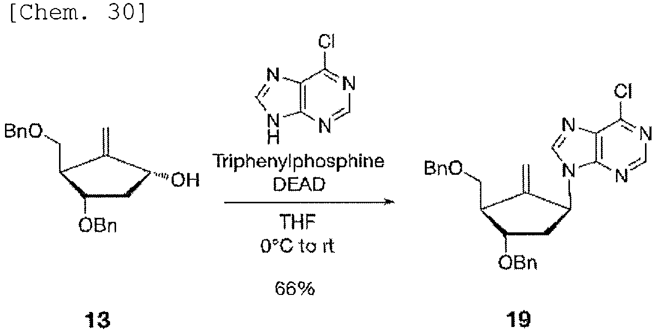

### 9-((1S,3R,4S)-4-(benzyloxy)-3-((benzyloxy)methyl)-2-methylenecyclopentyl)-6-chloro-9H-purine (19)

Under a nitrogen stream, to an anhydrous tetrahydrofuran solution (1 mL) of triphenyl phosphine (81 mg, 0.308 mmol) was added DEAD (140 µL, 0.308 mmol) at 0°C under stirring, and the mixture was stirred at the same temperature for 20 min. 6-Chloropurine (35.5 mg, 0.308 mmol) was added, and the mixture was further stirred for 10 min. Successively, compound 13 (50 mg, 0.154 mmol) diluted with anhydrous tetrahydrofuran solution (500 pL)was added, and the mixture was stirred at room temperature for 24 hr. To the reaction solution was added saturated aqueous sodium hydrogen carbonate solution (20 mL), and the mixture was extracted 3 times with ethyl acetate (20 mL). The organic layer was washed with saturated brine, dehydrated with anhydrous sodium sulfate, and concentrated under reduced pressure to give a crude product. The product was purified by silica gel column chromatography (SiO₂, ethyl acetate/hexane=1:2) to give compound 19 (47 mg, 66%) as a colorless oily substance. ¹H NMR (400 MHz, CDCl₃) δ 8.70 (d, J = 1.4 Hz, 1H), 8.21 (d, J = 1.3 Hz, 1H), 7.42 - 7.26 (m, 10H), 5.76 (t, J = 8.9 Hz, 1H), 5.24 (q, J = 2.1 Hz, 1H), 4.80 (q, J = 2.1 Hz, 1H), 4.57 - 4.48 (m, 4H), 4.25 - 4.15 (m, 1H), 3.76 - 3.66 (m, 2H), 3.05 (s, 1H), 2.45 (ddd, J = 9.3, 4.4, 1.4 Hz, 2H).; ¹³C NMR (101 MHz, CDCl₃) δ 171.05, 148.74, 138.53, 138.32, 128.46, 127.77, 127.70, 127.67, 111.95, 78.77, 77.46, 77.14, 76.82, 74.52, 73.23, 71.30, 70.98, 49.64, 37.42, 21.42. HRMS (FAB) m/z: [M + H]+ calcd for C₂₆H₂₆ClN₄O₂, 461.1739; found, 461.1754.

### [Example 34]

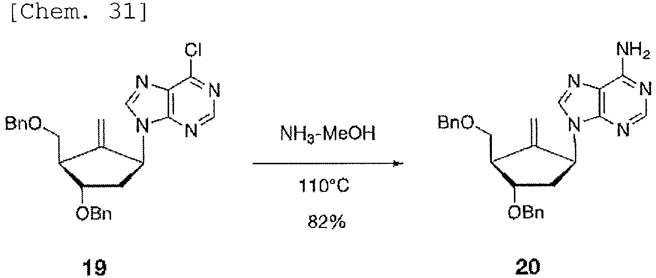

### 9-((1S,3R,4S)-4-(benzyloxy)-3-((benzyloxy)methyl)-2-methylenecyclopentyl)-9H-purin-6-amine (20)

Under a nitrogen stream, to compound 19 (180 mg, 0.392 mmol) was added 7M ammonia-methanol solution (3 mL), and the mixture was stirred at 110°C for 2 hr in a pressure-resistant vial. The reaction solution was cooled to room temperature and concentrated under reduced pressure to give a crude product. The product was purified by silica gel column chromatography (SiO₂, ethyl acetate/hexane=2:1 → dichloromethane/methanol=10:1) to give compound 20 (142 mg, 82%) as a white foamy solid. ¹H NMR (400 MHz, CD₃OD) δ 8.15 (s, 1H), 8.03 (s, 1H), 7.37 - 7.19 (m, 10H), 5.64 (td, J = 8.9, 2.6 Hz, 1H), 5.21 (t, J = 2.4 Hz, 1H), 4.70 (t, J = 2.5 Hz, 1H), 4.60 - 4.46 (m, 4H), 4.18 (dt, J = 4.6, 2.7 Hz, 1H), 3.78 - 3.67 (m, 2H), 3.03 (q, J = 4.4 Hz, 1H), 2.51 - 2.37 (m, 2H).; ¹³C NMR (101 MHz, CD₃OD) δ 155.95, 152.33, 150.08, 149.56, 140.26, 138.44, 138.25, 128.16, 128.04, 127.69, 127.58, 127.50, 127.30, 118.58, 110.14, 79.43, 73.00, 71.56, 70.29, 56.53, 49.34, 48.32, 48.11, 47.90, 47.68, 47.47, 47.26, 47.04, 36.77. HRMS (FAB) m/z: [M + H]+ calcd for C₂₆H₂₈N₅O₂, 442.2238; found, 442.2243.

### [Example 35]

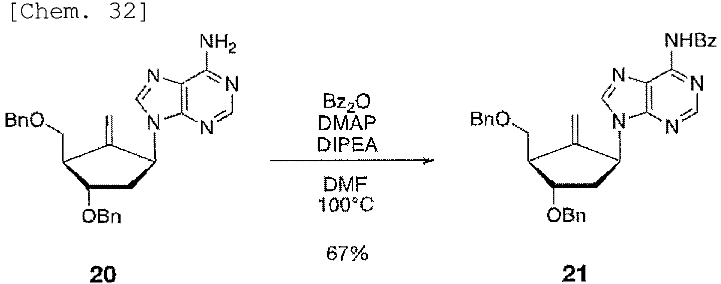

### N-(9-((1S,3R,4S)-4-(benzyloxy)-3-((benzyloxy)methyl)-2-methylenecyclopentyl)-9H-purin-6-yl)benzamide (21)

Under a nitrogen stream, to an anhydrous N,N-dimethylformamide solution (2.9 mL) of compound 20 (131 mg, 0.297 mmol) and 4-dimethylaminopyridine (3.6 mg, 0.030 mmol) was added N,N-diisopropyl ethylamine (76 µL, 0.446 mmol). Successively, benzoic anhydride (134 mg, 0.594 mmol) was added, and the mixture was stirred at 100°C for 7.5 hr. Under icecooling, 2M ammonia-methanol solution (1 mL) was added, and the mixture was stirred for 10 min. To the reaction solution was added saturated aqueous sodium hydrogen carbonate solution (50 mL), and the mixture was extracted 3 times with ethyl acetate (50 mL). The organic layer was washed with saturated brine, dehydrated with anhydrous sodium sulfate, and concentrated under reduced pressure to give a crude product. The product was purified by silica gel column chromatography (SiO₂, ethyl acetate/hexane=1:1) to give compound 21 (109 mg, 67%) as a white foamy solid. ¹H NMR (400 MHz, CDCl₃) δ 9.09 (s, 1H), 8.77 (s, 1H), 8.09 (s, 1H), 8.08 - 7.98 (m, 2H), 7.67 - 7.56 (m, 1H), 7.56 - 7.46 (m, 2H), 7.42 - 7.27 (m, 11H), 5.76 (t, J = 8.8 Hz, 1H), 5.24 (t, J = 2.4 Hz, 1H), 4.83 (t, J = 2.4 Hz, 1H), 4.63 - 4.44 (m, 4H), 4.21 (q, J = 3.8 Hz, 1H), 3.72 (d, J = 5.3 Hz, 2H), 3.05 (s, 1H), 2.47 (dd, J = 8.7, 3.9 Hz, 2H).; ¹³C NMR (101 MHz, CDCl₃) δ164.98, 152.48, 152.33, 149.55, 149.08, 142.70, 138.17, 137.96, 133.83, 132.80, 128.89, 128.62, 128.55, 128.05, 127.96, 127.82, 127.77, 123.22, 111.87, 79.61, 77.51, 77.39, 77.19, 76.87, 73.49, 71.58, 70.95, 56.96, 49.52, 37.63. HRMS (FAB) m/z: [M + H]+ calcd for C₃₃H₃₂N₅O₃, 546.2500; found, 546.2506.

### [Example 36]

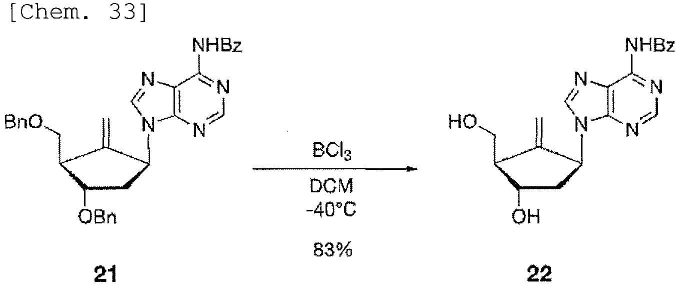

### N-(9-((1S,3R,4S)-4-hydroxy-3-(hydroxymethyl)-2-methylenecyclopentyl)-9H-purin-6-yl)benzamide (22)

Under a nitrogen stream, to an anhydrous dichloromethane solution (500 µL) of compound 21 (108 mg, 0.198 mmol) was added dropwise 1M boron trichloride (1.98 mL, 1.98 mL) at -40°C under stirring. After stirring at the same temperature for 20 min, to the reaction solution were added triethylamine (1 mL) and methanol (1 mL), and the mixture was stirred at room temperature for 30 min. The mixture was concentrated under reduced pressure and purified by silica gel column chromatography (SiO₂, dichloromethane/methanol=20:1). After concentration under reduced pressure, saturated ammonium chloride aqueous solution (40 mL) was added to the residue, and the mixture was extracted five times with ethyl acetate (40 mL). The organic layer was dehydrated with anhydrous sodium sulfate, and concentrated under reduced pressure to give compound 22 (60 mg, 83%) as a white foamy solid. ¹H NMR (400 MHz, CD₃OD) δ 8.67 (s, 1H), 8.49 (s, 1H), 8.12 - 8.01 (m, 2H), 7.67 - 7.60 (m, 1H), 7.55 (dd, J = 8.3, 6.8 Hz, 2H), 5.88 - 5.75 (m, 1H), 5.28 (t, J = 2.5 Hz, 1H), 4.80 (t, J = 2.5 Hz, 1H), 4.45 (dt, J = 5.2, 2.6 Hz, 1H), 3.94 - 3.77 (m, 2H), 2.73 (s, 1H), 2.58 (ddd, J = 13.2, 9.8, 5.0 Hz, 1H), 2.37 - 2.27 (m, 1H).; ¹³C NMR (101 MHz, CD₃OD) δ166.89, 152.14, 151.48, 149.77, 144.13, 133.65, 132.60, 128.45, 128.12, 123.79, 110.83, 71.83, 63.35, 57.16, 54.16, 48.34, 48.13, 47.91, 47.70, 47.48, 47.27, 47.06, 46.54, 39.20. HRMS (FAB) m/z: [M + H]+ calcd for C₁₉H₂₀N₅O₃, 366.1561; found, 366.1566.

### [Example 37]

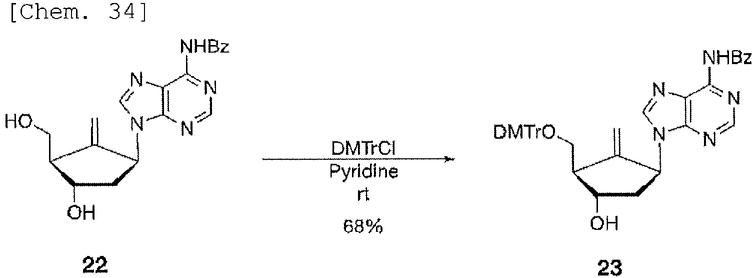

### N-(9-((1S,3R,4S)-3-((bis(4-methoxyphenyl)(phenyl)methoxy)methyl)-4-hydroxy-2-methylenecyclopentyl)-9H-purin-6-yl)benzamide (23)

To compound 22 (60 mg, 0.164 mmol) was added anhydrous pyridine (1.6 mL) under a nitrogen atmosphere, then 4,4'-dimethoxytrityl chloride (67 mg, 0.197 mmol) was added, and the mixture was stirred at room temperature for 6 hr. To the reaction solution was added saturated aqueous sodium hydrogen carbonate solution (40 mL), and the mixture was extracted 3 times with ethyl acetate (40 mL). The organic layer was washed with saturated brine, dehydrated with anhydrous sodium sulfate, and concentrated under reduced pressure to give a crude product. The crude product was purified by silica gel column chromatography (SiO₂, ethyl acetate/hexane=3:1 to dichloromethane/methanol=20:1) to give compound 23 (74 mg, 68%) as a white foamy solid. ¹H NMR (400 MHz, CDCl₃) δ 9.32 (s, 1H), 8.68 (s, 1H), 8.10 - 7.95 (m, 2H), 7.88 (s, 1H), 7.61 - 7.41 (m, 5H), 7.35 - 7.16 (m, 8H), 6.92 - 6.73 (m, 4H), 5.71 (t, J = 8.1 Hz, 1H), 4.99 (t, J = 2.5 Hz, 1H), 4.74 (d, J = 2.5 Hz, 1H), 4.52 (q, J = 5.2 Hz, 1H), 3.78 (s, 6H), 3.56 (ddd, J = 9.6, 5.2, 1.7 Hz, 1H), 3.34 (dd, J = 9.4, 8.1 Hz, 1H), 2.81 (d, J = 6.3 Hz, 1H), 2.36 (qdd, J = 13.6, 7.8, 5.3 Hz, 2H).; ¹³C NMR (101 MHz, CDCl₃) δ 175.48, 165.19, 158.68, 152.19, 149.74, 148.37, 144.67, 142.28, 135.91, 135.80, 133.69, 132.85, 130.11, 128.85, 128.22, 128.08, 127.12, 123.19, 113.33, 112.18, 86.86, 77.49, 77.37, 77.17, 76.85, 73.63, 64.76, 56.22, 55.35, 53.56, 51.95, 39.73, 29.79, 21.04. HRMS (FAB) m/z: [M + H]⁺ calcd for C₄₀H₃₈N₅O₅, 668.2867; found, 668.2873.

### [Example 38]

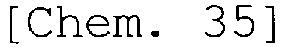

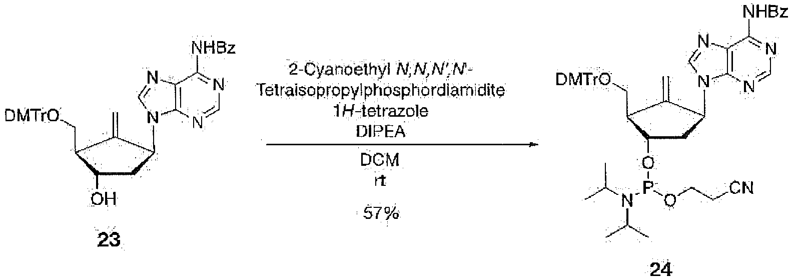

Compound 23 (74.1 mg, 0.111 mmol) and 1H-tetrazole (12 mg, 0.167 mmol) were added to a heated and dried reaction vessel, and anhydrous dichloromethane (1.1 mL) and DIPEA (28 µL, 0.167 mmol) were added under a nitrogen atmosphere. 2-Cyanoethyl N,N,N',N'-tetraisopropylphosphordiamidite (53 µL, 0.167 mmol) was added, and the mixture was stirred at room temperature for 1 hr. After confirming the consumption of the starting material by TLC, saturated aqueous sodium hydrogen carbonate solution (30 mL) was added, and the mixture was extracted three times with ethyl acetate (30 mL). The organic layer was washed with saturated brine, dehydrated with anhydrous sodium sulfate, and concentrated under reduced pressure to give a crude product. The crude product was purified by silica gel column chromatography (SiO₂, ethyl acetate/hexane=1:1 → 2/1, 1% triethylamine added). The residue was dissolved in dichloromethane (1 mL), hexane (49 mL) was added, and the mixture was centrifuged at 25°C, 5000 g for 5 min. The supernatant was removed, and the same operation was repeated on the precipitate. The precipitate was redissolved in dichloromethane and concentrated under reduced pressure to give compound 24 (54.6 mg, 57%) as a white foamy solid. ¹H NMR (400 MHz, CDCl₃) δ 9.00 (s, 1H), 8.68 (d, J = 5.9 Hz, 1H), 8.04 - 7.97 (m, 2H), 7.93 (d, J = 8.4 Hz, 1H), 7.64 - 7.55 (m, 1H), 7.55 - 7.40 (m, 5H), 7.39 - 7.17 (m, 10H), 6.87 - 6.78 (m, 5H), 5.72 (dd, J = 10.2, 7.7 Hz, 1H), 5.06 (dt, J = 6.2, 2.4 Hz, 1H), 4.70 (dt, J = 13.5, 2.4 Hz, 1H), 4.65 - 4.57 (m, 1H), 3.92 - 3.69 (m, 9H), 3.62 (dp, J = 10.3, 6.8 Hz, 2H), 3.47 - 3.34 (m, 2H), 3.01 (d, J = 7.2 Hz, 1H), 2.62 (t, J = 6.3 Hz, 1H), 2.56 - 2.29 (m, 3H), 1.24 - 1.09 (m, 12H).; ³¹P NMR (162 MHz, CDCl₃) δ 148.18, 148.11.; HRMS (FAB) m/z: [M + H]⁺ calcd for C₄₉H₅₅N₇O₆P, 868.3946; found,868.3951.

### [Example 39]

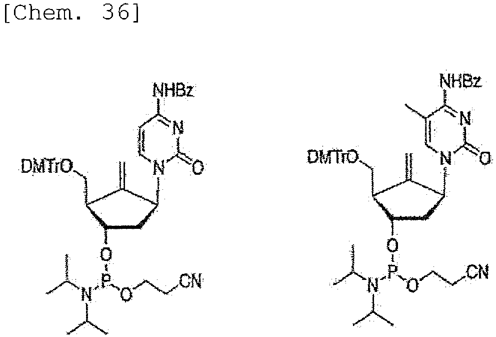

The amidite forms of the two cytosine derivatives shown in the above-mentioned structural formulas can be prepared by those of ordinary skill in the art, for example, by starting with compound 13 (Example 27) and referring to the methods described in Examples 28 to 32, as well as other methods known in the art.

### [Example 40]

### Purpose: Synthesis of siRNA

### Method and Results:

### (1) Synthesis scheme

siRNA was synthesized by preparing commercially available 2'OMe-A(Bz), 2'OMe-G(iBu), 2'OMe-C(Ac), 2'OMe-U, 2'F-A(Bz), 2'F-G(iBu), 2F-C(Ac), 2'F-U, and Entecavir phosphoramidites as 0.1M anhydrous acetonitrile solutions and following the phosphoramidite method.

### (2) Sequence and structure of synthesized siRNA

Oligonucleotides having the sequences shown in the following Table 10 were prepared based on the structure of Vutrisiran (circles indicate PS bonds, and the remaining linkages are phosphodiester bonds). In the Table, E represents Entecavir, N represents 2'-OMe RNA, and Nf represents 2'-F RNA.

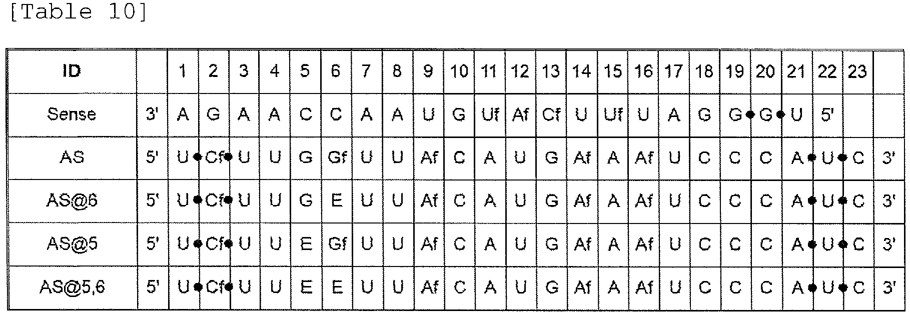

Discussion: The above shows that oligonucleotides containing Entecavir derivatives can be synthesized and siRNA can be constructed according to conventional methods.

### [Example 41]

### Purpose: Evaluation of double-strand formation ability of siRNA with Entecavir derivative introduced into antisense strand Method and Results:

A sample solution (10 µL) prepared by mixing the sense strand (Sense) and each antisense strand synthesized in Example 40 (AS, AS@6, AS@5, AS@5,6) in equivalent amounts was heated to 95°C for 3 min, then annealed by slow cooling to 20°C at a rate of 1°C per min. 10 pmol of each sample was mixed with 20 µM Orange DNA Ladder Dye (6X) (Thermo Fisher Scientific) and 10x TBE, applied to a 20% TBE gel, and electrophoresis was performed at 4°C, 200V for 60 min. The gel after electrophoresis was stained with SYBR Gold Nucleic Acid Gel Stain (Thermo Fisher Scientific) and imaged using FAS-V (Genetics Japan). The evaluation results are shown in Fig. 24.
Discussion: It was suggested that any AS strand with one or more Entecavir derivatives introduced into the strand can form a double strand, like unmodified siRNA, by mixing with the Sense strand.

### [Example 42]

### Purpose: Evaluation of in vitro target gene expression suppressive effect and IC₅₀ of siRNA with Entecavir derivative introduced into the AS strand

### Method and Results:

A sample prepared by mixing the sense strand (Sense) and each antisense strand (AS, AS@6, AS@5, AS@5,6) in equivalent amounts was heated to 95°C for 3 min, then annealed by slow cooling to 20°C at a rate of 1°C per min. The siRNAs annealed in cell culture medium (CaCl₂ added) were diluted to respective final concentrations of 0, 0.01, 0.1, 1, 10, and 100 nM, and added to 96-well plates. Huh-7 cells diluted in the above-mentioned medium were seeded by 10,000 cells into each well and cultured in an incubator for 24 hr. Then, cDNA was prepared according to the manufacturer's protocol from the cell lysate by using SuperPrep (registered trademark) II Cell Lysis & RT Kit for qPCR (TOYOBO). The expression level of TTR mRNA was analyzed using QuantStudio3 (Thermo Fisher Scientific). For the analysis of human TTR, FAM-labeled TaqMan probe (Hs00174914_m1) was used. For the analysis of human Gapdh as a housekeeping gene, VIC-labeled TaqMan probe (Hs02758991_g1) was used. KD activity was calculated based on relative expression levels, by converting the difference in Ct values to the difference in expression levels. 50% Inhibitory concentration (IC₅₀) was calculated from the dose reaction curve. A graph of the evaluation results is shown in Fig. 25. IC₅₀ of each was as shown in Table 11.

**[Table 11]**

| | Vutrisiran | Vutrisiran-A S@6 | Vutrisiran-A S@5 | Vutrisiran-A S@5, 6 |
|---|---|---|---|---|
| IC₅₀ (nM) | 0.33 | 0.37 | 0.43 | 0.34 |

Discussion: High knockdown activity of the same level as Vutrisiran was found even when one or more Entecavir derivatives were introduced into the AS strand of the siRNA of cultured cells.

### [Production of nucleoside (A)]

The following shows the synthesis schemes for representative compounds of nucleoside (A-i-2) and phosphoramidite forms thereof. 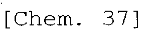 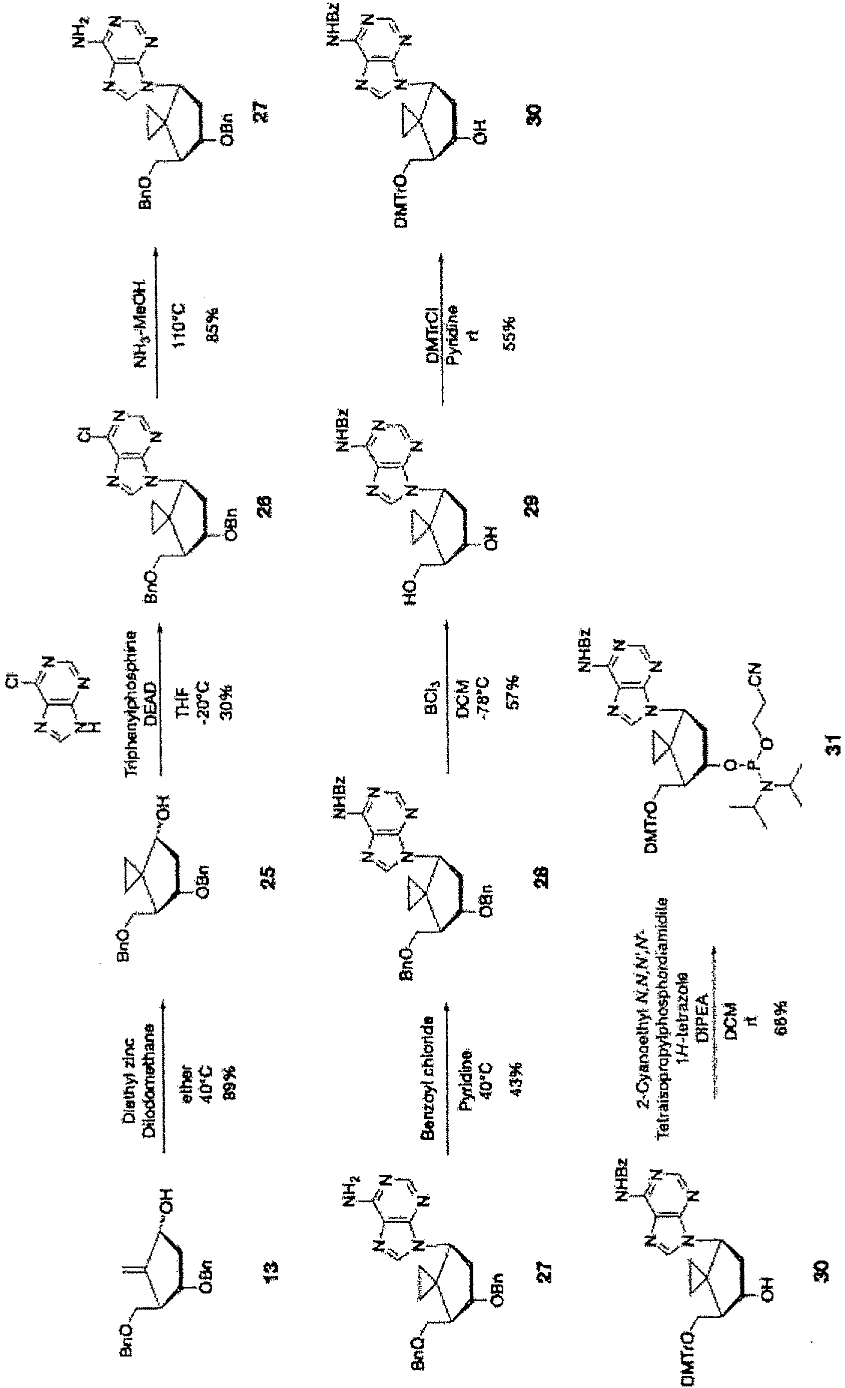 The following describes in detail each synthesis step in the above-mentioned synthesis scheme.

### [Example 43]

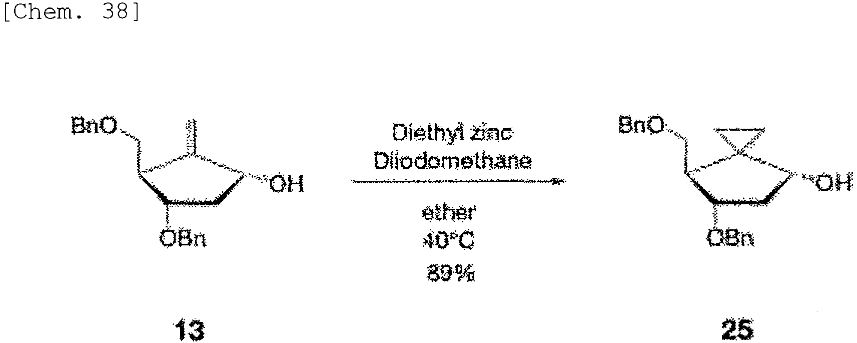

### N-(1-((1S,2S,3S,4S)-4-(benzyloxy)-3-((benzyloxy)methyl)-2-hydroxycyclopentyl)-5-methyl-2-oxo-1,2-dihydropyrimidin-4-yl)benzamide (25).

Under a nitrogen stream, to an anhydrous diethyl ether solution (98.4 mL) of compound 13 (3.189 g, 9.84 mmol) were added diethyl zinc (about 15% hexane solution, about 1 mol/L) (29.5 mL, 29.5 mmol, 3.0 eq.) and diiodomethane (4.75 mL, 59.0 mmol, 6.0 eq.) at 0°C under stirring. After stirring at 40°C for 6.5 hr, saturated sodium hydrogen carbonate (100 mL) was added to the reaction solution at 0°C under stirring, and the mixture was extracted 3 times with ethyl acetate (200 mL). The organic layer was washed with saturated brine, dehydrated with anhydrous sodium sulfate, and concentrated under reduced pressure to give a crude product. The product was purified by silica gel column chromatography (SiO₂, ethyl acetate/hexane=1:10 to 1:4) to give compound 25 (2.97 g, 89%) as a yellow oily substance. 1H NMR (400 MHz, CDCl3) δ 7.37 - 7.21 (m, 10H), 4.54 (d, J = 1.2 Hz, 2H), 4.49 - 4.37 (m, 2H), 4.05 - 4.00 (m, 1H), 3.50 (br, 1H), 3.26 (dd, J = 9.4, 6.0 Hz, 1H), 3.18 (dd, J = 9.4, 7.3 Hz, 1H), 2.48 (m, 2H), 2.12 (m, 1H), 2.05 - 1.95 (m, 2H), 0.93 (ddd, J = 10.2, 5.9, 4.5 Hz, 1H), 0.68 (ddd, J = 10.0, 5.8, 4.5 Hz, 1H), 0.52 (ddd, J = 9.4, 6.3, 4.5 Hz, 1H), 0.37 (ddd, J = 9.8, 6.2, 4.5 Hz, 1H).; 13C NMR (101 MHz, CDCl3) δ 128.46, 127.76, 127.67, 127.63, 127.58, 83.19, 80.40, 73.19, 70.79, 70.28, 48.06, 39.97, 30.10, 8.78, 7.53. HRMS (FAB) m/z: [M + H]+ calcd for C₂₂H₂₇O₃, 339.1955; found, 339.1978.

### [Example 44]

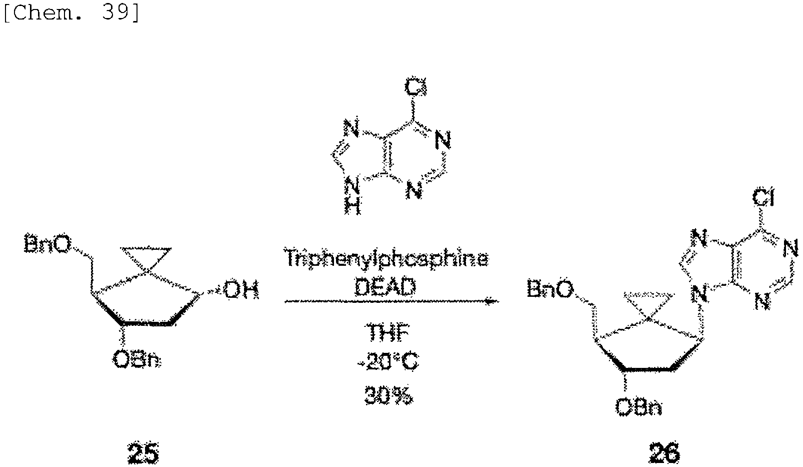

### 9-((4S,6S,7R)-6-(benzyloxy)-7-((benzyloxy)methyl)spiro[2.4]heptan-4-yl)-6-chloro-9H-purine (26).

Under a nitrogen stream, to an anhydrous tetrahydrofuran solution (8 mL) of triphenyl phosphine (939 mg, 3.58 mmol) were added 6-chloropurine (553 mg, 3.58 mmol, 2.5 eq.) and DEAD (1.64 mL, 3.58 mmol) at -20°C under stirring, and the mixture was stirred at the same temperature for 10 min. Compound 25 (485 mg, 1.43 mmol) diluted with anhydrous tetrahydrofuran solution (6.3 mL) was added, and the mixture was stirred at room temperature for 5 hr. To the reaction solution was added saturated aqueous sodium hydrogen carbonate solution (100 mL), and the mixture was extracted 3 times with ethyl acetate (100 mL). The organic layer was washed with saturated brine, dehydrated with anhydrous sodium sulfate, and concentrated under reduced pressure to give a crude product. The crude product was purified by silica gel column chromatography (SiO₂, ethyl acetate/hexane=1:10 to 3:5) to give compound 26 (203 mg, 30%) as a yellow oily substance. ¹H NMR (400 MHz, CDCl₃) δ 8.68 (s, 1H), 8.64 (s, 1H), 7.41 - 7.23 (m, 10H), 5.01 (t, J = 6.3 Hz, 1H), 4.58 - 4.44 (m, 4H), 4.29 (q, J = 5.8 Hz, 1H), 3.55 (dd, J = 9.8, 3.8 Hz, 1H), 3.35 (dd, J = 9.7, 4.1 Hz, 1H), 2.55 - 2.39 (m, 2H), 2.25 (q, J = 4.3 Hz, 1H), 0.93 - 0.85 (m, 1H), 0.85 - 0.70 (m, 2H), 0.06 (t, J = 4.8 Hz, 1H).; ¹³C NMR (101 MHz, CDCl₃) δ 151.97, 151.71, 150.88, 145.60, 138.23, 137.66, 128.67, 128.54, 128.16, 128.13, 127.86, 127.71, 80.39, 73.58, 71.64, 67.86, 61.12, 50.61, 38.99, 25.52, 17.32, 8.87. HRMS (FAB) m/z: [M + H]+ calcd for C₂₇H₂₈ClN₄O₂, 475.1895; found, 475.1901.

### [Example 45]

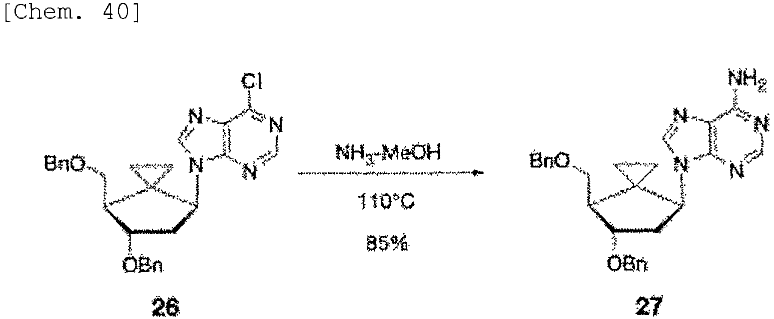

### 9-((4S,6S,7R)-6-(benzyloxy)-7-((benzyloxy)methyl)spiro[2.4]heptan-4-yl)-9H-purin-6-amine (27).

Under a nitrogen stream, to compound 26 (200 mg, 0.420 mmol) was added 7M ammonia-methanol solution (10 mL), and the mixture was stirred at 110°C for 18 hr in a pressure-resistant vial. The reaction solution was cooled to room temperature, and concentrated under reduced pressure to give a crude product. The product was purified by silica gel column chromatography (SiO₂, ethyl acetate/hexane=1:3 → dichloromethane/methanol=15:1) to give compound 27 (162 mg, 85%) as a white foamy solid. ¹H NMR (400 MHz, CDCl₃) δ 8.29 (s, 1H), 8.17 (s, 1H), 7.40 - 7.22 (m, 10H), 6.02 (s, 2H), 4.96 (dd, J = 7.2, 5.7 Hz, 1H), 4.58 - 4.42 (m, 4H), 4.28 (q, J = 5.8 Hz, 1H), 3.56 (dd, J = 9.7, 4.3 Hz, 1H), 3.28 (dd, J = 9.6, 4.4 Hz, 1H), 2.54 - 2.40 (m, 2H), 2.29 (q, J = 4.7 Hz, 1H), 0.87 (ddd, J = 9.4, 4.9, 2.9 Hz, 1H), 0.79 - 0.70 (m, 2H), 0.12 - 0.06 (m, 1H).; ¹³C NMR (101 MHz, CDCl₃) δ 155.60, 152.80, 150.24, 140.55, 138.41, 137.96, 128.62, 128.50, 128.01, 127.75, 127.70, 119.46, 80.33, 73.53, 71.49, 68.40, 60.05, 50.46, 38.75, 25.73, 16.37, 8.49. HRMS (FAB) m/z: [M + H]+ calcd for C₂₇H₃₀N₅O, 456.2394; found, 456.2400.

### [Example 46]

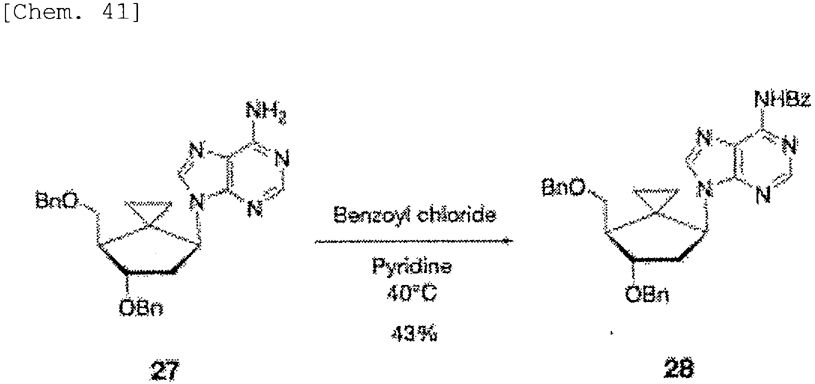

### N-(9-((4S,6S,7R)-6-(benzyloxy)-7-((benzyloxy)methyl)spiro[2.4]heptan-4-yl)-9H-purin-6-yl)benzamide (28).

Under a nitrogen stream, to an anhydrous N,N-dimethylformamide solution (3.0 mL) of compound 27 (135 mg, 0.296 mmol) and 4-dimethylaminopyridine (3.6 mg, 0.03 mmol) was added N,N-diisopropyl ethylamine (76 µL, 0.446 mmol). Successively, benzoic anhydride (134 mg, 0.594 mmol) was added, and the mixture was stirred at 100°C for 2 hr. Under icecooling, 2M ammonia-methanol solution (3 mL) was added, and the mixture was stirred for 10 min. To the reaction solution was added saturated aqueous sodium hydrogen carbonate solution (10 mL), and the mixture was extracted 3 times with ethyl acetate (10 mL). The organic layer was washed with saturated brine, dehydrated with anhydrous sodium sulfate, and concentrated under reduced pressure to give a crude product. The product was purified by silica gel column chromatography (SiO₂, ethyl acetate/hexane=1:1) to give compound 28 (71.5 mg, 43%) as a colorless oily substance. ¹H NMR (400 MHz, CDCl₃) δ 9.11 (br, 1H), 8.74 (s, 1H), 8.46 (s, 1H), 8.06 - 7.99 (m, 2H), 7.83 - 7.77 (m, 1H), 7.64 - 7.55 (m, 1H), 7.55 - 7.50 (m, 3H), 7.47 - 7.40 (m, 1H), 7.40 - 7.23 (m, 10H), 5.03 (dd, J = 7.2, 5.4 Hz, 1H), 4.58 - 4.46 (m, 4H), 4.31 (q, J = 5.9 Hz, 1H), 3.56 (dd, J = 9.7, 4.0 Hz, 1H), 3.38 (dd, J = 9.7, 4.0 Hz, 1H), 2.57 - 2.39 (m, 2H), 2.28 (q, J = 4.5 Hz, 1H), 0.90 (ddd, J = 9.4, 4.7, 2.7 Hz, 1H), 0.83 - 0.69 (m, 2H), 0.13 - 0.04 (m, 1H).; ¹³C NMR (101 MHz, CDCl₃) δ 164.78, 152.36, 152.28, 149.35, 143.25, 138.31, 137.84, 133.82, 132.81, 132.12, 128.93, 128.72, 128.63, 128.52, 128.08, 128.02, 127.98, 127.81, 127.72, 127.43, 122.92, 80.34, 73.54, 71.59, 68.06, 60.61, 50.56, 38.86, 25.57, 16.97, 8.77. HRMS (FAB) m/z: [M + H]+ calcd for C₃₄H₃₄N₅O₃, 560.2656; found, 560.2662.

### [Example 47]

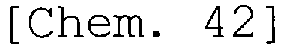

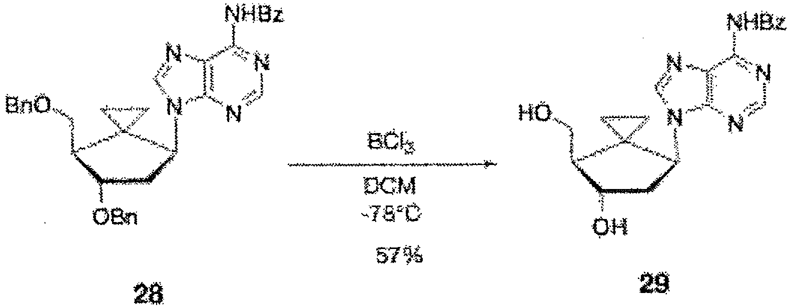

### N-(9-((4S,6S,7R)-6-hydroxy-7-(hydroxymethyl)spiro[2.4]heptan-4-yl)-9H-purin-6-yl)benzamide (29).

Under a nitrogen stream, to an anhydrous dichloromethane solution (6.3 mL) of compound 28 (351 mg, 0.627 mmol) was added dropwise 1M boron trichloride (6.3 mL, 6.3 mmol) at -78°C under stirring. After stirring, to the reaction solution was added methanol (10 mL), and the mixture was stirred for 10 min. The mixture was concentrated under reduced pressure and purified by silica gel column chromatography (SiO₂, dichloromethane/methanol=20:1 to 10:1) to give compound 29 (136 mg, 57%) as a white foamy solid. ¹H NMR (400 MHz, CD₃OD) δ 8.88 (s, 1H), 8.67 (s, 1H), 8.06 (d, J = 7.5 Hz, 2H), 7.63 (t, J = 7.4 Hz, 1H), 7.54 (t, J = 7.4 Hz, 2H), 5.07 (t, J = 6.1 Hz, 1H), 4.50 (p, J = 6.1 Hz, 1H), 3.75 (dd, J = 11.3, 4.2 Hz, 1H), 3.61 (dd, J = 11.2, 4.6 Hz, 1H), 2.54 (dt, J = 11.1, 5.1 Hz, 1H), 2.37 (dt, J = 13.8, 6.9 Hz, 1H), 1.98 (p, J = 4.6 Hz, 1H), 0.92 (t, J = 6.8 Hz, 2H), 0.89 - 0.73 (m, 1H), 0.03 (t, J = 6.4 Hz, 1H).; ¹³C NMR (101 MHz, CD₃OD) δ 132.72, 128.46, 128.17, 72.53, 61.21, 59.70, 54.22, 48.32, 48.11, 47.89, 47.68, 47.47, 47.25, 47.04, 40.56, 25.38, 16.15, 7.73. HRMS (FAB) m/z: [M + H]⁺calcd for C₂₀H₂₂N₅O₃, 380.1717; found, 380.1723.

### [Example 48]

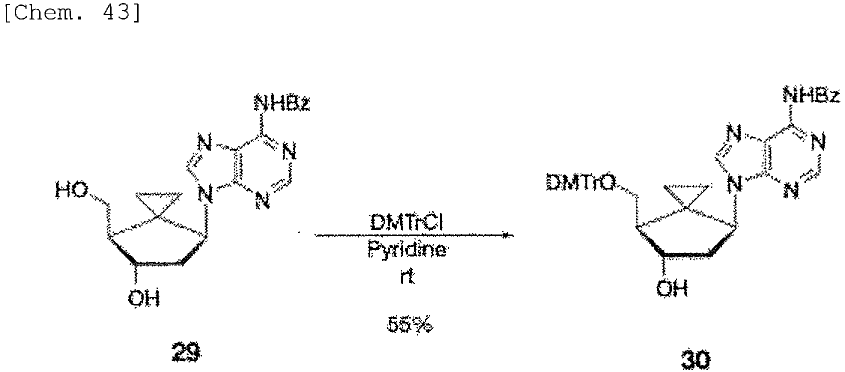

### N-(9-((4S,6S,7R)-7-((bis(4-methoxyphenyl)(phenyl)methoxy)methyl)-6-hydroxyspiro[2.4]heptan-4-yl)-9H-purin-6-yl)benzamide (30).

To compound 29 (32.2 mg, 0.0849 mmol) was added an anhydrous pyridine solution (1.2 mL) under a nitrogen atmosphere, then 4,4'-dimethoxytrityl chloride (40.7 mg, 0.127 mmol) was added, and the mixture was stirred at room temperature for 4 hr and half. To the reaction solution was added saturated aqueous sodium hydrogen carbonate solution (40 mL), and the mixture was extracted three times with ethyl acetate (40 mL). The organic layer was washed with saturated brine, dehydrated with anhydrous sodium sulfate, and concentrated under reduced pressure to give a crude product. The crude product was purified by silica gel column chromatography (SiO₂, ethyl acetate/hexane=2:1 to dichloromethane/methanol=20:1) to give compound 30 (30 mg, 55%)as a yellow foamy solid. ¹H NMR (400 MHz, CD₃OD) δ 8.55 (s, 1H), 8.27 (s, 1H), 8.09 - 8.05 (m, 2H), 7.67 - 7.58 (m, 1H), 7.58 - 7.53 (m, 2H), 7.49 - 7.43 (m, 2H), 7.37 - 7.26 (m, 6H), 7.23 - 7.11 (m, 1H), 6.88 - 6.79 (m, 4H), 5.08 - 5.04 (m, 1H), 4.57 (q, J = 5.7 Hz, 1H), 3.74 (s, 6H), 3.24 (d, J = 6.0 Hz, 2H), 2.53 (dt, J = 13.5, 5.8 Hz, 1H), 2.34 (ddd, J = 13.7, 8.0, 6.1 Hz, 1H), 2.23 (q, J = 5.9 Hz, 1H), 0.83 - 0.73 (m, 2H), 0.54 (dt, J = 9.7, 6.0 Hz, 1H), -0.31 (dt, J = 9.9, 5.9 Hz, 1H).; ¹³C NMR (101 MHz, CD₃OD) δ 166.79, 158.78, 152.22, 151.53, 149.59, 145.08, 143.79, 136.01, 135.92, 133.68, 132.56, 129.98, 129.94, 128.42, 128.08, 128.00, 127.49, 126.49, 123.42, 112.77, 86.47, 72.85, 62.42, 60.67, 54.38, 52.64, 40.01, 26.49, 14.78, 6.86. HRMS (FAB) m/z: [M+ H]⁺ calcd for C₄₁H₄₀N₅O₅, 682.3024; found, 682.3028.

### [Example 49]

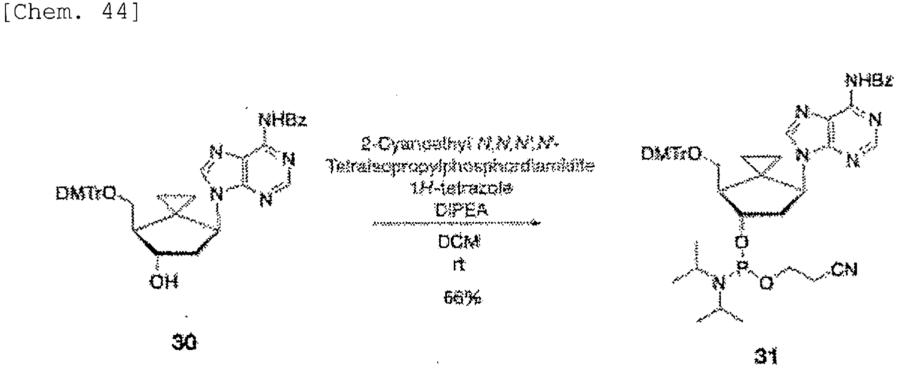

### (4R,5S,7S)-7-(6-benzamide-9H-purin-9-yl)-4-((bis(4-methoxyphenyl) (phenyl)methoxy)methyl)spiro[2.4]heptan-5-yl(2-cyanoethyl)diisopropylphosphoramidite (31)

Compound 30 (53.0 mg, 0.078 mmol) and 1H-tetrazole (8.2 mg, 0.012 mmol) were added to a heated and dried reaction vessel, and anhydrous dichloromethane solution (0.78 mL) and DIPEA (20 µL, 0.012 mmol) were added under a nitrogen atmosphere. 2-Cyanoethyl N,N,N',N'-tetraisopropylphosphordiamidite (37 µL, 0.012 mmol) was added, and the mixture was stirred at room temperature for 1 hr. After confirming the consumption of the starting material by TLC, saturated aqueous sodium hydrogen carbonate solution (20 mL) was added, and the mixture was extracted 3 times with dichloromethane (20 mL). The organic layer was washed with saturated brine, dehydrated with anhydrous sodium sulfate, and concentrated under reduced pressure to give a crude product. The crude product was purified by silica gel column chromatography (SiO2, ethyl acetate/hexane=1:1 → 4/1, 1% triethylamine added) to give compound 31 (45 mg, 66%) as a white foamy solid. ¹H NMR (400 MHz, CDCl₃) δ 9.20 (br, 1H), 8.68 - 8.67 (m, 1H), 8.04 - 7.97 (m, 2H), 7.88 - 7.83 (m, 1H), 7.62 - 7.58 (m, 1H), 7.53 - 7.49 (m, 2H), 7.45 - 7.41 (m, 2H), 7.36 - 7.27 (m, 6H), 7.24 - 7.20 (m, 1H), 6.82 (ddd, J = 8.9, 4.3, 1.4 Hz, 4H), 5.06 (dt, J = 8.9, 7.4 Hz, 1H), 4.64 - 4.51 (m, 1H), 3.87 - 3.68 (m, 8H), 3.67 - 3.56 (m, 2H), 3.53 - 3.10 (m, 2H), 2.66 - 2.39 (m, 5H), 1.23 - 1.14 (m, 12H), 0.92 - 0.74 (m, 2H), 0.60 - 0.50 (m, 1H), -0.25 -0.36 (m, 1H).; ³¹P NMR (162 MHz, CDCl₃) δ 148.15, 147.89. HRMS(FAB) m/z: [M + H]⁺ calcd for C₅₀H₅₇N₇O₆P, 882.4102; found, 882.4109.

### [Production of nucleoside (A)]

Synthesis of phosphoramidites via an alternative route for Entecavir derivative (5-methyl Cytosine), introduction into oligonucleotides, and functional evaluation thereof are described in detail in the following.

First, the synthesis scheme for the phosphoramidites is shown below.

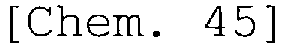

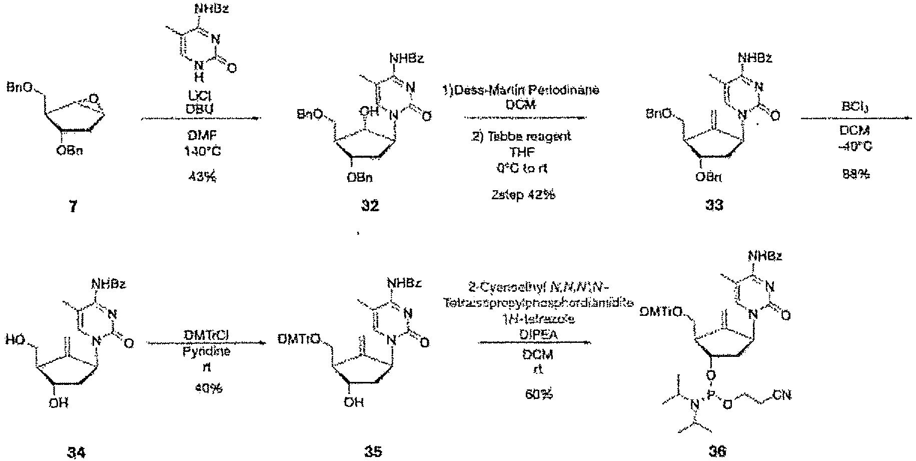

### [Example 50]

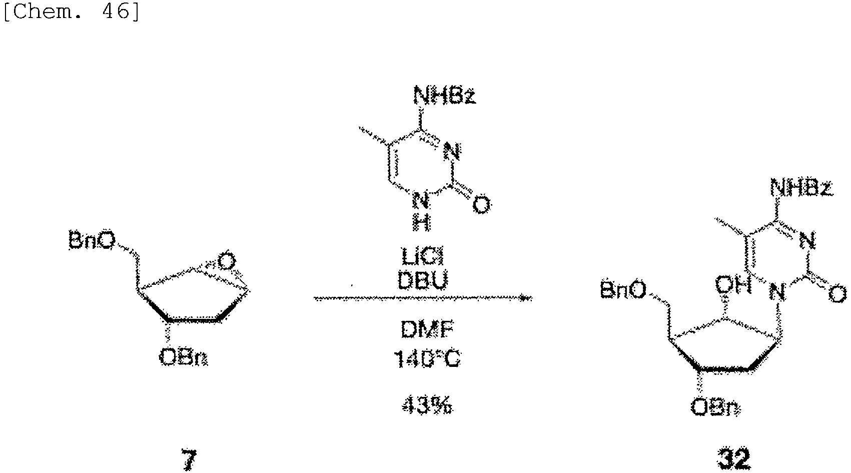

### N-(1-((1S,2S,3S,4S)-4-(benzyloxy)-3-((benzyloxy)methyl)-2-hydroxycyclopentyl)-5-methyl-2-oxo-1,2-dihydropyrimidin-4-yl)benzamide (32).

To compound 7 (56.0 mg, 0.181 mmol) was added anhydrous N,N-dimethylformamide solution (1.8 mL) under a nitrogen atmosphere, and lithium chloride (15.3 mg, 0.362 mmol) and DBU (54.0 µL, 0.362 mmol) were added. Successively, N-Benzoyl-5-Methylcytosine (83 mg, 0.362 mmol) was added, and the mixture was stirred at 140°C for 7 hr. Saturated aqueous sodium hydrogen carbonate solution (20 mL) was added, and the mixture was extracted 3 times with ethyl acetate (20 mL). The organic layer was washed with saturated brine, dehydrated with anhydrous sodium sulfate, and concentrated under reduced pressure to give a crude product. The crude product was purified by silica gel column chromatography (SiO₂, ethyl acetate/hexane=1:2) to give compound 32 (42.4 mg, 43%) as a colorless oily substance. ¹H NMR (400 MHz, CDCl₃) δ 8.30 (dd, J = 8.2, 1.5 Hz, 2H), 7.54 - 7.48 (m, 1H), 7.46 - 7.40 (m, 2H), 7.31 (qd, J = 8.9, 4.4 Hz, 16H), 7.25 (d, J = 0.9 Hz, 4H), 7.17 (d, J = 1.4 Hz, 1H), 4.61 - 4.46 (m, 7H), 4.42 (d, J = 11.8 Hz, 1H), 4.32 (t, J = 8.4 Hz, 1H), 4.18 (s, 1H), 3.93 - 3.87 (m, 1H), 3.78 (dd, J = 9.0, 4.7 Hz, 1H), 3.61 (dd, J = 9.0, 7.4 Hz, 1H), 2.34 - 2.21 (m, 3H), 2.05 (q, J = 1.0 Hz, 3H), 1.28 - 1.22 (m, 2H).; ¹³C NMR (126 MHz, CDCl₃) δ 159.94, 149.01, 140.37, 138.03, 137.89, 137.28, 132.51, 129.97, 128.66, 128.58, 128.51, 128.23, 128.03, 127.92, 127.82, 111.86, 77.33, 76.64, 76.36, 73.67, 71.41, 70.85, 65.70, 51.93, 33.52, 13.58. HRMS(FAB) m/z: [M + H]⁺calcd for C₃₂H₃₄N₃O₅, 540.2493; found, 540.2498.

### [Example 51]

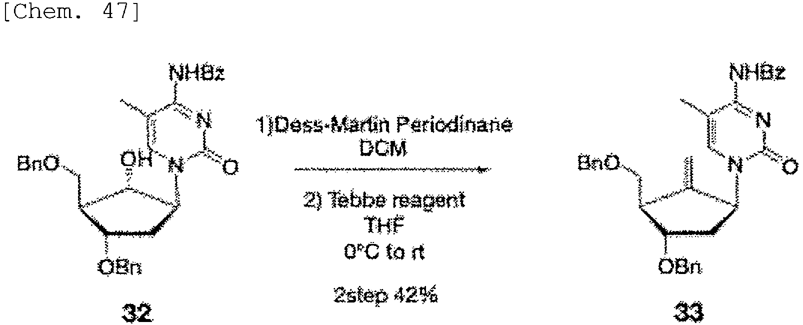

### N-(1-((1S,3R,4S)-4-(benzyloxy)-3-((benzyloxy)methyl)-2-methylenecyclopentyl)-5-methyl-2-oxo-1,2-dihydropyrimidin-4-yl)benzamide (33).

To compound 32 (56.0 mg, 0.104 mmol) was added anhydrous dichloromethane solution (1.0 mL) under a nitrogen atmosphere, then Dess-Martin periodinane (57.3 mg, 0.135 mmol) was added at 0°C under stirring, and the mixture was stirred at room temperature for 3 hr. Sodium thiosulfate aqueous solution (15 mL) and saturated sodium hydrogen carbonate (5 mL) were added at 0°C under stirring, and the mixture was stirred at the same temperature for 20 min. The reaction solution was extracted 3 times with dichloromethane (20 mL). The organic layer was washed with saturated brine, dehydrated with anhydrous sodium sulfate, and concentrated under reduced pressure. The residue was dissolved in anhydrous tetrahydrofuran solution (1.0 mL) under a nitrogen atmosphere, 0.5 M Tebbe reagent (0.18 mL, 0.091 mmol) was added at 0°C under stirring, and the mixture was stirred at room temperature for 1 hr and half. After quenching by adding 0.1 M NaOH aq (20 mL) and stirring for 30 min at 0°C, H₂O (20 mL) was added to the reaction solution, and the mixture was extracted 3 times with ethyl acetate (20 mL). The organic layer was washed with saturated brine, dehydrated with anhydrous sodium sulfate, and concentrated under reduced pressure to give a crude product. The product was purified by silica gel column chromatography (SiO₂, ethyl acetate/hexane=1:12 to 1:3) to give compound 33 (23.4 mg, 2 step 42%) as a yellow oily substance. ¹H NMR (500 MHz, CDCl₃) δ 8.36 - 8.25 (m, 2H), 7.54 - 7.48 (m, 1H), 7.46 - 7.40 (m, 3H), 7.39 - 7.27 (m, 15H), 5.76 (dd, J = 10.4, 7.9 Hz, 1H), 5.30 (t, J = 2.4 Hz, 1H), 5.02 (t, J = 2.5 Hz, 1H), 4.70 (s, 1H), 4.60 - 4.46 (m, 5H), 4.13 (tt, J = 5.5, 2.8 Hz, 1H), 3.82 - 3.72 (m, 2H), 2.93 (s, 1H), 2.40 (ddt, J = 13.2, 8.0, 1.9 Hz, 1H), 2.11 - 2.05 (m, 1H), 1.73 (d, J = 1.1 Hz, 3H).; ¹³C NMR (126 MHz, CDCl₃) δ 179.49, 159.81, 149.18, 148.99, 139.85, 138.06, 137.75, 137.31, 132.30, 129.83, 128.59, 128.57, 128.44, 128.10, 128.07, 127.96, 127.68, 127.60, 127.56, 127.53, 126.99, 111.94, 111.60, 80.22, 77.22, 73.64, 72.90, 70.70, 65.41, 58.11, 49.58, 36.77, 13.15. HRMS(FAB) m/z: [M + H]⁺ calcd for C₃₃H₃₄N₃O₄, 536.2544; found, 536.2550.

### [Example 52]

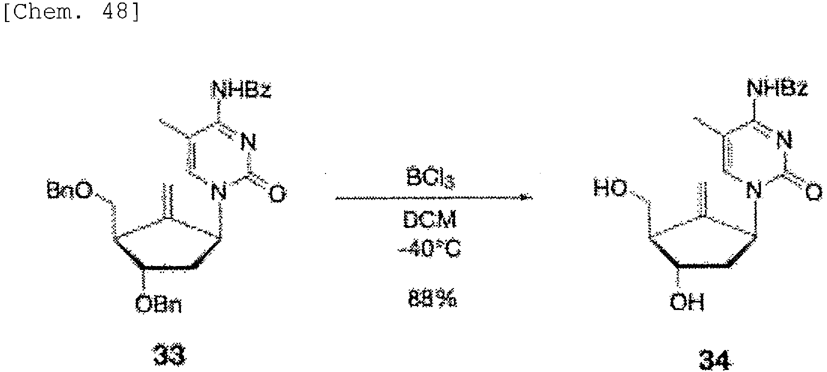

### N-(1-((1S,3R,4S)-4-hydroxy-3-(hydroxymethyl)-2-methylenecyclopentyl)-5-methyl-2-oxo-1,2-dihydropyrimidin-4-yl)benzamide (34).

Under a nitrogen stream, to an anhydrous dichloromethane solution (1.2 mL) of compound 33 (65.9 mg, 0.123 mmol) was added dropwise 1M boron trichloride (1.23 mL, 1.23 mL) at -40°C under stirring. After stirring, to the reaction solution was added methanol (2 mL), and the mixture was stirred for 1 hr. The mixture was concentrated under reduced pressure and purified by silica gel column chromatography (SiO₂, dichloromethane/methanol=20:1 to 10:1) to give compound 34 (38.5 mg, 88%) as a white foamy solid. ¹H NMR (400 MHz, CD₃OD) δ 8.26 (d, J = 7.6 Hz, 2H), 7.64 (s, 1H), 7.52 (s, 1H), 7.44 (d, J = 7.7 Hz, 2H), 5.67 (s, 1H), 5.31 (t, J = 2.5 Hz, 1H), 4.99 (s, 1H), 4.33 (d, J = 3.4 Hz, 1H), 3.78 (q, J = 6.7 Hz, 2H), 2.61 (s, 1H), 2.15 (d, J = 10.5 Hz, 2H), 2.06 (s, 3H). ¹³C NMR (101 MHz, CD₃OD) δ 149.56, 132.30, 130.12, 129.36, 127.93, 112.83, 111.64, 110.38, 71.90, 63.27, 58.90, 54.44, 53.82, 47.49, 47.27, 47.06, 46.42, 38.45, 12.29, 8.02. HRMS (FAB) m/z: [M + H]⁺ calcd for C₁₉H₂₂N₃O₄, 356.1605; found, 356.1610.

### [Example 5 3]

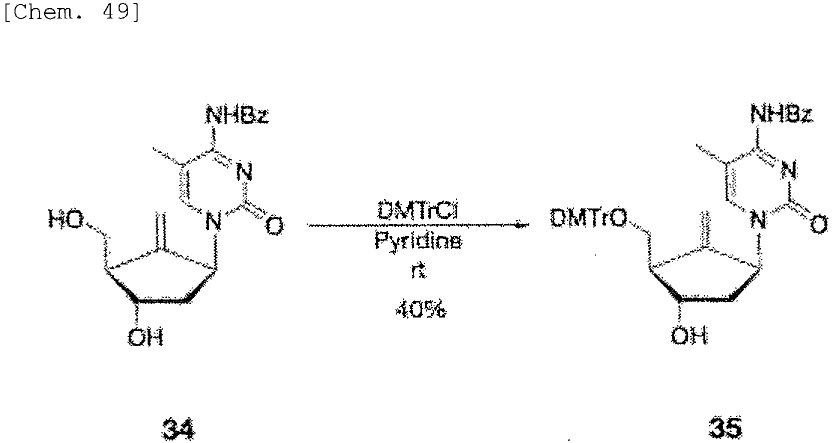

### N-(1-((1S,3R,4S)-3-((bis(4-methoxyphenyl) (phenyl)methoxy)methyl)-4-hydroxy-2-methylenecyclopentyl)-5-methyl-2-oxo-1,2-dihydropyrimidin-4-yl)benzamide (35).

To compound 34 (38.5 mg, 0.108 mmol) was added anhydrous pyridine solution (1.1 mL) under a nitrogen atmosphere, then 4,4'-dimethoxytrityl chloride (54.9 mg, 0.162 mmol) was added, and the mixture was stirred at room temperature for 1 hr. To the reaction solution was added saturated aqueous sodium hydrogen carbonate solution (10 mL), and the mixture was extracted three times with ethyl acetate (10 mL). The organic layer was washed with saturated brine, dehydrated with anhydrous sodium sulfate, and concentrated under reduced pressure to give a crude product. The crude product was purified by silica gel column chromatography (SiO₂, ethyl acetate/hexane=2:1 to dichloromethane/methanol=20:1) to give compound 35 (28.7 mg, 40%) as a yellow foamy solid. ¹H NMR (400 MHz, CDCl₃) δ 8.32 - 8.26 (m, 2H), 7.54 - 7.47 (m, 1H), 7.46 - 7.37 (m, 5H), 7.33 - 7.26 (m, 7H), 7.11 (d, J = 1.2 Hz, 1H), 6.84 (dd, J = 8.9, 1.4 Hz, 4H), 5.77 (t, J = 9.1 Hz, 1H), 4.97 (t, J = 2.7 Hz, 1H), 4.88 (t, J = 2.6 Hz, 1H), 4.44 (q, J = 4.0 Hz, 1H), 3.79 (s, 7H), 3.61 (dd, J = 9.4, 4.5 Hz, 2H), 3.32 - 3.21 (m, 3H), 2.70 (s, 1H), 2.29 (ddd, J = 12.8, 8.9, 3.6 Hz, 2H), 2.17 (ddd, J = 13.9, 9.1, 5.7 Hz, 1H), 1.96 (s, 3H).; ¹³C NMR (101 MHz, CDCl₃) δ 159.82, 158.77, 149.13, 148.89, 144.52, 139.44, 137.30, 135.74, 135.45, 132.49, 132.14, 130.22, 129.95, 129.23, 128.74, 128.26, 128.20, 128.10, 127.46, 127.20, 113.34, 112.40, 111.50, 87.16, 77.33, 73.94, 65.25, 57.54, 55.37, 52.98, 51.69, 39.68, 34.59, 14.93, 13.15, 8.07. HRMS (FAB) m/z: [M+ H]⁺calcd for C₄₀H₄₀N₃O₆, 658.2912; found, 658.2916.

### [Example 54]

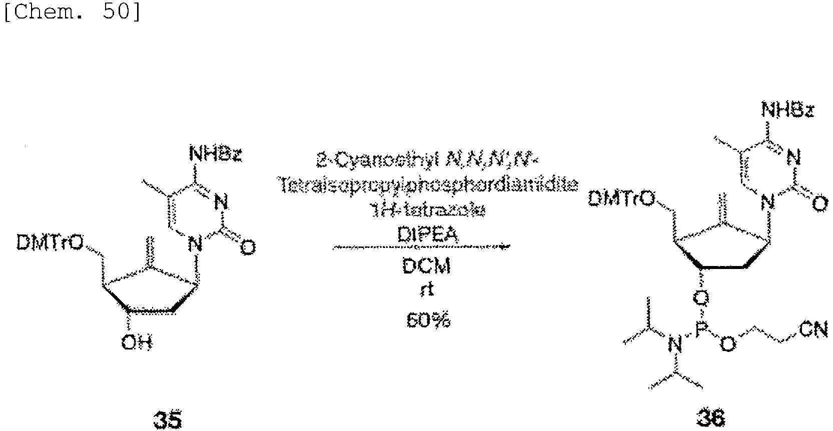

### (1S,2R,4S)-4-(4-benzamide-5-methyl-2-oxopyrimidin-1(2H)-yl)-2-((bis(4-methoxyphenyl) (phenyl)methoxy)methyl)-3-methylenecyclopentyl(2-cyanoethyl)diisopropylphosphoramidite (36).

Compound 35 (84.0 mg, 0.128 mmol) and 1H-tetrazole (13.5 mg, 0.192 mmol) were added to a heated and dried reaction vessel, and anhydrous dichloromethane solution (1.28 mL) and DIPEA (32.6 µL, 0.192 mmol) were added under a nitrogen atmosphere. 2-Cyanoethyl N,N,N',N'-tetraisopropylphosphordiamidite (60.9 µL, 0.192 mmol) was added, and the mixture was stirred at room temperature for 1 hr and half. After confirming the consumption of the starting material by TLC, saturated aqueous sodium hydrogen carbonate solution (10 mL) was added, and the mixture was extracted twice with dichloromethane (10 mL). The organic layer was washed with saturated brine, dehydrated with anhydrous sodium sulfate, and concentrated under reduced pressure to give a crude product. The crude product was purified by silica gel column chromatography (SiO₂, ethyl acetate/hexane=2:5, 1% triethylamine added) to give compound 36 (66.3 mg, 60%) as a white foamy solid. ¹H NMR (400 MHz, CDCl₃) δ 8.28 (dq, J = 7.2, 1.4 Hz, 2H), 7.58 - 7.46 (m, 1H), 7.46 - 7.34 (m, 5H), 7.25 (s, 14H), 6.87 - 6.79 (m, 5H), 5.79 (t, J = 9.5 Hz, 1H), 5.05 - 4.85 (m, 2H), 4.56 (dd, J = 10.0, 4.6 Hz, 1H), 3.91 - 3.65 (m, 12H), 3.65 - 3.41 (m, 4H), 3.24 (ddd, J = 23.8, 9.3, 4.8 Hz, 1H), 2.86 (d, J = 23.5 Hz, 1H), 2.64 (q, J = 6.9 Hz, 2H), 2.51 (t, J = 6.4 Hz, 1H), 2.48 - 2.30 (m, 1H), 2.24 - 2.11 (m, 1H), 1.61 (d, J = 1.1 Hz, 3H), 1.26 - 1.09 (m, 18H).; ³¹P NMR (162 MHz, CDCl₃) δ 148.02, 147.75. HRMS(FAB) m/z: [M + H]⁺ calcd for C₄₉H₅₇N₅O₇P, 858.3990; found, 858.3996.

### [Example 55]

### Purpose: Measurement of melting temperature (Tₘ) of double-strand formed with complementary strand RNA

### Method and Results

### (1) Synthesis of oligonucleotide

In the same manner as in the method described in Example 2, various oligonucleotides with mPCS2 as the parent sequence (nucleotide linkage: all phosphorothioate linked) described in the following Table 12 were synthesized and subjected to the following evaluation. In the Table, E^{A} represents Entecavir and E^{A3} represents a 4'-spiro form. E^{A3} is a compound synthesized in the above-mentioned Examples 43 - 49.

**[Table 12]**

| Abbreviation | ID | | 1 | 2 | 3 | 4 | 5 | 6 | 7 | 8 | 9 | 10 | 11 | 12 | 13 | |
|---|---|---|---|---|---|---|---|---|---|---|---|---|---|---|---|---|
| mPCS2 | mPCSK9-1418-BNA(13) | 5' | C | T | g | t | g | a | t | g | a | c | C | T | c | 3' |
| mPCS2-gapA1 | mPCSK9-1418-BNA(13)-gapA1 | | C | T | g | t | g | E^{A} | t | g | a | c | C | T | c | |
| mPCS2-gapA2 | mPCSK9-1418-BNA(13)-gapA2 | | C | T | g | t | g | a | t | g | E^{A} | c | C | T | c | |
| mPCS2-gapA3 | mPCSK9-1418-BNA(13)-gapA3 | | C | T | g | t | g | E^{A3} | t | g | a | c | C | T | c | |
| | mPCS2-cRNA | 3' | G | A | C | A | C | U | A | C | U | G | G | A | G | 5' |

### (2) Measurement of melting temperature (Tₘ) of double-strand formed with complementary strand RNA

A sample solution (150 µL) with final concentrations of 10 mM phosphate buffer (pH 7.0), 100 mM sodium chloride, 0.1 mM ethylenediaminetetraacetic acid, and 4 µM of oligonucleotide described in Table 12 and complementary RNA was heated to 95°C for 3 min, then annealed by slow cooling to 20°C at a rate of 1°C per min, and the measurement was started. The temperature was increased to 95°C at a rate of 0.5°C per min, and the absorbance at 260 nm was plotted at 1°C intervals. All Tₘ values were calculated using the midline method.

The measurement results are shown in the following Table 13.

**[Table 13]**

| Abbreviation | ID | | 1 | 2 | 3 | 4 | 5 | 6 | 7 | 8 | 9 | 10 | 11 | 12 | 13 | | *Tₘ* | Δ*T*ₘ |
|---|---|---|---|---|---|---|---|---|---|---|---|---|---|---|---|---|---|---|
| mPCS2 | mPCSK9-1418-BNA(13) | 5' | C | T | g | t | g | a | t | g | a | c | c | T | c | 3' | 60.3 | |
| mPCS2-gapA1 | mPCSK9-1418-BNA(13)-gapA1 | | C | T | g | t | g | E^{A} | t | g | a | c | C | T | c | | 60.1 | -0.17 |
| mPCS2-gapA2 | mPCSK9-1418-BNA(13)-gapA2 | | C | T | g | t | g | a | t | g | E^{A} | c | C | T | c | | 61.4 | +1.15 |
| mPCS2-gapA3 | mPCSK9-1418-SNA(13)-gapA3 | | C | T | g | t | g | E^{A3} | t | g | a | c | C | T | c | | 59.4 | -0.84 |
| | mPCS2-cRNA | 3' | G | A | C | A | C | U | A | C | U | G | G | A | G | 5' | | |

### Discussion:

From the above, oligonucleotides incorporating the nucleoside analog shown in the present invention could be synthesized according to conventional methods. When the analogs were introduced, there were some variations in thermodynamic stability with the complementary strand depending on the analog, but the binding affinity was not greatly impaired. Previous studies have shown that unsubstituted carbocyclic nucleotide (Org. Lett. 2019, 21, 7, 1963-1967) exhibit significantly impaired binding force to complementary strand RNA. Therefore, it is considered that suppressing conformational fluctuations of the cyclopentane ring due to sp2 carbons and spiro carbons led to the maintenance of high binding force. In addition, it is suggested that thermodynamic stability can be finely adjusted by the type of substituents extending outside the ring.

### [Example 56]

### Purpose: Evaluation of stability of oligonucleotide with Entecavir analog introduced into strand against 3'-exonuclease Method and Results:

### Synthesis of oligonucleotide

In the same manner as in the method described in Example 2, various oligonucleotides (nucleotide linkage: all phosphodiester linked) described in the following Table 14 were synthesized and subjected to the following evaluation. In the Table, mC represents 2'-deoxy-5-methylcytidine, and E^{mC} represents 5-methylcytidine derivative of carbocyclic DNA. Evaluation of stability of oligonucleotide against 3'-exonuclease

A buffer solution [10 mM MgCl₂, 50 mM Tris-HCl (pH 8.0)] containing antisense nucleic acid (2.7 nmol) with Entecavir analog (E^{mC}) introduced into the 3' end (Table 14) was mixed with 0.003 units of Crotalus admanteus venom phosphodiesterase (CAVP) (Worthington Biochemical) and incubated at 37°C. The reaction solution was collected at 5, 15, 30, 45, and 60 min, heated at 90°C for 2 min, and the time course changes of full-length oligonucleotides were analyzed by reversed-phase HPLC. The HPLC measurement conditions are shown below.

### (Eluent)

Solution A: 100 mM hexafluoro-2-propanol 8.6 mM triethylamine (pH 8.36)
Solution B: methanol

### (Gradient)

Solution B concentration: 0-30% (10 min)

### (Column)

1) YMC Accura Triart Bio C18, 5.0 µm (4.6 A x 50 mm)
2) column temperature 60°C

### (Flow rate)

1.0 mL/min

### (Detection)

UV (260 nm)

### (Evaluated oligonucleotide)

The sequence of the evaluated oligonucleotide is shown in the following Table 14.

**[Table 14]**

| ID | | 1 | 2 | 3 | 4 | 5 | 6 | |
|---|---|---|---|---|---|---|---|---|
| 3'-dmC | 5' | t | t | t | t | t | mC | 3' |
| 3'-eneNA(mC) | 5' | t | t | t | t | t | E^{mC} | 3' |

In the Table, mC represents 5-methylcytosine DNA, and E^{mC} represents an Entecavir analog with 5-methylcytosine as the base.

### (2) The area ratio (%) to unreacted oligonucleotides is shown in a table and a graph (Table 15, Fig. 26).

**[Table 15]**

| | Area ratio of remaining full length | |
|---|---|---|
| Time (min) | 3'-dmC | 3'-eneNA(mC) |
| 0 | 100 | 100 |
| 5 | 0 | 85 |
| 15 | 0 | 74 |
| 30 | 0 | 61 |
| 45 | 0 | 52 |
| 60 | 0 | 43 |

### Discussion:

From the Figure, the parent strand 3'-dmC was completely degraded within 5 min of nuclease treatment, whereas 40% or more of 3'-eneNA(mC), into which nucleoside (E^{mC}) was introduced, remained unreacted even after 60 min of nuclease treatment, demonstrating high enzyme resistance. Oligonucleotides with this DNA analog introduced thereinto can impart metabolic stability to nucleic acids through the effect of substituents that protrude outside the ring.

### [Example 57]

### Purpose: Evaluation of in vitro effect of oligonucleotide drug incorporating carbocyclic nucleoside with spiro ring structure in the strand on inhibiting target gene expression

### Method and Results:

ASO described in Table 16 was diluted to a final concentration of 1 µM in a cell culture medium (9 mM CaCl₂ added) and added to a 96-well plate. A well without ASO was prepared as a negative control. Huh-7 cells diluted in the above-mentioned medium were seeded by 10,000 cells into each well and cultured in an incubator for 24 hr. Then, cDNA was prepared according to the manufacturer's protocol from the cell lysate by using SuperPrep (registered trademark) II Cell Lysis & RT Kit for qPCR (TOYOBO). The expression level of human PCSK9 mRNA was analyzed using a QuantStudio(R) 3 real-time PCR system (Thermo Fisher Scientific). For the analysis of human Pcsk9, TaqMan Gene Expression Assay (FAM) Assay ID:Hs00545399_m1 (Thermo Fisher Scientific) was used. For the analysis of human Gapdh as a housekeeping gene, TaqMan Gene Expression Assay (VIC primer-limited)Assay ID:Hs02758991_g1 (Thermo Fisher Scientific) was used. KD activity was calculated based on relative expression levels, by converting the difference in Ct values to the difference in expression levels. Significance was tested using Tukey's multiple comparison test after one-way analysis of variance (ANOVA). **p < 0.01, ***p < 0.001. The evaluation results are shown in Fig. 27.

**[Table 16]**

| Abbreviation | ID | | 1 | 2 | 3 | 4 | 5 | 6 | 7 | 8 | 9 | 10 | 11 | 12 | 13 | |
|---|---|---|---|---|---|---|---|---|---|---|---|---|---|---|---|---|
| mPCS2 | mPCSK9-1418-BNA(13) | 5' | C | T | g | t | g | a | t | g | a | c | C | T | c | 3' |
| mPCS2-gapA3 | mPCSK9-1418-BNA(13)-gapA3 | 5' | C | T | g | t | g | E^{A3} | t | g | a | c | C | T | c | 3' |

Nucleotide linkage: all phosphorothioate linked

In the Table, E^{A3} represents the 4'-spiro form of Entecavir.

### Discussion:

From the Figure, gapA3 significantly suppressed the expression of the target gene. Therefrom it was shown that nucleic acid drugs in which a portion of monomers in the nucleic acid drug strand is replaced with a carbocyclic nucleoside having a spiro ring structure, or in which a carbocyclic nucleoside having a spiro ring structure is present in the strand, retain the activity.

### [Example 58]

### Purpose: Evaluation of cytotoxicity (cell survival rate) of oligonucleotide drug with carbocyclic nucleoside with spiro ring structure introduced into strand

### Method and Results:

ASOs listed in Table 16 in Example 57 were diluted with cell culture medium (with 9 mM CaCl₂ added) to a final concentration of 0.001 µM to 3 µM and added to a 96-well plate. A well without ASO was prepared as a negative control. HuH-7 cells diluted with the aforementioned medium were seeded by 10,000 cells into each well and cultured in an incubator for 72 hr. Then, the medium was replaced with 110 µL of medium containing 10 µL (/well) of Cell Counting Kit-8 (Dojin Chemical Co., Ltd.) and the cells were incubated in the incubator for 2 hr. Absorbance was measured at 450 nm and 650 nm as a reference using a microplate reader. Cell survival rate was calculated by subtracting the 650 nm absorbance value from each 450 nm absorbance value, and then subtracting the background value from the control value or assay value. The IC50 value for each ASO was calculated using a four-parameter logistic model, based on the obtained cell survival rate.

The evaluation results are shown in Fig. 28.

### Discussion:

From the Figure, a significant reduction in cytotoxicity was observed in gapA3 compared to the parent strand mPCS2. The IC₅₀ value for gapA3 was 0.290 nM, compared to 0.0391 nM for the parent strand, showing a difference of about seven times. Therefrom it was shown that nucleic acid drugs containing carbocyclic nucleoside with a spiro ring structure in the strand, or nucleic acid drugs obtained by substituting a part of monomers in the nucleic acid drug strand with carbocyclic nucleoside with a spiro ring structure, are useful for obtaining nucleic acid drugs with reduced cytotoxicity.

### [Industrial Applicability]

According to the present invention, in one embodiment, an oligonucleotide that exhibits superior effects on target RNA and the like, while maintaining high safety, and is useful as a medicament is provided.

This application is based on a patent application No. 2023-187759 filed in Japan (filing date: November 1, 2023), the contents of which are incorporated in full herein.

## Claims

1. An oligonucleotide or a salt thereof, comprising, in an oligonucleotide sequence thereof, at least one carbocyclic nucleoside derivative residue (B) ("nucleoside residue (B)") which is a divalent group represented by the following formula (B): 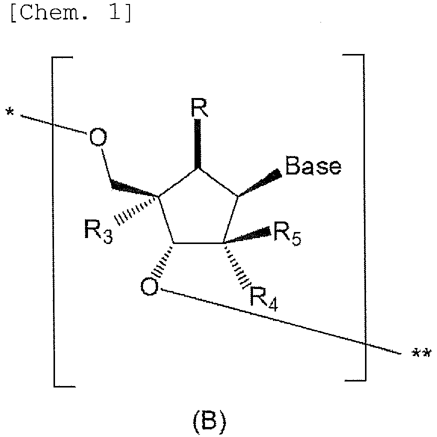 wherein
Base is a purin-9-yl group or a 2-oxo-1,2-dihydropyrimidin-1-yl group, each optionally having any one or more substituents selected from substituent group (a),
wherein the substituent group (a) consists of a hydroxyl group, a hydroxyl group protected by a nucleic acid synthesis protecting group, an oxo group, a linear alkyl group having 1 to 6 carbon atoms, a linear alkoxy group having 1 to 6 carbon atoms, a mercapto group, a mercapto group protected by a nucleic acid synthesis protecting group, a linear alkylthio group having 1 to 6 carbon atoms, an amino group, a linear alkylamino group having 1 to 6 carbon atoms, an amino group protected by a nucleic acid synthesis protecting group, and a halogen atom (wherein if the purin-9-yl group or the 2-oxo-1,2-dihydropyrimidin-1-yl group has an oxo group as a substituent selected from substituent group (a), the bond between the carbon atom to which the oxo group is bonded and the adjacent atom is a single bond);
R₃ and R₄ are each a hydrogen atom;
R₅ is a hydrogen atom;
a group represented by the following partial structural formula (i): 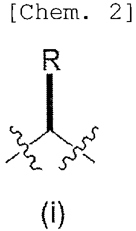
is a group represented by the following partial structural formula (i-1) or (i-2): 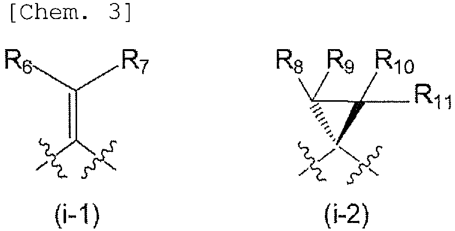 wherein
R₆ and R₇ are each independently a hydrogen atom, a halogen atom, or an alkyl group having 1 to 6 carbon atoms, or
R₆ and R₇ are bonded to each other to form, together with the adjacent carbon atom, a carbocycle having 3 to 6 carbon atoms,
R₈, R₉, R₁₀ and R₁₁ are each independently a hydrogen atom, a halogen atom, or an alkyl group having 1 to 6 carbon atoms;
* is a binding site to an adjacent oligonucleotide component, or R₁ if the nucleoside residue (B) is located at the 5' end of the oligonucleotide sequence
wherein R₁ is a hydrogen atom, a hydroxyl-protecting group in nucleic acid synthesis, an optionally branched or optionally ring-forming alkyl group having 1 to 7 carbon atoms, an optionally branched or optionally ring-forming alkenyl group having 2 to 7 carbon atoms, an aryl group having 3 to 10 carbon atoms, which optionally has any one or more substituents selected from substituent group (a) and which optionally contains heteroatoms, an aralkyl group having an aryl moiety having 3 to 12 carbon atoms, wherein said aryl moiety optionally has any one or more substituents selected from substituent group (a) and optionally contains heteroatoms, an acyl group optionally having any one or more substituents selected from substituent group (a), a silyl group optionally having any one or more substituents selected from substituent group (a), a phosphate group optionally having any one or more substituents selected from substituent group (a), a phosphate group protected by a nucleic acid synthesis protecting group, or -P(R₁₂)R₁₃ wherein R₁₂ and R₁₃ are each independently a hydroxy group, a hydroxyl group protected by a nucleic acid synthesis protecting group, a mercapto group, a mercapto group protected by a nucleic acid synthesis protecting group, an amino group, an alkoxy group having 1 to 6 carbon atoms, an alkylthio group having 1 to 6 carbon atoms, a cyanoalkoxy group having 1 to 6 carbon atoms, or a dialkylamino group having an alkyl group having 1 to 6 carbon atoms; and
** is a binding site to an adjacent oligonucleotide component, or R₂ if the nucleoside residue (B) is located at the 3' end of the oligonucleotide sequence
wherein R₂ is as defined for the R₁.

2. The oligonucleotide according to claim 1, wherein, in the nucleoside residue (B),
the group represented by the partial structural formula (i): 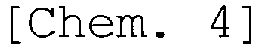 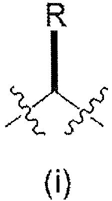
is a group represented by the following partial structural formula: (i-1): 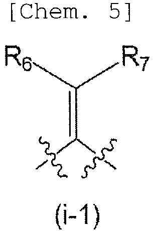
wherein R₆ and R₇ are each as defined above,
or a salt thereof.

3. The oligonucleotide or a salt thereof according to claim 1, wherein, in the nucleoside residue (B),
the group represented by the partial structural formula (i): 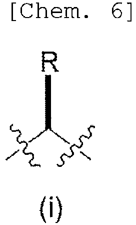
is a group represented by the following partial structural formula (i-2): 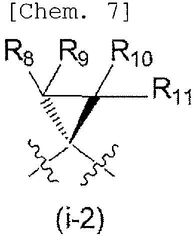
wherein R₈, R₉, R₁₀ and R₁₁ are each as defined above.

4. The oligonucleotide or a salt thereof according to any one of claims 1 to 3, wherein the oligonucleotide sequence comprises 1 to 10 nucleoside residues (B).

5. The oligonucleotide or a salt thereof according to any one of claims 1 to 3, wherein the oligonucleotide is 7 to 30 bases in length.

6. The oligonucleotide or a salt thereof according to any one of claims 1 to 3, wherein the oligonucleotide is 10 to 20 bases in length.

7. The oligonucleotide or a salt thereof according to any one of claims 1 to 3, wherein toxicity is reduced compared to before the introduction of nucleoside residue (B).

8. The oligonucleotide or a salt thereof according to any one of claims 1 to 3, wherein the oligonucleotide is a gapmer consisting of a gap region of 2 to 14 bases in length, a 5'-wing region of 2 to 5 bases in length, and a 3'-wing region of 2 to 5 bases in length, and the gap region is located between the 5'-wing region and the 3'-wing region.

9. The oligonucleotide or a salt thereof according to claim 8, wherein the gap region comprises at least one nucleoside residue (B).

10. The oligonucleotide or a salt thereof according to claim 9, wherein the 5'-wing region and/or 3'-wing region contain at least one nucleoside residue (B).

11. The oligonucleotide or a salt thereof according to any one of claims 1 to 3, wherein at least one of internucleotide linkages in the oligonucleotide is a phosphorothioate linkage.

12. The oligonucleotide or a salt thereof according to any one of claims 1 to 3, wherein all of internucleotide linkages in the oligonucleotide are phosphorothioate linkages.

13. The oligonucleotide or a salt thereof according to any one of claims 1 to 3, wherein, in the nucleoside residue (B), Base is a purin-9-yl group or a 2-oxo-1,2-dihydropyrimidin-1-yl group, each optionally having any 1 to 3 substituents selected from substituent group (a),
wherein the substituent group (a) consists of a hydroxyl group, a hydroxyl group protected by a nucleic acid synthesis protecting group, an oxo group, a linear alkyl group having 1 to 6 carbon atoms, a linear alkoxy group having 1 to 6 carbon atoms, a mercapto group, a mercapto group protected by a nucleic acid synthesis protecting group, a linear alkylthio group having 1 to 6 carbon atoms, an amino group, a linear alkylamino group having 1 to 6 carbon atoms, an amino group protected by a nucleic acid synthesis protecting group, and a halogen atom.

14. The oligonucleotide or a salt thereof according to claim 13, wherein, in the nucleoside residue (B),
R₆ and R₇ are hydrogen atoms;
* is a binding site to an adjacent oligonucleotide component, or a hydrogen atom if the nucleoside residue (B) is located at the 5' end of the oligonucleotide sequence; and
** is a binding site to an adjacent oligonucleotide component, or a hydrogen atom if the nucleoside residue (B) is located at the 3' end of the oligonucleotide sequence.

15. The oligonucleotide or a salt thereof according to claim 13, wherein, in the nucleoside residue (B),
R₈, R₉, R₁₀ and R₁₁ are each a hydrogen atom;
* is a binding site to an adjacent oligonucleotide component, or a hydrogen atom if the nucleoside residue (B) is located at the 5' end of the oligonucleotide sequence; and
** is a binding site to an adjacent oligonucleotide component, or a hydrogen atom if the nucleoside residue (B) is located at the 3' end of the oligonucleotide sequence.

16. Use of the oligonucleotide or a salt thereof according to any one of claims 1 to 3, as an antisense oligonucleotide, or as an oligonucleotide constituting siRNA.

17. A method for reducing the toxicity of an oligonucleotide or a salt thereof, comprising introducing at least one carbocyclic nucleoside derivative residue (B) ("nucleoside residue (B)"), which is a divalent group represented by the following formula (B) 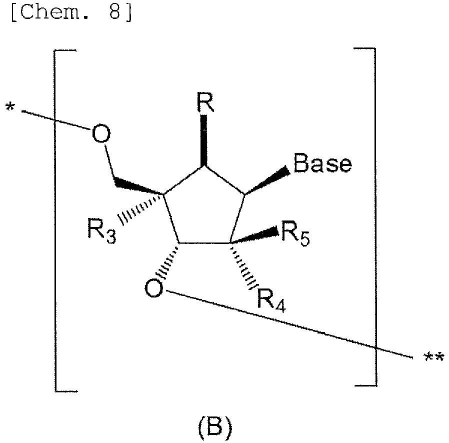 wherein each group and partial structure are as defined for the corresponding group and partial structure defined for "nucleoside residue (B)" in claim 1, into the oligonucleotide sequence.

18. Use of a carbocyclic nucleoside derivative represented by the following formula (A) ("nucleoside (A)") or a salt thereof, for reducing the toxicity of an oligonucleotide 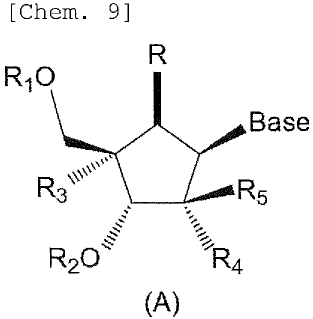 wherein each group and partial structure are as defined for the corresponding group and partial structure defined for "nucleoside residue (B)" in claim 1.

19. The use according to claim 18, wherein the reduction of the toxicity of the oligonucleotide comprises introducing at least one nucleoside (A) into the oligonucleotide sequence.

20. A medicament comprising the oligonucleotide or a salt thereof according to any one of claims 1 to 3 as an active ingredient.
